(19) Europäisches Patentamt
European Patent Office
Office européen des brevets

(11) **EP 4 745 128 A1**

(12) **EUROPEAN PATENT APPLICATION**
published in accordance with Art. 153(4) EPC

(43) Date of publication:
**20.05.2026 Bulletin 2026/21**

(21) Application number: **24842281.8**

(22) Date of filing: **12.07.2024**

(51) International Patent Classification (IPC):
***C07D 241/12*** (2006.01) ***C07D 213/74*** (2006.01)
***A61K 31/165*** (2006.01) ***C07D 231/14*** (2006.01)
***C07D 237/10*** (2006.01) ***C07D 261/08*** (2006.01)
***C07D 277/32*** (2006.01) ***C07D 307/56*** (2006.01)

(52) Cooperative Patent Classification (CPC):
**A61K 31/165; C07D 213/74; C07D 231/14; C07D 237/10; C07D 241/12; C07D 261/08; C07D 277/32; C07D 307/56**

(86) International application number:
**PCT/CN2024/105066**

(87) International publication number:
**WO 2025/016287 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.07.2023 CN 202310866584**

(71) Applicant: **Shenzhen Zhongge Biological Technology Co., Ltd.**
**Futian District**
**Shenzhen, Guangdong 518017 (CN)**

(72) Inventors:
- **CAO, Bin**
  **Shenzhen, Guangdong 518017 (CN)**
- **ZHAO, Liyu**
  **Shenzhen, Guangdong 518017 (CN)**
- **DOU, Guosheng**
  **Shenzhen, Guangdong 518017 (CN)**
- **LU, Sizhu**
  **Shenzhen, Guangdong 518017 (CN)**
- **XIE, Dewei**
  **Shenzhen, Guangdong 518017 (CN)**
- **ZHANG, Qihua**
  **Shenzhen, Guangdong 518017 (CN)**
- **WANG, Shaohui**
  **Shenzhen, Guangdong 518017 (CN)**
- **CHEN, Bin**
  **Shenzhen, Guangdong 518017 (CN)**
- **ZHANG, Peiyu**
  **Shenzhen, Guangdong 518017 (CN)**

(74) Representative: **Marks & Clerk LLP**
**15 Fetter Lane**
**London EC4A 1BW (GB)**

(54) **COMPOUND, PHARMACEUTICAL COMPOSITION COMPRISING SAME, AND USE THEREOF**

(57) The present application provides a compound of Formula 0, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof:

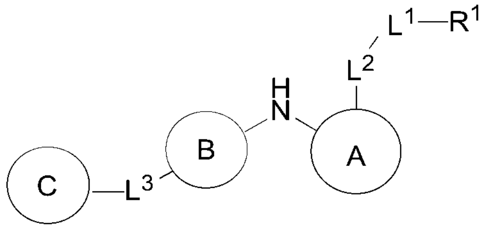

Formula 0.

EP 4 745 128 A1

## Description

### TECHNICAL FIELD

The present application relates to the field of biomedicine, in particular, to a compound, a pharmaceutical composition comprising same, and uses thereof.

### BACKGROUND

Amyotrophic lateral sclerosis (ALS), also known as motor neuron disease (MND), often called Lou Gehrig's disease, is an irreversible and fatal motor neuron disease. The main symptoms of the disease are progressively worsening muscle weakness and atrophy in the limbs and trunk muscles, leading to a gradual loss of motor function, as if the body were being "frozen". Therefore, the disease is colloquially referred to in Chinese as the "gradually frozen person" disease. The core pathological findings in ALS are the death of motor neurons in the motor cortex and spinal cord, and the degeneration of corticospinal axons, which leads to thinning and scarring (sclerosis) of the lateral parts of the spinal cord.

Loss of protein folding homeostasis is a hallmark of many of the most prevalent neurodegenerative diseases. As a mechanism for coping with folding stress within the endoplasmic reticulum (ER), the unfolded protein response (UPR) includes a set of signaling mechanisms that initiate gene expression programs to restore protein homeostasis or, when stress is chronic or excessive, promote neuronal death. This function of the UPR has been proposed to play a key role in ALS.

The integrated stress response (ISR) is an evolutionarily conserved intracellular signaling network that helps cells, tissues, and organisms adapt to changing environments and remain healthy. The ISR responds to various changes and restores balance by reprogramming gene expression. Long-term memory formation in the brain requires new protein synthesis. Therefore, inhibition of the ISR can enhance long-term memory formation, while activation of the ISR blocks this process. Furthermore, age-related cognitive impairment is often associated with ISR activation.

As a central regulator of protein homeostasis, ISR activation has been observed in a wide range of brain diseases. This activation process has been confirmed by detecting eIF2-P and phosphorylation of PKR, PERK and GCN2 in the brain of samples from patients and animal models of neurodegenerative diseases such as Alzheimer's disease, Parkinson's disease, Huntington's disease, traumatic brain injury, Down syndrome, and Charcot-Marie-Tooth. Notably, ISR activation leads to cognitive deficits in mouse models of traumatic brain injury, aging and Alzheimer's disease.

eIF2B (eukaryotic translation initiation factor 2B) is a key enzyme for regulating protein synthesis and is a guanine nucleotide exchange factor (GEF) specific for translation initiation factor 2. The eIF2B activator ISRIB can restore protein translation, restore UPR transcription to basal levels, and reduce the integrated stress response (ISR). Additionally, the eIF2B activators ABBV-CLS-7262 (AbbVie/Calico) and DNL-343 (Denali Therapeutics) are both being developed for ALS and have entered Phase 1 clinical trials. Phase 1 data from healthy subjects for DNL-343 has been published, demonstrating that DNL-343 is safe and well-tolerated.

A large number of animal experiments have confirmed that the eIF2B activator ISRIB can improve long-term memory in mouse models. After three days of oral administration of the eIF2B activator ABBV-CLS-7262, the brain function of the model animals can be restored to youthful levels. This suggests that this drug may suppress some neurodegenerative diseases at later stages and has the potential to treat diseases such as Alzheimer's disease and Parkinson's disease.

### SUMMARY

The present application provides a compound, a pharmaceutical composition comprising same, and synthesis methods therefor, and uses thereof, to obtain a compound capable of significantly attenuating the integrated stress response (ISR), activating eIF2B activity, and restoring normal protein synthesis in cells, while exhibiting high cellular activity and/or excellent pharmacokinetic properties.

According to a first aspect, the present application provides a compound of Formula 0, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof:

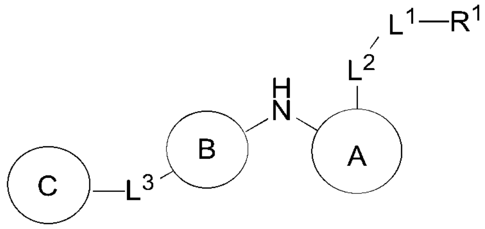

Formula 0

wherein in Formula 0, $R^1$ is selected from halogen, C1-C3 alkyl, C1-C3 haloalkyl, C1-C3 deuteroalkyl, C1-C3 alkylhydroxy, C1-C6 alkoxy and C1-C6 haloalkoxy;

$L^1$ is selected from C3-C10 cycloalkylene, 3-10-membered heterocycloalkylene and C1-C6 alkylene;

$L^2$ is selected from -O-, -NH-, C1-C6 alkylene and carbonyl;

ring A is selected from 5-10-membered heteroarylene containing at least one heteroatom N, and is optionally substituted with X $R^A$, where $R^A$ is selected from halogen, hydroxy, nitro, cyano, C1-C6 alkyl, C1-C6 alkoxy, C1-C6 haloalkoxy, C1-C6 haloalkyl, C3-C10 cycloalkyl and 3-10-membered heterocycloalkyl, and X is any integer from 0 to 4;

$L^2$ and -NH- are linked to ortho carbon atoms of ring A, respectively;

ring B is selected from C3-C10 cycloalkylene and 5-10-membered heterocycloalkylene, and is optionally substituted with Y $R^B$, where $R^B$ is selected from halogen, hydroxy, nitro, cyano, C1-C6 alkyl, C1-C6 alkoxy, C1-C6 haloalkyl, C1-C6 haloalkoxy, C3-C10 cycloalkyl and 3-10-membered heterocycloalkyl, and Y is any integer from 0 to 4;

$L^3$ is selected from a single bond, an amide bond, a thioamide bond, a -O-$(CH_2)$n-amide bond, amide bond-$(CH_2)$n-O-, a -O-$(CH_2)$m-thioamide bond, -thioamide bond-$(CH_2)$m-O- and 5-10-membered heteroarylene, where m and n are each independently 0, 1, 2 or 3; and

ring C is selected from C3-C10 cycloalkyl, 3-10-membered heterocycloalkyl, C6-C10 aryl, 5-10-membered heteroaryl, 3-10-membered benzoheterocycloalkyl, and is optionally substituted with Z $R^C$, where $R^C$ is selected from halogen, hydroxy, amino, nitro, cyano, C1-C6 alkyl, C1-C6 alkoxy, C1-C6 haloalkyl and C1-C6 haloalkoxy, and Z is any integer from 0 to 4.

In some embodiments, $R^1$ is selected from C1-C3 alkoxy and C1-C3 haloalkoxy, where preferably, the halogens of the C1-C3 haloalkoxy are each independently selected from F, Cl or Br, the number of C atoms of the C1-C3 haloalkoxy is preferably p, and the number of the halogens is any integer from 1 to 2p+1; the halogens of the C1-C3 haloalkoxy are preferably F or Cl; $R^1$ is preferably C1-C3 perfluoroalkyl, more preferably $-O-CF_3$.

In some embodiments, $L^1$ is selected from C3-C6 cycloalkylene, 3-6-membered heterocycloalkylene and C1-C3 alkylene; preferably selected from cyclobutylene, cyclopentylene, cyclohexylene, azacyclobutylene, oxacyclobutylene, azacyclopentylene, oxacyclopentylene, azacyclohexylene, oxacyclohexylene, methylene, ethylene and propylene, more preferably from

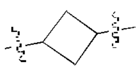 , 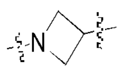 ,

methylene and ethylene, and even more preferably from

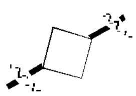 ,

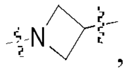

methylene and ethylene.

In some embodiments, the compound of Formula 0 is selected from those as defined in any one of structures shown in the following Formulas I-1 and I-2:

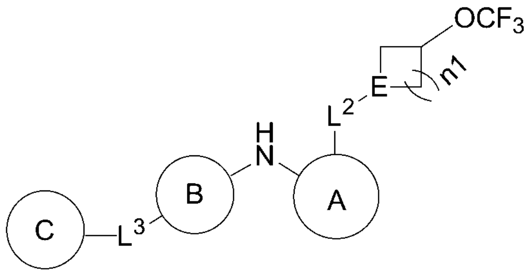

Formula I-1

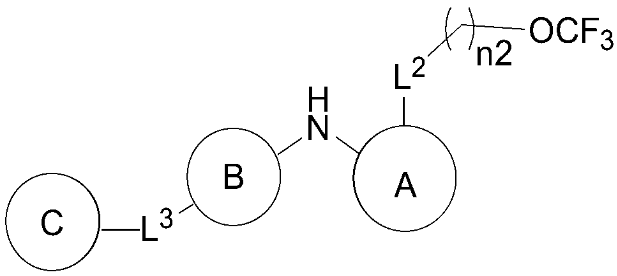

Formula I-2

wherein E is CH or N; n1 is 1, 2 or 3, preferably 1; n2 is 1, 2, 3 or 4, preferably 2; and $L^2$, ring A, $R^A$, X, ring B, $R^B$, Y, $L^3$, ring C, $R^C$ and Z are as defined in the above embodiments.

In some embodiments, the compound of Formula I-1 is any one selected from the structures shown in the following Formulas I-1-1 and I-1-2:

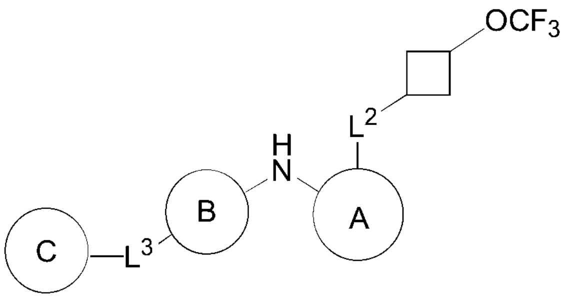

Formula I-1-1

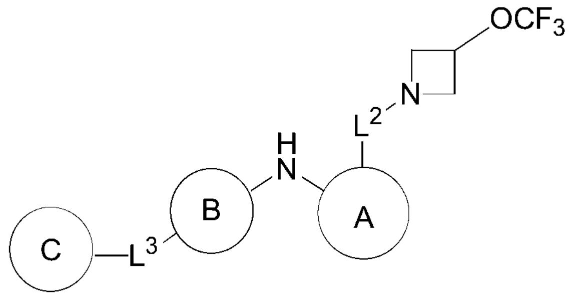

Formula I-1-2

wherein $L^2$, ring A, $R^A$, X, ring B, $R^B$, Y, $L^3$, ring C, $R^C$ and Z are as defined in the above embodiments.

In some embodiments, $L^2$ is selected from -O-, -NH-, C1-C3 alkylene and carbonyl, preferably from -O-, -NH-, methylene, ethylene and carbonyl.

In some embodiments, the compound of Formula 0 is any one selected from the structures shown in the following Formulas II-1 to II-4:

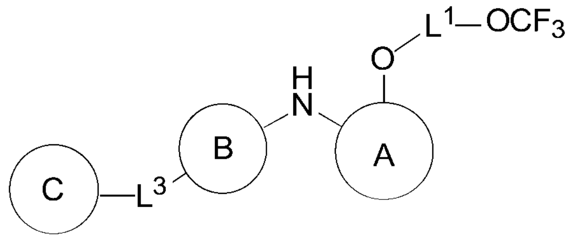

Formula II-1

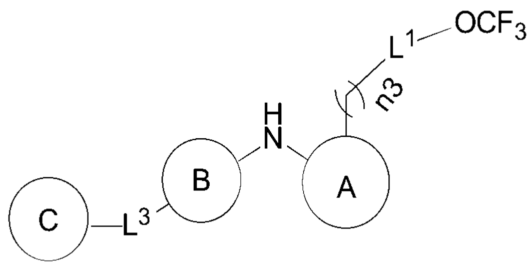

Formula II-2

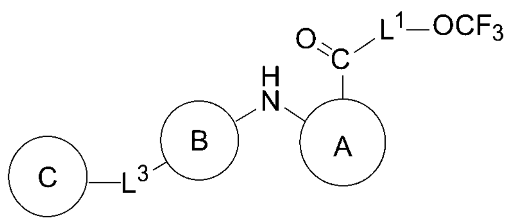

Formula II-3

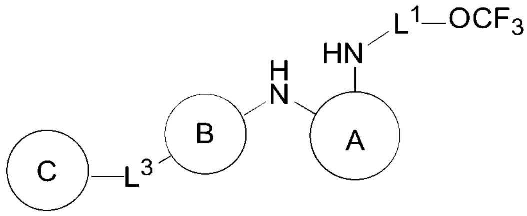

Formula II-4

wherein n3 is 1, 2 or 3, preferably 1; $L^1$, ring A, $R^A$, X, ring B, $R^B$, Y, $L^3$, ring C, $R^C$ and Z are as defined in the above embodiments.

In some embodiments, ring A is selected from 5-6-membered heteroarylene containing at least one heteroatom N, preferably from pyridylene, pyrimidylene, pyrazinylene, pyridazinylene, imidazolylene, pyrazolylene, triazolylene, oxazolylene, oxadiazolylene, furazanylene, furylene, thiazolylene, isoxazolylene, isothiazolylene and

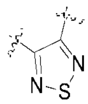.

In some embodiments, ring A is selected from pyridylene, pyrazinylene, triazolylene, furazanylene, isoxazolylene, and

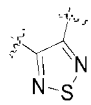.

In some embodiments, ring A is selected from

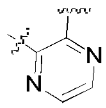, 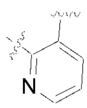, 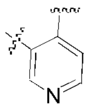, 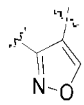, 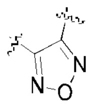,

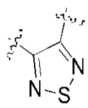, 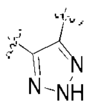, and 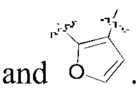.

In some embodiments, ring A is selected from

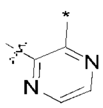, 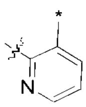, 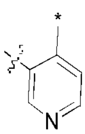, 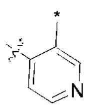, 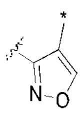,

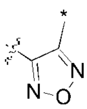, 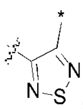, 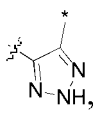, 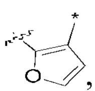, and 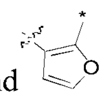,

where

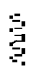

end is linked to -NH- and * end is linked to $L^2$.

In some embodiments, $R^A$ is selected from halogen, hydroxy, nitro, cyano, C1-C3 alkyl, C1-C3 alkoxy, C1-C3 haloalkoxy, C1-C3 haloalkyl, C3-C6 cycloalkyl, 3-6-membered heterocycloalkyl, where X is any integer from 0 to 4, preferably 0 or 1.

In some embodiments, the compound of Formula 0 is any one selected from the structures shown in the following Formulas III-1 and III-2:

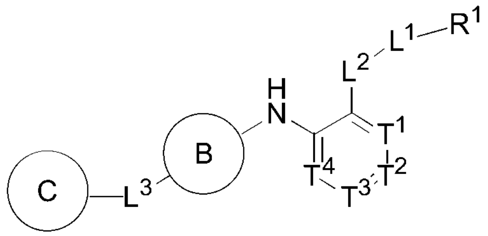

Formula III-1

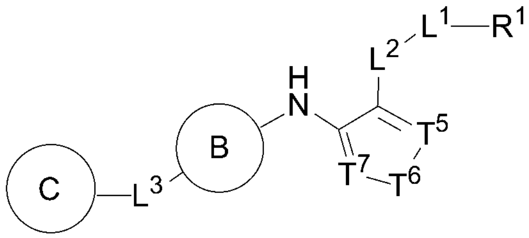

Formula III-2

wherein $T^1$, $T^2$, $T^3$ and $T^4$ are each independently C or N, and at least one of $T^1$, $T^2$, $T^3$ and $T^4$ is N; preferably, $T^1$ is N, $T^2$ and $T^3$ are C, and $T^4$ is C or N; or preferably, one of $T^1$, $T^2$ and $T^3$ is N, and $T^4$ is C;

$T^5$, $T^6$ and $T^7$ are each independently C, N, O or S; and

$R^1$, $L^1$, $L^2$, ring B, $R^B$, Y, $L^3$, ring C, $R^C$ and Z are as defined in any of the above embodiments.

In some embodiments, the compound of Formula III-1 is any one selected from the structures shown in the following Formula III-1-1:

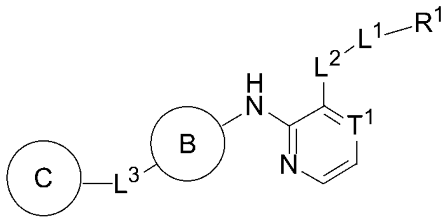

Formula III-1-1

wherein $T^1$ is C or N, and $R^1$, $L^1$, $L^2$, ring B, $R^B$, Y, $L^3$, ring C, $R^C$ and Z are as defined in any of the above embodiments.

In some embodiments, the compound of Formula III-2 is any one selected from the structures shown in the following Formulas III-2-1 or III-2-2:

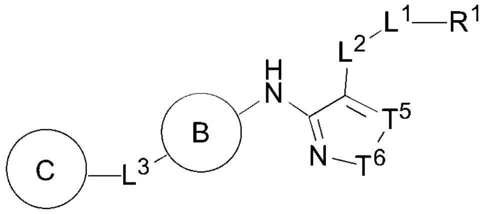

Formula III-2-1

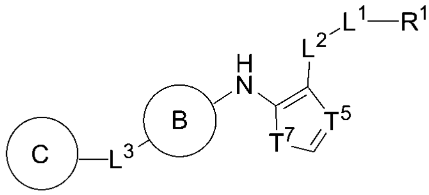

Formula III-2-2

wherein in Formula III-21, $T^5$ and $T^6$ are each independently C, N, O or S, and preferably, $T^5$ is C or N, and $T^6$ is N, O or S;

in Formula III-22, $T^5$ and $T^7$ are each independently C, O or S, and preferably, $T^5$ is C or O, $T^7$ is C or O, and $T^5$ is different from $T^7$; and

$R^1$, $L^1$, $L^2$, ring B, $R^B$, Y, $L^3$, ring C, $R^C$ and Z are as defined in any of the above embodiments.

In some embodiments, ring B is selected from C4-C8 cycloalkylene and 5-6-membered heterocycloalkylene; the C4-C8 cycloalkylene is preferably C4-C8 monocycloalkyl or C4-C8 bridged cycloalkyl; ring B is preferably selected from cyclobutylene, cyclopentylene, cyclohexylene, bicyclo[2.2.1]heptanylene, bicyclo[2.2.2]octanylene, bicyclo[1.1.2]pentanylene, piperidylene and oxanylene, more preferably from

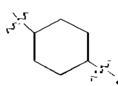, 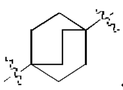, 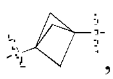, 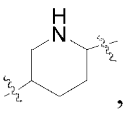,

and

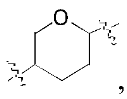,

even more preferably from

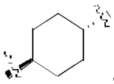, 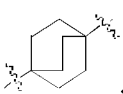, 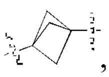, 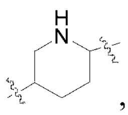, and 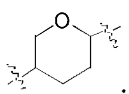.

In some embodiments, $R^B$ is selected from halogen, hydroxy, nitro and cyano; and Y is any integer from 0 to 4, preferably 0.

In some embodiments, the compound of Formula 0 is selected from any of structures shown in the following Formulas IV-1 to IV-3:

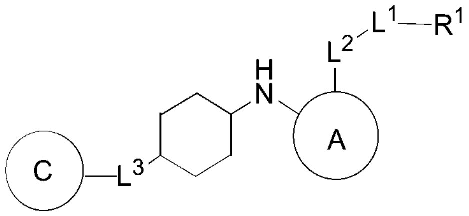

Formula IV-1

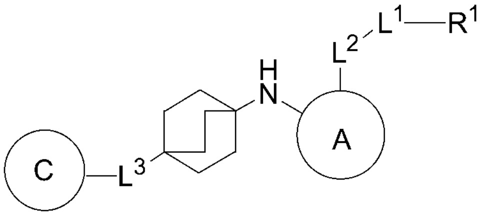

Formula IV-2

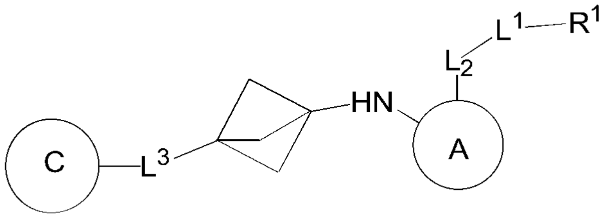

Formula IV-3

wherein $R^1$, $L^1$, $L^2$, ring A, $R^A$, X, $L^3$, ring C, $R^C$ and Z are as defined in any of the above embodiments.

In some embodiments, the compound of Formula IV-1 is any one selected from the structures shown in the following Formulas IV-1-1 and IV-1-2:

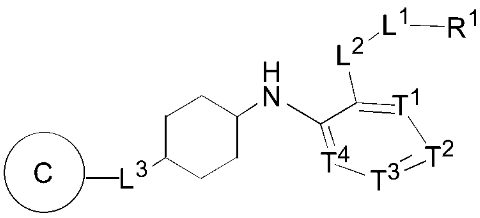

Formula IV-1-1

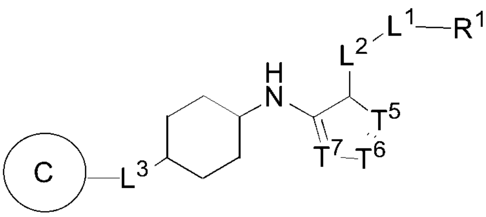

Formula IV-1-2.

Preferably, the compound of Formula IV-2 is any one selected from the structures shown in the following Formulas IV-2-1 and IV-2-2:

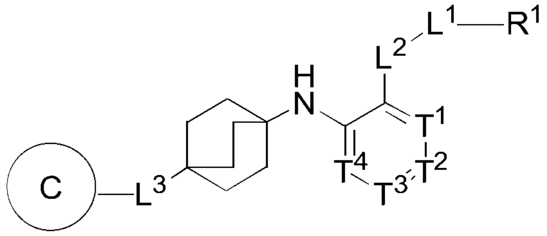

Formula IV-2-2

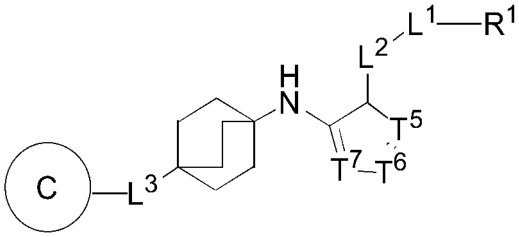

Formula IV-2-2

wherein $R^1$, $L^1$, $L^2$, $R^A$, X, $L^3$, ring C, $R^C$ and Z are as defined in any of the above embodiments.

In some embodiments, the compound of Formula IV-3 is selected from any of structures shown in the following Formulas IV-3-1 and IV-3-2:

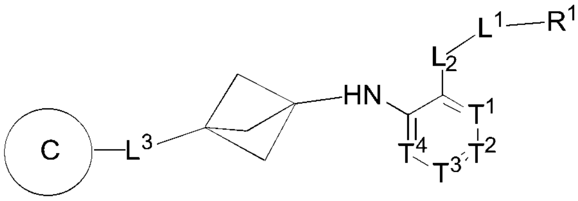

Formula IV-3-1

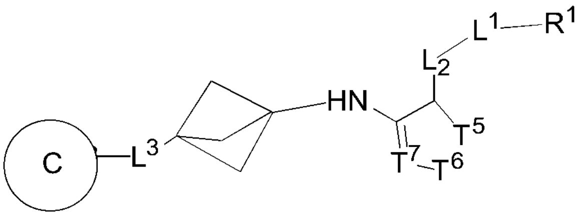

Formula IV-3-2

wherein $R^1$, $L^1$, $L^2$, $R^A$, X, $L^3$, ring C, $R^C$ and Z are as defined in any of the above embodiments.

In some embodiments, $L^3$ is selected from

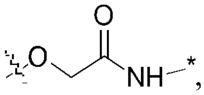,

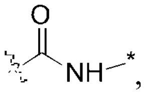,

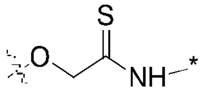

and 5-6-membered heteroarylene, where preferably,

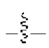

end is linked to ring C, and -* end is linked to ring B; the 5-6-membered heteroarylene is preferably selected from pyrrolylene, pyrazolylene, imidazolylene, triazolylene, oxazolylene, isoxazolylene, oxadiazolylene, and

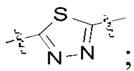;

$L^3$ is preferably selected from

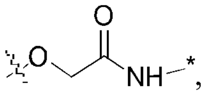, 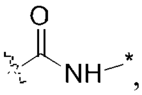, 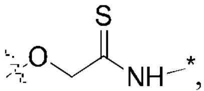, 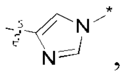,

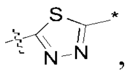, and 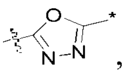,

where

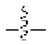

end is linked to ring C, and -* end is linked to ring B; and $L^3$ is more preferably selected from

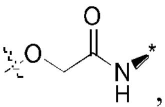, 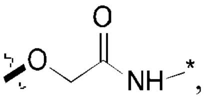, 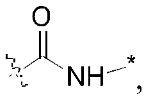, 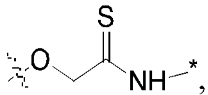,

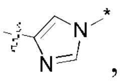, 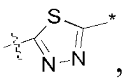, and 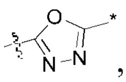,

where

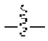

end is linked to ring C, and -* end is linked to ring B.

In some embodiments, the compound of Formula 0 is any one selected from the structures shown in the following Formulas V-1 to V-3:

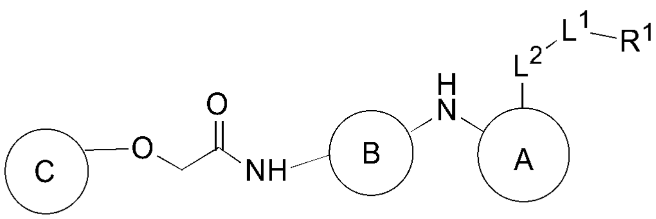

Formula V-1

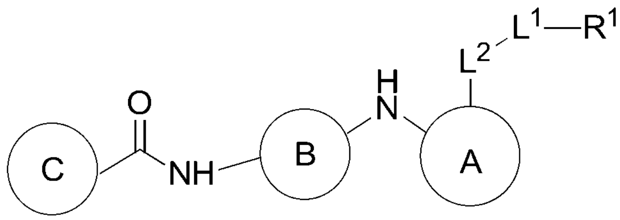

Formula V-2

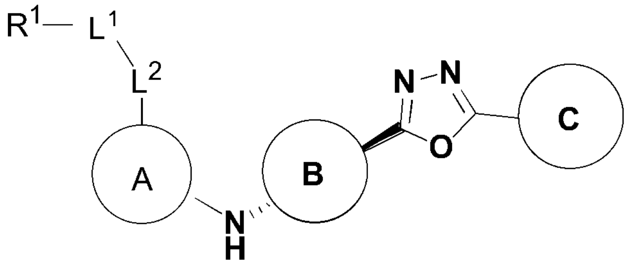

Formula V-3

wherein $R^1$, $L^1$, $L^2$, ring A, $R^A$, X, ring B, $R^B$, Y, ring C, $R^C$ and Z are as defined in any of the above embodiments.

In some embodiments, the compound of Formula V-1 is any one selected from the structures shown in the following Formulas V-1-1 to V-1-11:

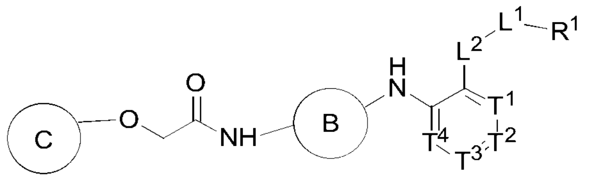

Formula V-1-1

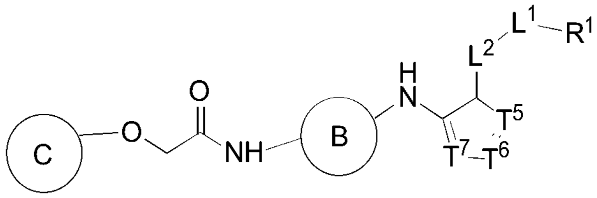

Formula V-1-2

wherein $R^1$, $L^1$, $L^2$, $R^A$, X, ring B, $R^B$, Y, ring C, $R^C$ and Z are as defined in any of the above embodiments;

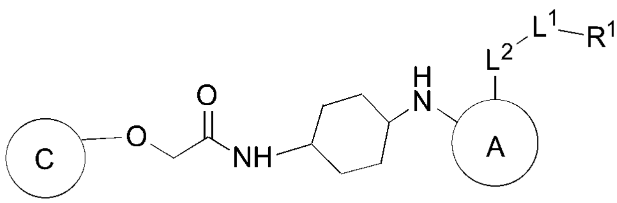

Formula V-1-3

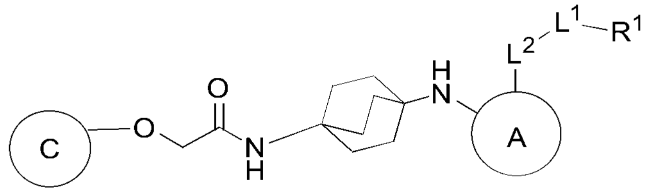

Formula V-1-4

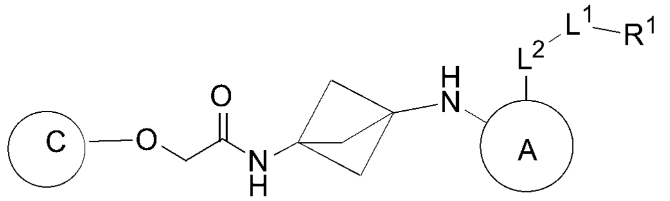

Formula V-1-5

$R^1$, $L^1$, $L^2$, ring A, $R^A$, X, $R^B$, Y, ring C, $R^C$ and Z are as defined in any of the above embodiments; and

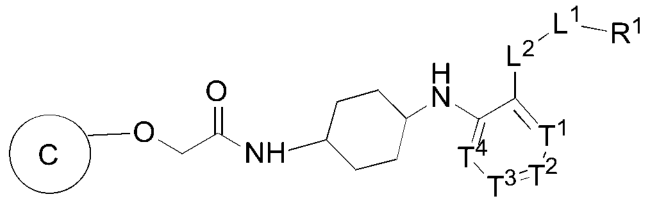

Formula V-1-6

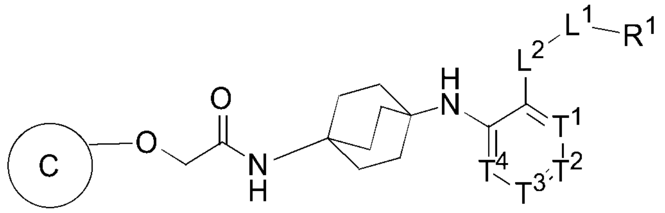

Formula V-1-7

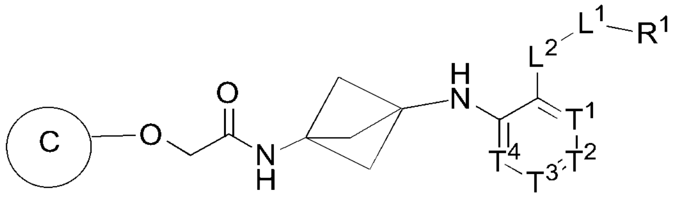

Formula V-1-8

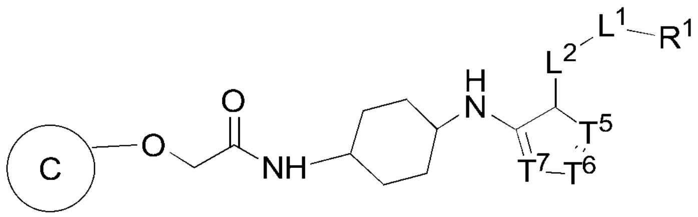

Formula V-1-9

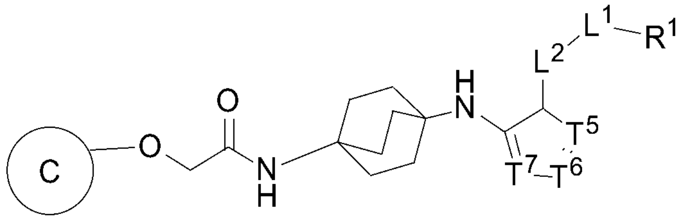

Formula V-1-10

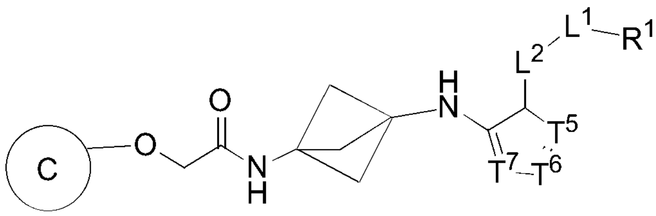

Formula V-1-11

$R^1$, $L^1$, $L^2$, $R^A$, X, $R^B$, Y, ring C, $R^C$ and Z are as defined in any of the above embodiments.

In some embodiments, the compound of Formula V-2 is any one selected from the structures shown in the following Formulas V-2-1 to V-2-11:

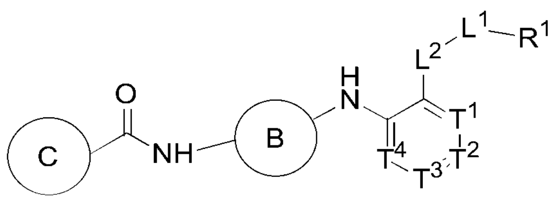

Formula V-2-1

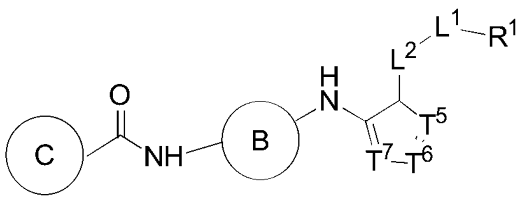

Formula V-2-2

wherein $R^1$, $L^1$, $L^2$, $R^A$, X, ring B, $R^B$, Y, ring C, $R^C$ and Z are as defined in any of the above embodiments;

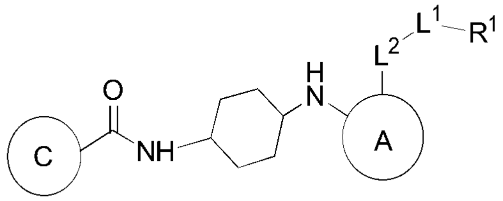

Formula V-2-3

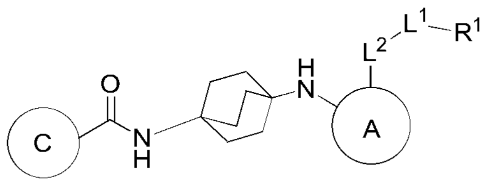

Formula V-2-4

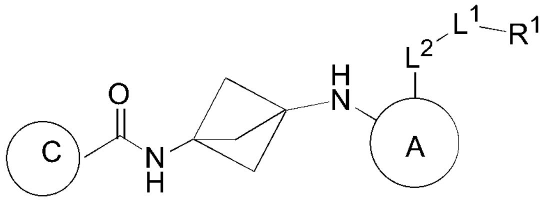

Formula V-2-5

$R^1$, $L^1$, $L^2$, ring A, $R^A$, X, $R^B$, Y, ring C, $R^C$ and Z are as defined in any of the above embodiments; and

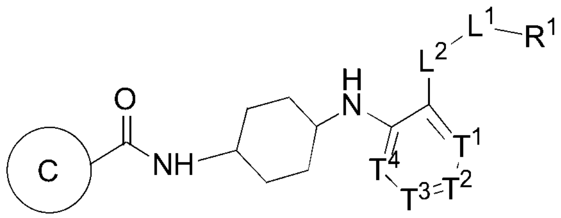

Formula V-2-6

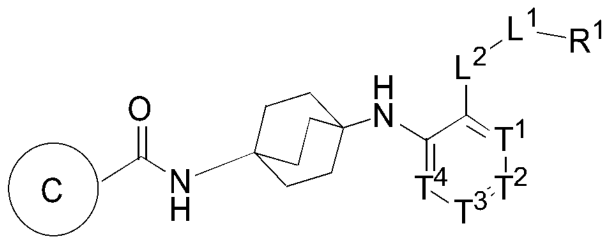

Formula V-2-7

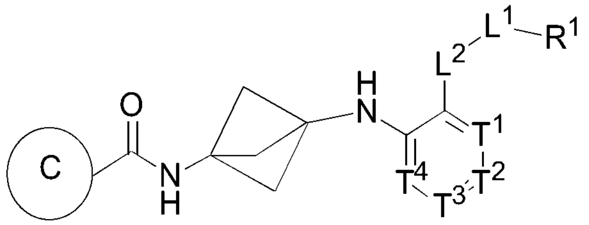

Formula V-2-8

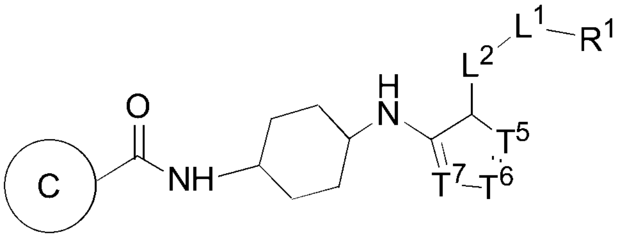

Formula V-2-9

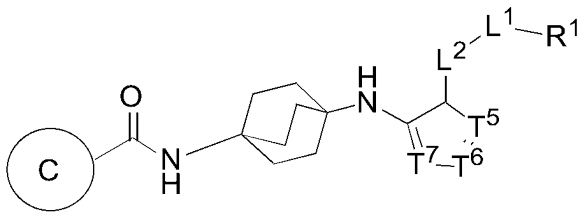

Formula V-2-10

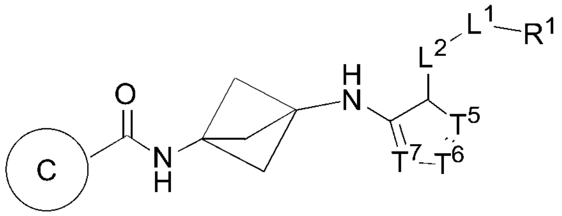

Formula V-2-11

$R^1$, $L^1$, $L^2$, $R^A$, X, $R^B$, Y, ring C, $R^C$ and Z are as defined in any of the above embodiments.

In some embodiments, ring C is selected from C3-C6 cycloalkyl, 3-6-membered heterocycloalkyl, C6-C10 aryl, 5-6-membered heteroaryl and 3-6-membered benzoheterocycloalkyl, preferably from cyclobutyl, cyclohexyl, azacyclobutyl, piperidyl, phenyl, thienyl, furyl, pyrrolyl, pyrazolyl, imidazolyl, triazolyl, pyridyl, pyrimidyl, pyrazinyl, pyridazinyl, triazinyl, quinolyl, isoquinolyl, quinazolinyl, quinoxalinyl, benzomorpholinyl and benzoxocyclohexyl, more preferably from cyclobutyl, phenyl, pyrazinyl, quinolyl, benzomorpholinyl and benzoxocyclohexyl, even more preferably from

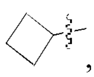, 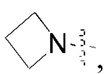, 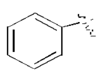,

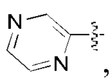, 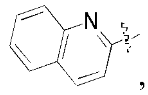, 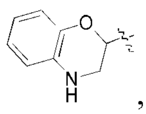, and 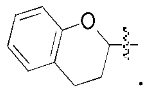.

In some embodiments, ring C is selected from

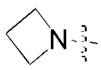, 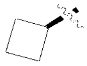, 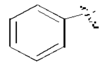, 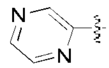,

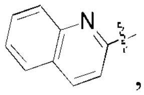, 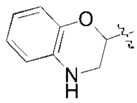, and 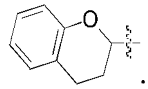.

In some embodiments, $R^C$ is selected from halogen, hydroxy, C1-C3 alkyl, C1-C3 alkoxy, C1-C3 haloalkyl and -C1-C3 haloalkoxy; Z is any integer from 0 to 4; preferably, halogen, the halogen in the C1-C3 haloalkyl and the halogen in the C1-C3 haloalkoxy are each independently F, Cl or Br, more preferably F or Cl; $R^C$ is preferably selected from F, Cl, hydroxy, $-CH_2F$, $-CHF_2$, $-CF_3$ and $-O-CF_3$; and Z is preferably 1 or 2.

In some embodiments, the compound of Formula 0 is any one selected from the structures shown in the following Formulas VI-1 and VI-2:

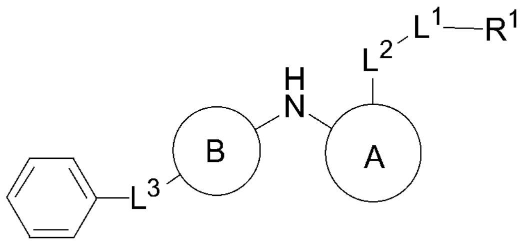

Formula VI-1

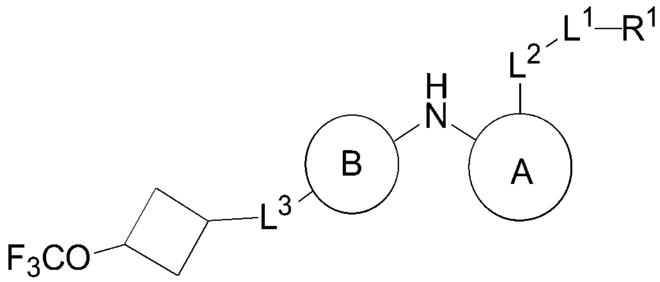

Formula VI-2

wherein $R^C$ in Formula VI-1 is selected from F, Cl, hydroxy, -CN, $-CH_3$, $-CH_2F$, $-CHF_2$, $-CF_3$, $-OCH_3$ and $-OCF_3$; Z is preferably 1 or 2; and

in Formulas VI-1 to VI-2, ring A, $R^A$, X, ring B, $R^B$, Y and $L^3$ are as defined in any of the above embodiments.

In some embodiments, the compound of Formula 0 is any one selected from the structures shown in the following Formula VII:

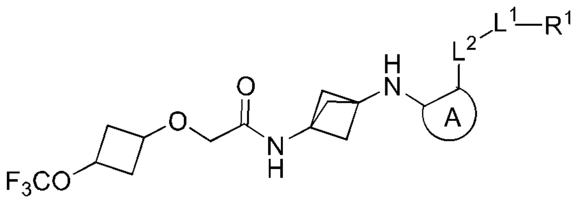

Formula VII

wherein ring A is selected from 5-membered nitrogen-containing heteroarylene or 6-membered nitrogen-containing heteroarylene; $L^1$ is selected from C3-C6 cycloalkylene or 4-6-membered heterocyclylene; $L^2$ is selected from -O-; and $R^1$ is selected from C1-C3 alkoxy or C1-C3 haloalkoxy.

In some embodiments, ring A is selected from 6-membered nitrogen-containing heteroarylene.

In some embodiments, $L^1$ is selected from C3-C5 cycloalkylene or 4-5-membered heterocyclylene.

In some embodiments, $L^1$ is selected from cyclobutylene or azacyclobutanylene, preferably cyclobutylene.

In some embodiments, $R^1$ is selected from C1-C3 haloalkoxy, preferably $-OCF_3$.

In some embodiments, the compound of Formula 0 is any one selected from the structures shown in the following Formula VIII:

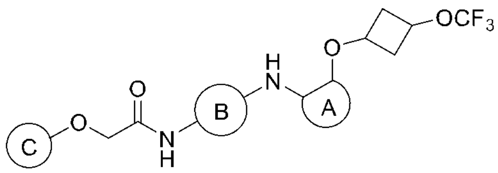

Formula VIII

wherein ring A is selected from 5-membered nitrogen-containing heteroarylene or 6-membered nitrogen-containing heteroarylene; ring B is selected from 4-8-membered cycloalkylene; ring C is selected from phenyl or cyclobutyl; and the rings are each optionally substituted with 1 or 2 $R^C$ that is selected from halogen, C1-C3 alkyl, C1-C3 haloalkyl, C1-C3 alkoxy or C1-C3 haloalkoxy.

In some embodiments, ring A is selected from 6-membered nitrogen-containing heteroarylene.

In some embodiments, the compound of Formula VIII is any one selected from the structures shown in the following Formula VIII-A:

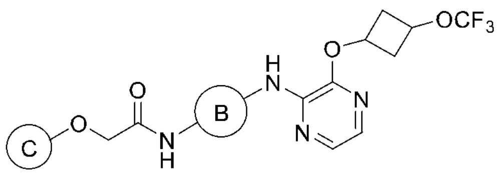

Formula VIII-A.

In some embodiments, the compound of Formula VIII-A is any one selected from the structures shown in the following Formulas VIII-A-1 or VIII-A-2:

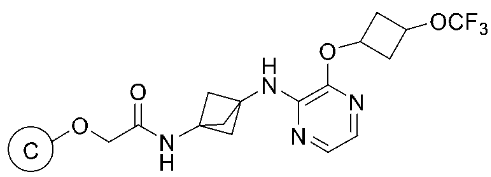

Formula VIII-A-1

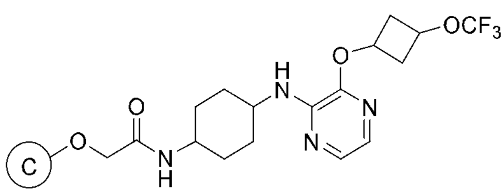

Formula VIII-A-2.

In some embodiments, ring C is selected from cyclobutyl optionally substituted with C1-C3 haloalkoxy.

In some embodiments, the compound is any one selected from the following compounds:

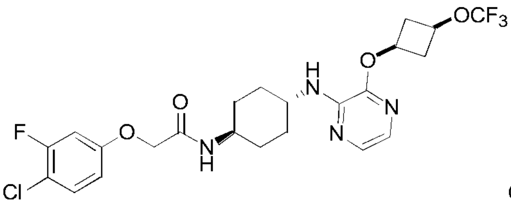
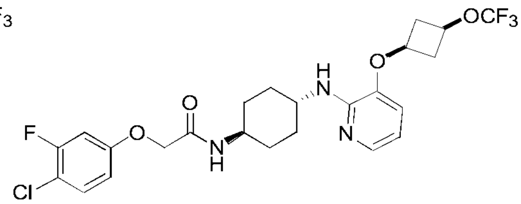
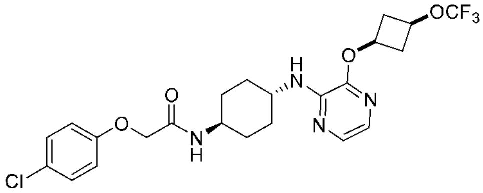
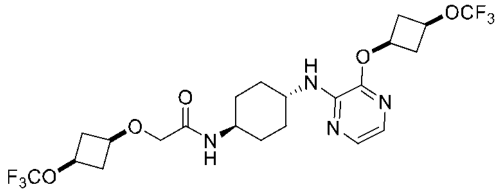
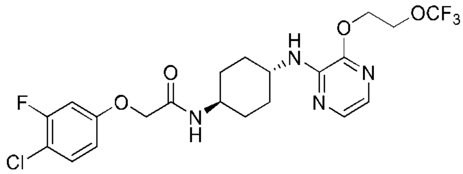
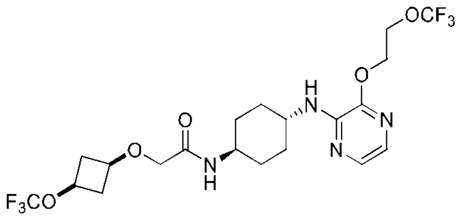
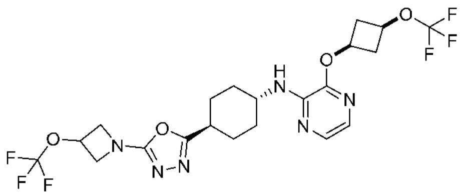
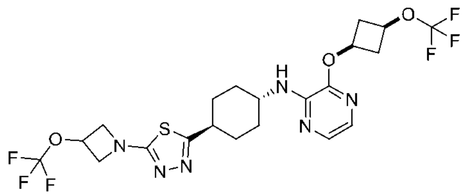
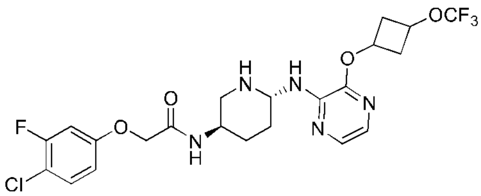
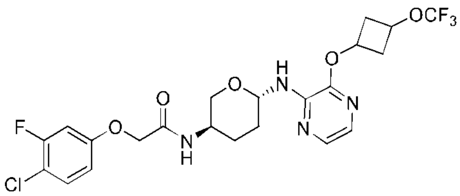
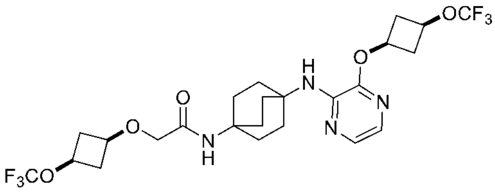
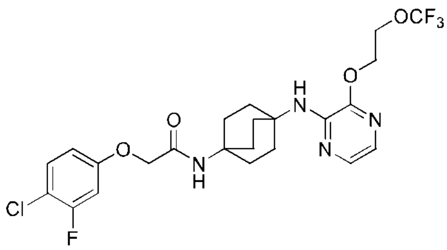
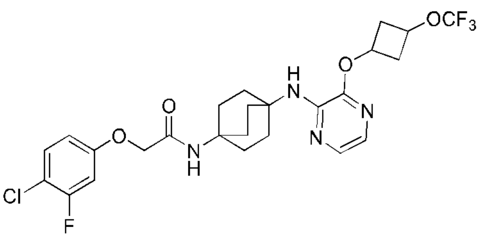
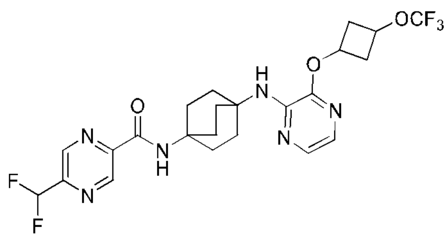

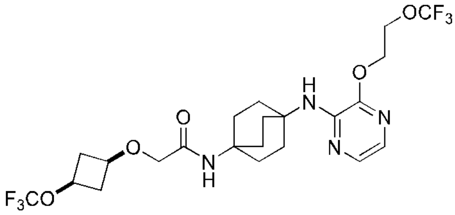

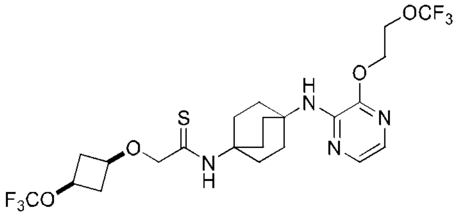

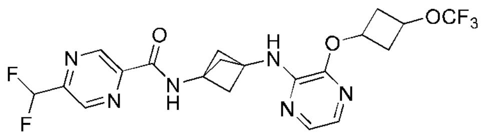

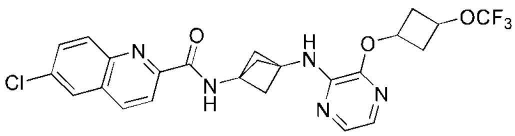

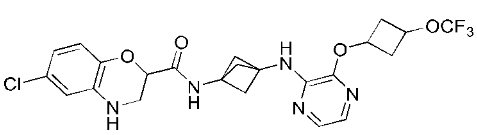

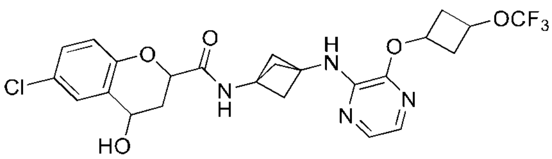

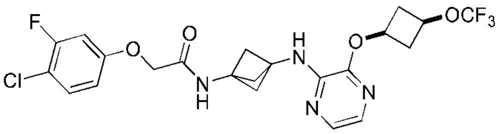

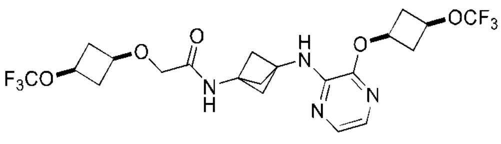

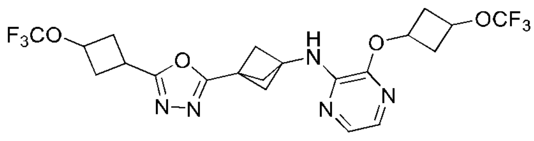

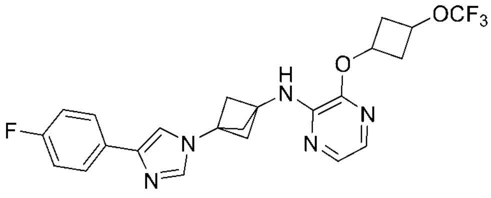

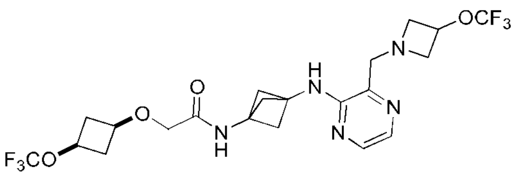

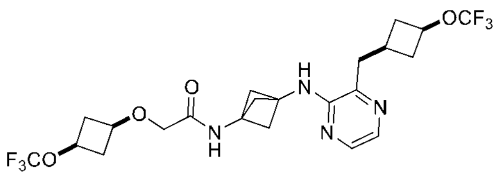

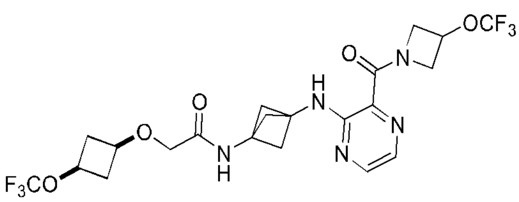

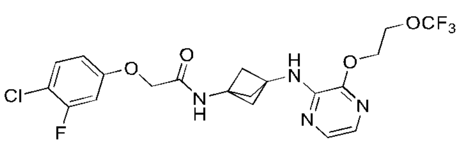

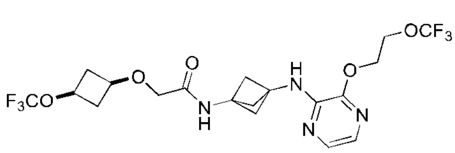

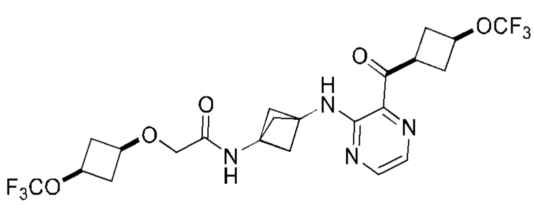

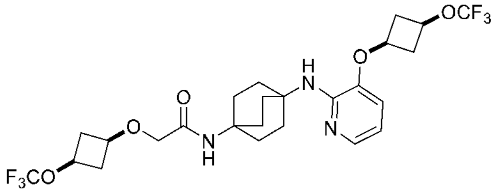
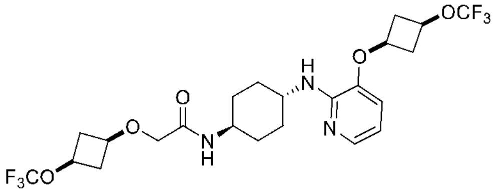

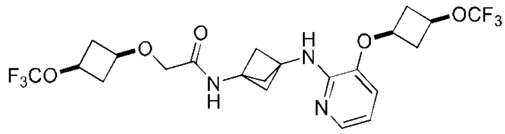
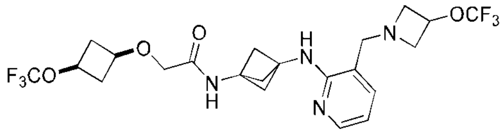

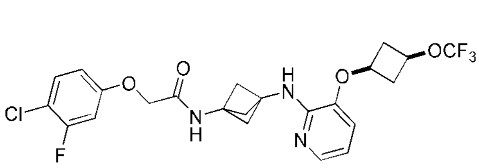
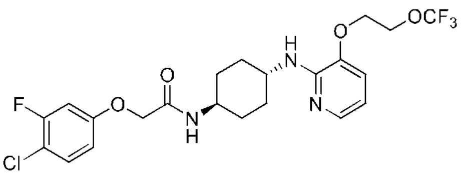

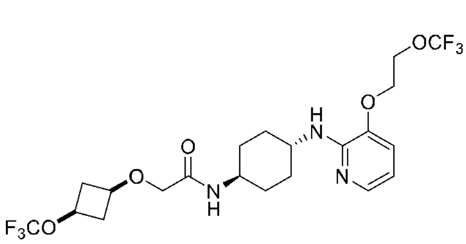
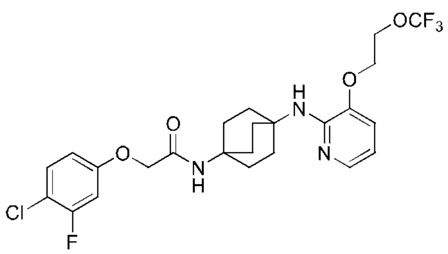

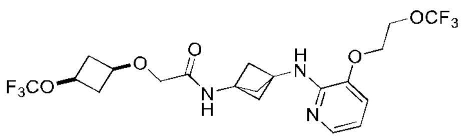
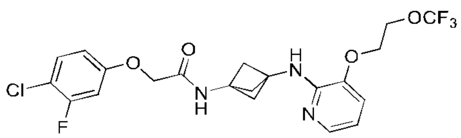

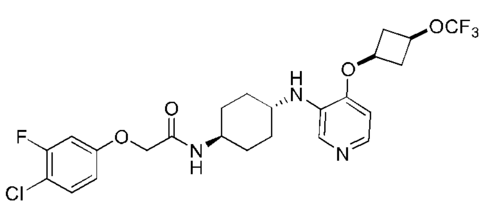
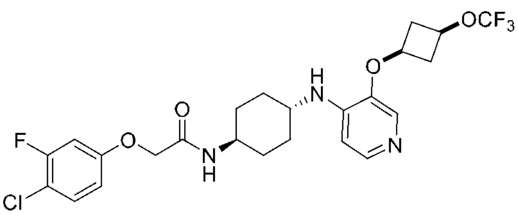

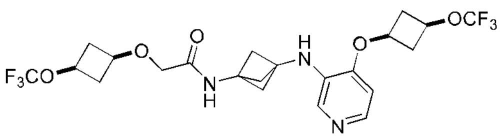
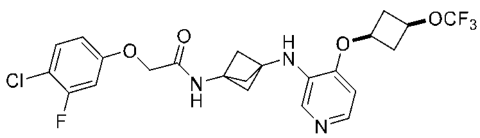

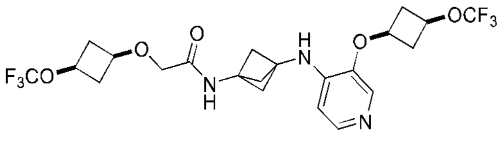
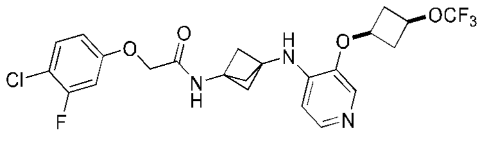

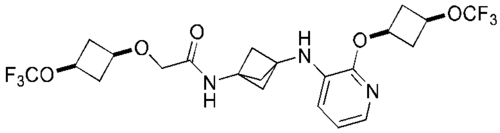
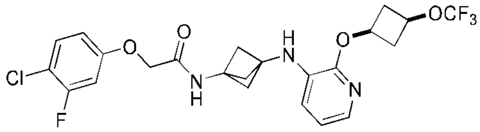
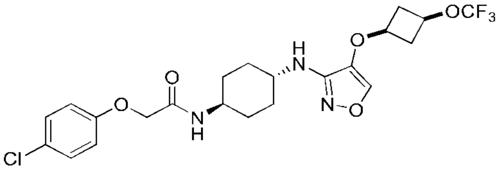
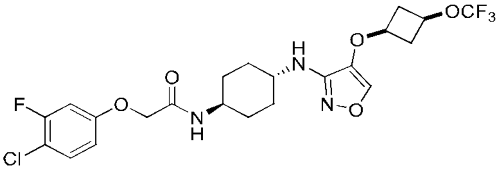
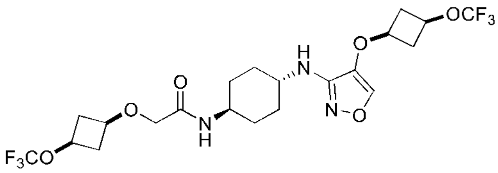
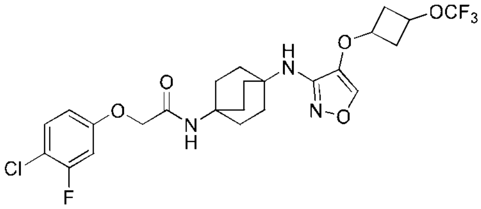
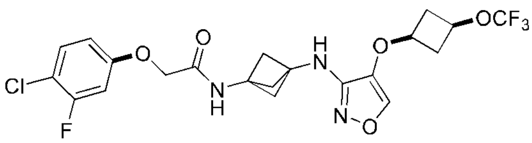
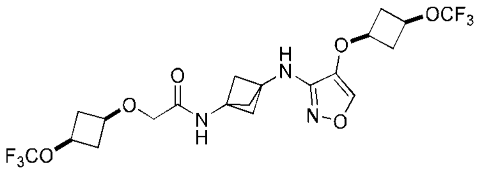
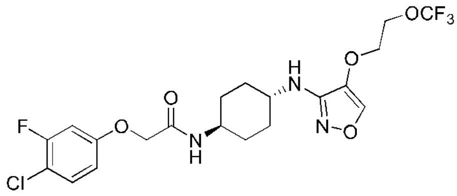
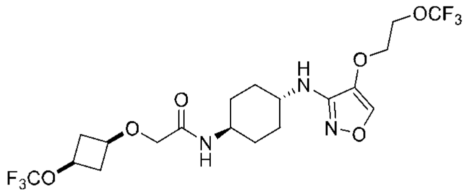
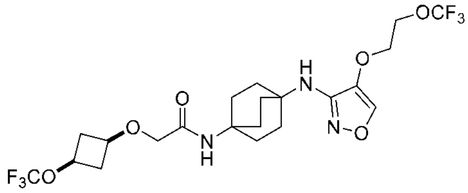
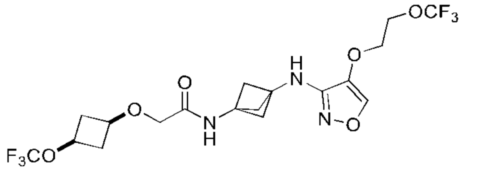
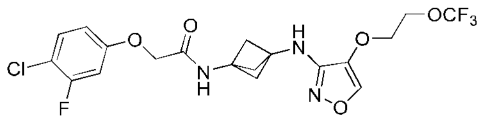
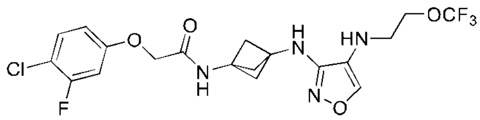
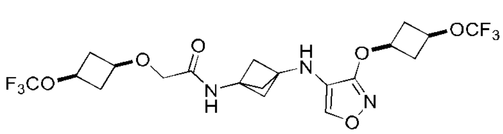
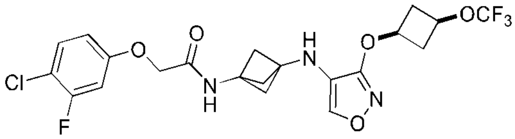

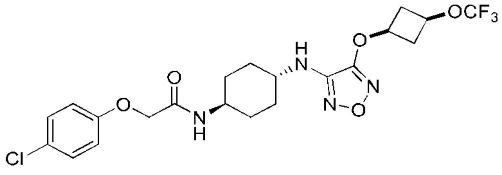

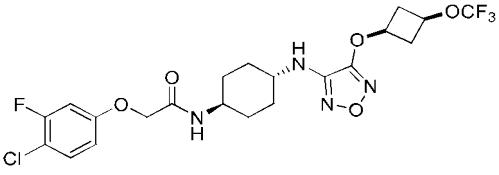

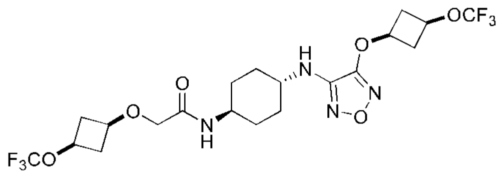

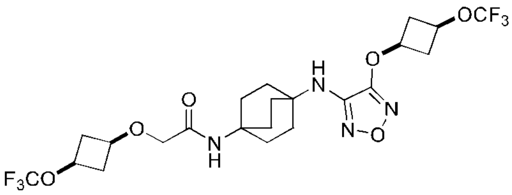

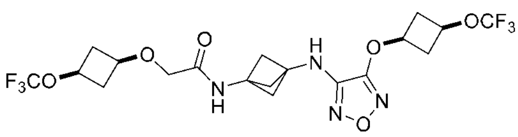

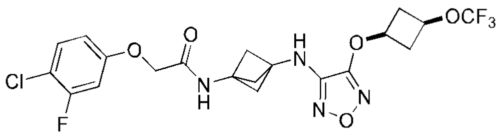

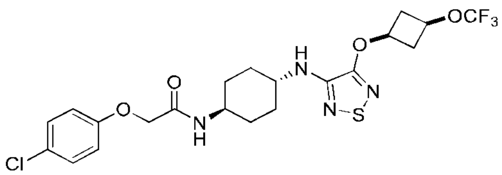

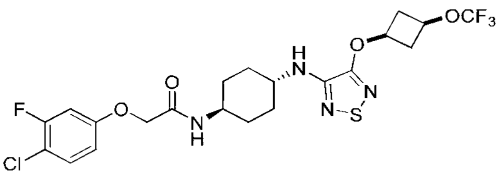

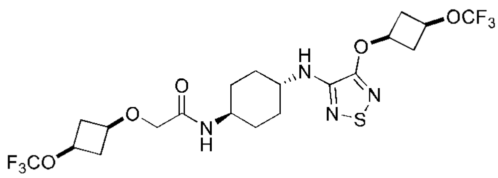

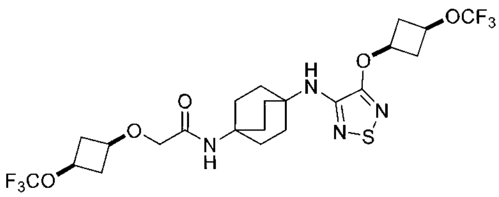

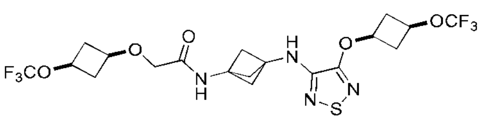

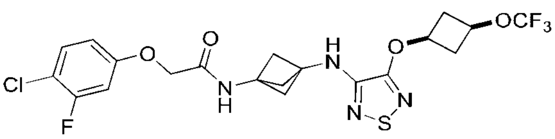

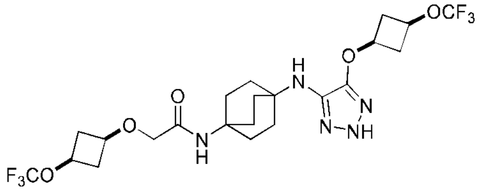

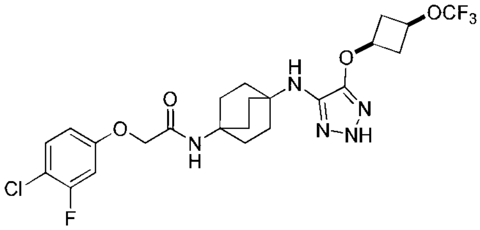

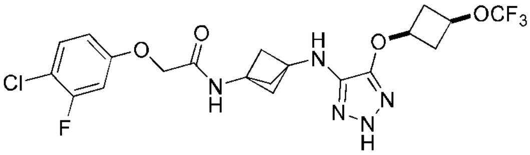

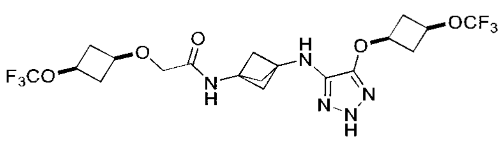

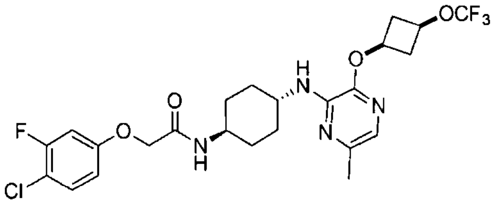

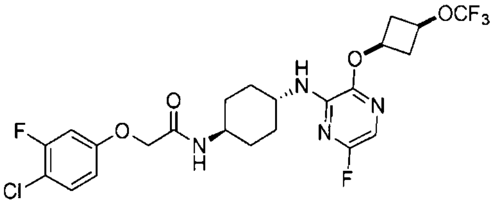

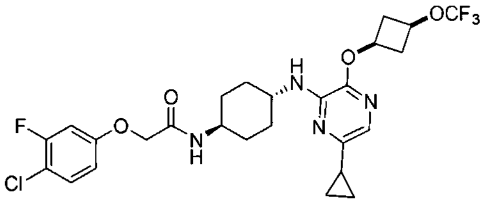

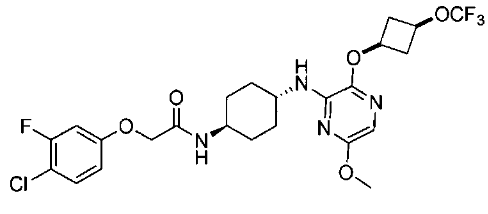

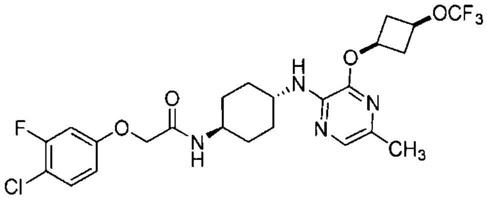

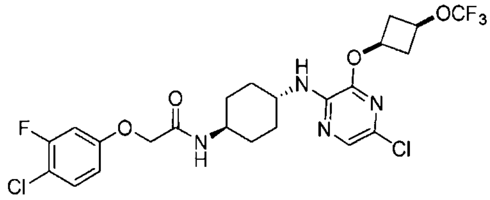

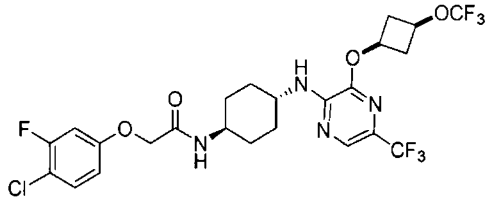

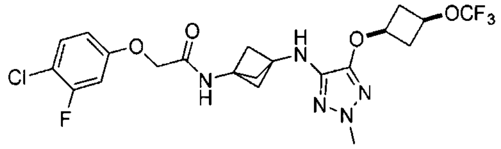

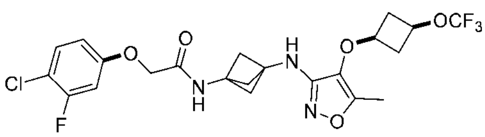

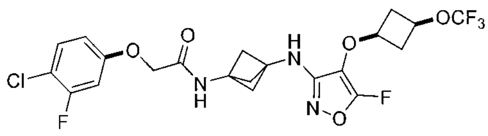

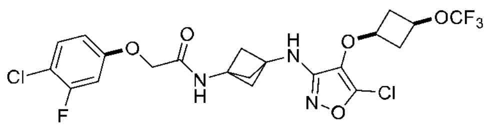

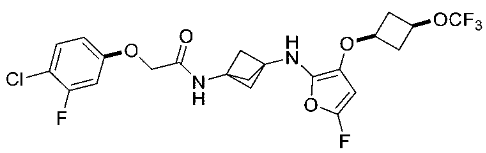

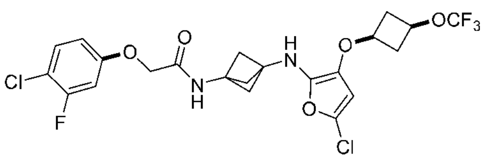

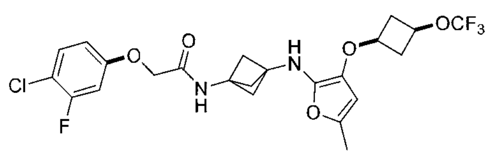

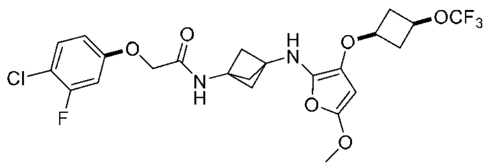

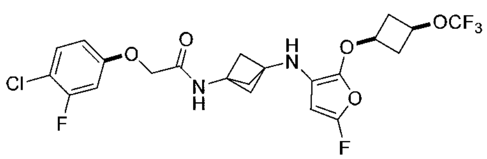

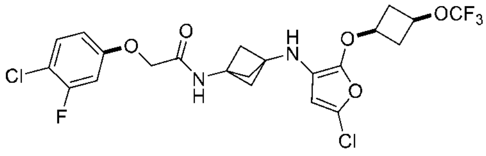

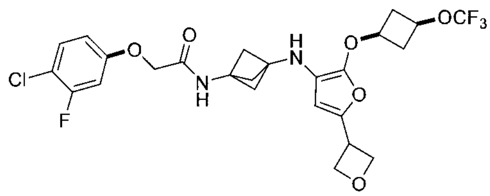

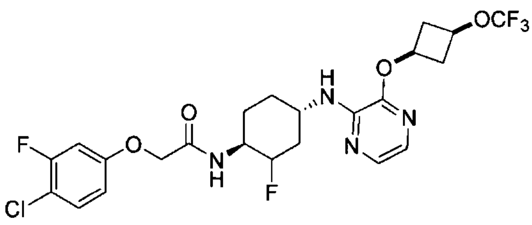

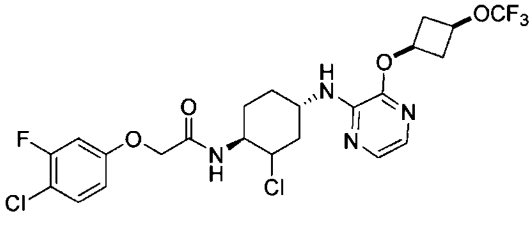

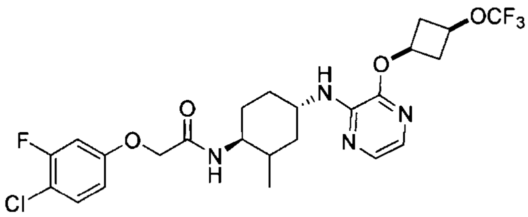

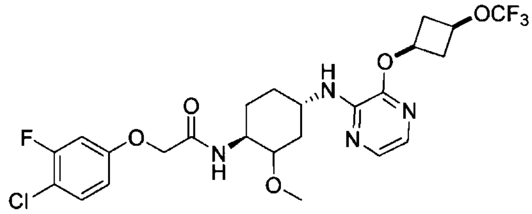

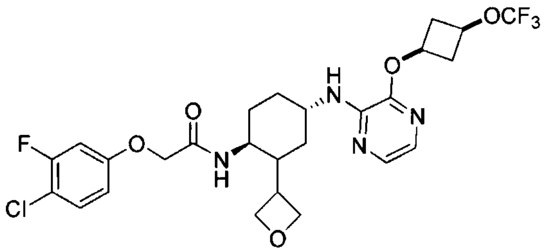

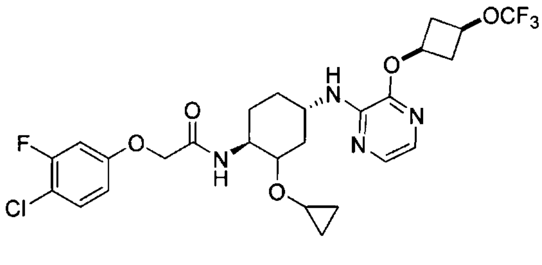

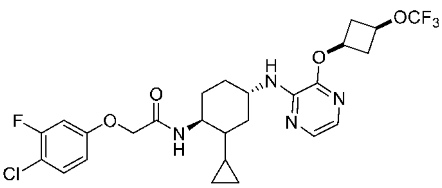

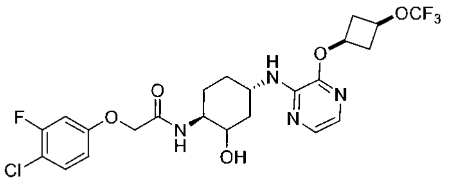

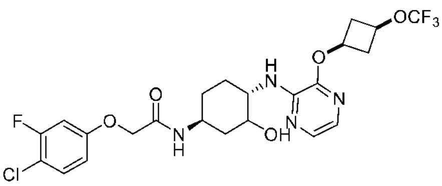

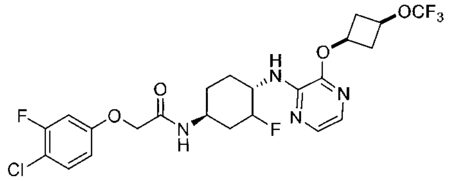

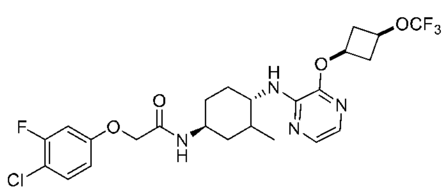

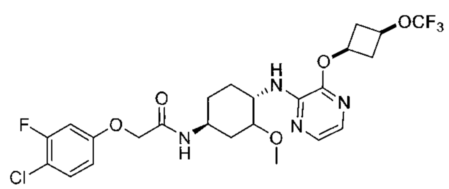

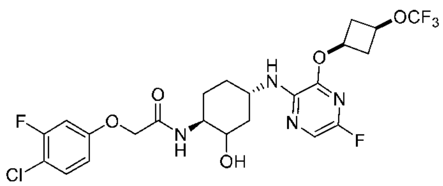

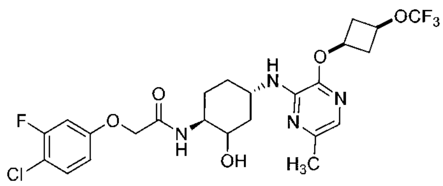

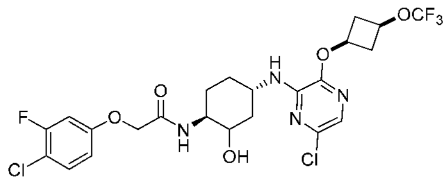

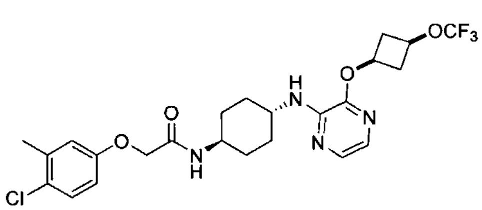

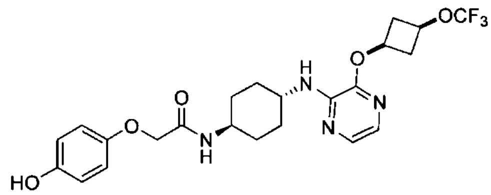

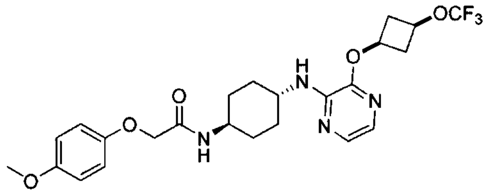

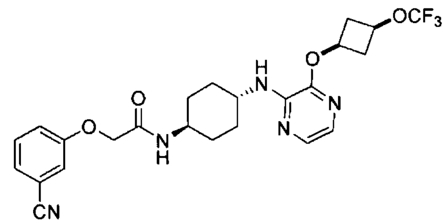

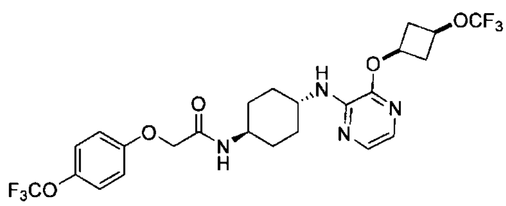

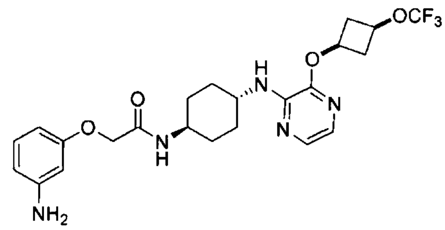

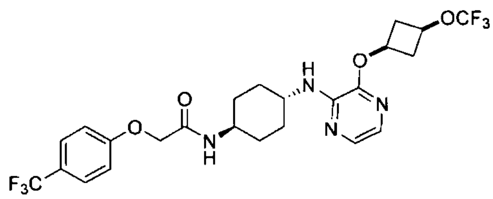

According to a second aspect, the present application further provides a synthesis method for compounds with the structures shown in above Formulas V-1 to V-3, including:

bringing raw material 1

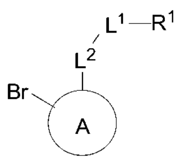

to undergo a substitution reaction with raw material 2

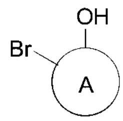

to afford intermediate 3, or bringing raw material 4

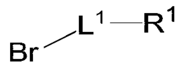

to undergo a substitution reaction with raw material 5

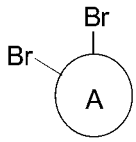

to afford intermediate 3, so as to synthesize the intermediate 3

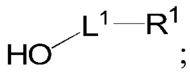

synthesizing the compound with the structure shown in Formula V-1 according to Scheme 1,

Scheme 1:

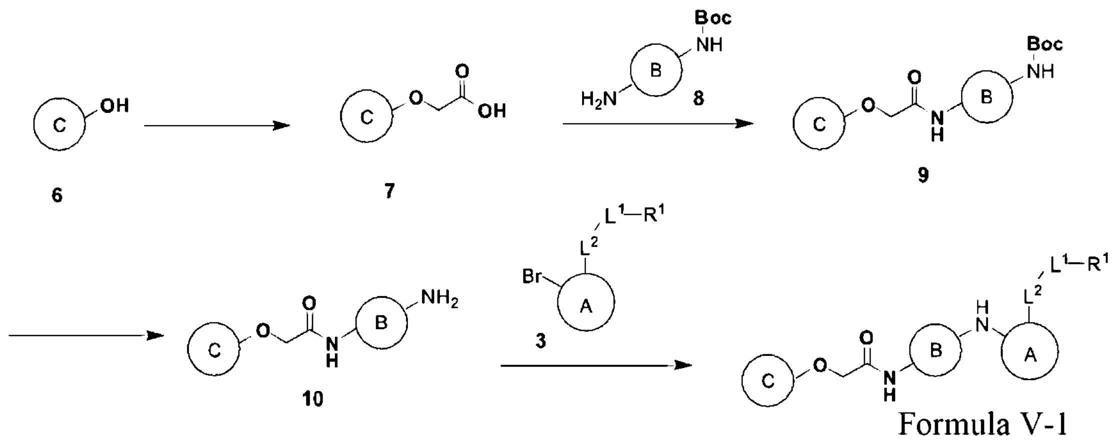

Formula V-1

where raw material 6, after undergoing a substitution reaction with methyl bromoacetate, was hydrolyzed to afford intermediate 7, which was then subjected to an amidation reaction with raw material 8 to give intermediate 9; then, the intermediate 9 was deprotected to provide intermediate 10; and the compound with the structure shown in Formula V-1 was obtained via a coupling reaction between the intermediate 10 and the intermediate 3;

synthesizing the compound with the structure shown in Formula V-2 according to Scheme 2,

Scheme 2:

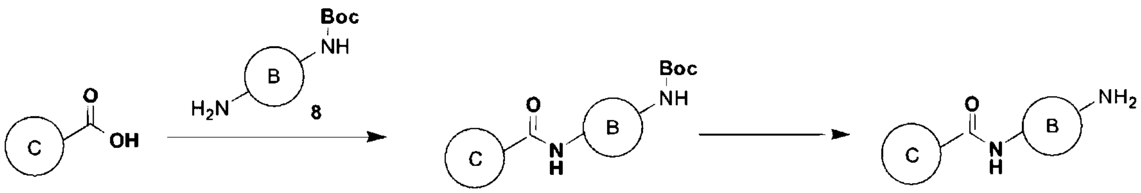

8

11 12 13

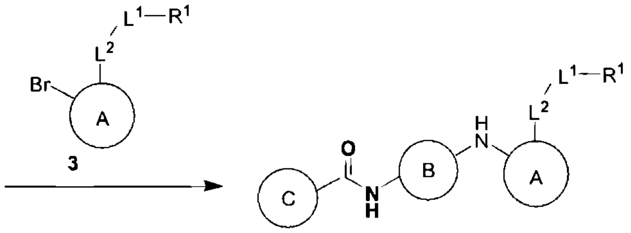

3

Formula V-2

where raw material 11 was subjected to an amidation reaction with the raw material 8 to give intermediate 12; then, the intermediate 12 was deprotected to provide intermediate 13; and the compound with the structure shown in Formula V-2 was obtained via a coupling reaction between the intermediate 13 and the intermediate 3; and

synthesizing the compound with the structure shown in Formula V-3 according to Scheme 3,

Scheme 3:

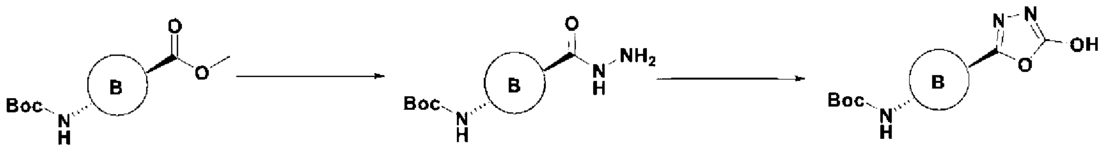

14 15 16

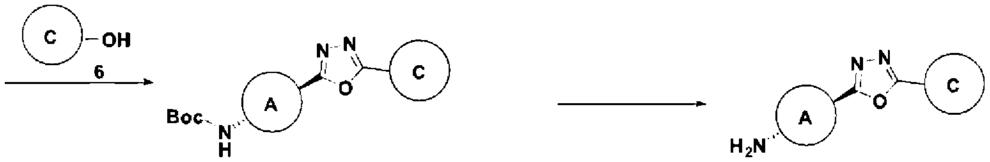

6

17 18

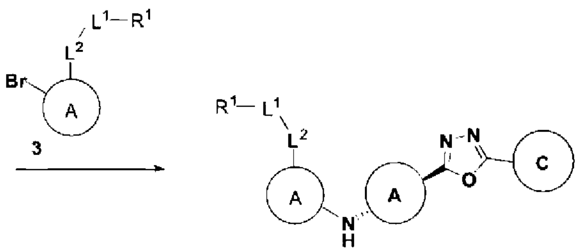

3

Formula V-3

where raw material 14 was reacted with hydrazine hydrate, was subjected to functional group transformation to give

intermediate 15, which was subjected to cyclization reaction with N,N-carbonyl diimidazole to give intermediate 16; then, condensation reaction was conducted between the intermediate 16 and the raw material 6 to afford intermediate 17, which was deprotected to provide intermediate 18; and the compound with the structure shown in Formula V-2 was obtained by coupling the intermediate 18 with the intermediate 3.

According to a third aspect, the present application provides a pharmaceutical composition comprising a preparation prepared from any one of the compounds, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof according to the first aspect.

In some embodiments, the pharmaceutical composition described above further includes a pharmaceutically acceptable carrier, adjuvant and vehicle.

According to a fourth aspect, the present application provides uses of the compound, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof according to the first aspect, and the pharmaceutical composition provided in the second aspect in the manufacture of a medicament for preventing and/or treating a neurodegenerative disease, cancer, inflammatory disease, autoimmune disease, virus infection, skin disease, fibrotic disease, hemoglobinopathy, kidney disease, hearing impairment, ophthalmic disease, disease caused by mutations that induce unfolded protein response (UPR), malaria infection, musculoskeletal disease, metabolic disease or mitochondrial disease.

According to a fifth aspect, the present application provides uses of the compound, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof according to the first aspect, and the pharmaceutical composition provided in the third aspect in the manufacture of a medicament for preventing and/or treating a disease or disorder mediated by an integrated stress response (ISR) pathway.

In some embodiments, provided herein is a method for treating a disease or disorder mediated by an integrated stress response (ISR) pathway in a subject in need thereof, including administering to the subject a therapeutically effective amount of any one of the compound, or stereoisomers, pharmaceutically acceptable salts, tautomers, polymorphs, solvates, hydrates, N-oxides, isotopically labeled compounds, metabolites, esters, or prodrugs thereof described above, or a therapeutically effective amount of any one of the pharmaceutical compositions described above.

In some embodiments, provided herein is a method for treating a disease related to modulation of the activity or level of eIF2B or that of eIF2 or ISR pathway, including administering to a subject a therapeutically effective amount of any one of the compound, or stereoisomers, pharmaceutically acceptable salts, tautomers, polymorphs, solvates, hydrates, N-oxides, isotopically labeled compounds, metabolites, esters, or prodrugs thereof described above, or a therapeutically effective amount of any one of the pharmaceutical compositions described above.

In some embodiments, provided herein is a method for preventing and/or treating any one of the diseases described above, including administering to a subject in need thereof an effective amount of any one of the compound, or stereoisomers or pharmaceutically acceptable salts thereof, or the pharmaceutical composition.

In some embodiments, provided herein is a method for preventing and/or treating a cancer, including administering to a subject in need thereof an effective amount of any one of the compound, or stereoisomers or pharmaceutically acceptable salts thereof, or the pharmaceutical composition.

In some embodiments, the neurodegenerative disease includes, but is not limited to leukodystrophy, leukoencephalopathy, dysmyelination or demyelinating disease, intellectual disability syndrome, cognitive dysfunction, neurogliocyte dysfunctionor brain injury (for example, traumatic brain injury or toxin-induced brain injury), Alexander's disease, Alper's disease, Alzheimer's disease, amyotrophic lateral sclerosis (ALS), ataxia telangiectasia, Batten disease (also referred to as Spielmeyer-Vogt-Sjogren-Batten disease), bovine spongiform encephalopathy (BSE), Canavan disease, Cockayne syndrome, corticobasal Degeneration, Creutzfeldt-Jakob disease, dystonia, frontotemporal dementia (FTD), Gerstmann-Straussler-Scheinker syndrome, Huntington's disease, HIV-associated dementia, Kennedy's disease, Krabbe disease, kuru disease, Lewy body dementia, Machado-Joseph disease (spinocerebellar ataxia type 3), multiple system atrophy, multisystem proteinopathy, narcolepsy, Neuroborreliosis, Parkinson's disease, Pelizaeus-Merzbacher Disease, Pick's disease, primary lateral sclerosis, Prion disease, Refsum's disease, Sandhoff disease, Schilder's disease, Subacute combined degeneration of spinal cord secondary to Pernicious Anaemia, schizophrenia, spinocerebellar ataxia (with various types characterized by different features, for example, spinocerebellar ataxia type 2 or 8), spinal muscular atrophy, Steele-Richardson-01szewski disease, progressive supranuclear palsy, corticobasal degeneration, adrenoleukodystrophy, X-linked adrenoleukodystrophy, cerebral adrenoleukodystrophy, Pelizaeus-Merzbacher disease, Krabbe disease, or leukodystrophy caused by mutations in DARS2 gene (sometimes referred to as leukoencephalopathy with brain stem and spinal cord involvement and lactate elevation (LBSL)), DARS2 related spectrum disorders or Tabes dorsalis.

The cancer mentioned above includes, but is not limited to: a human cancer and carcinoma, sarcoma,

adenocarcinoma, lymphoma, leukemia, melanoma and the like, including solid carcinoma and lymphatic cancer, kidney cancer, breast cancer, lung cancer, bladder cancer, colon cancer, ovarian cancer, prostate cancer, pancreatic cancer, gastric cancer, brain cancer, head and neck cancer, skin cancer, uterine cancer, testicular cancer, glioma, esophageal cancer, liver cancer (including hepatocarcinoma), lymphoma (including acute B lymphoblastic leukemia, non-Hodgkin's lymphoma (for example, Burkitt's lymphoma, small cell lymphoma and large cell lymphoma)), Hodgkin's Lymphoma, leukemia (including AML, ALL and CML) and/or multiple myeloma. In some other instance, "cancer" refers to a lung cancer, breast cancer, ovarian cancer, leukemia, lymphoma, melanoma, pancreatic cancer, sarcoma, bladder cancer, osteocarcinoma, brain cancer, cervical cancer, colon cancer, esophageal cancer, gastric cancer, liver cancer, head and neck cancer, kidney cancer, myeloma, thyroid cancer, prostate cancer, metastatic cancer or carcinoma.

The leukemia mentioned above includes, but is not limited to: an acute non-lymphocytic leukemia, chronic lymphocytic leukemia, acute granulocytic leukemia, chronic granulocytic leukemia, acute promyelocytic leukemia, adult T-cell leukemia, non-leukemic leukemia, aleukemic leukemia, basophilic leukemia, blastic leukemia, bovine leukemia, chronic myelocytic leukemia, leukemia cutis, stem cell associated leukemia, eosinophilic leukemia, Gross' leukemia, hairy cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymph-derived leukemia, lymphoid leukemia, lymphosarcoma cell leukemia, mast cell leukemia, megakaryocytic leukemia, small myeloblastic leukemia, monocytic leukemia, myeloblastic leukemia, myelocytic leukemia, myeloid granulocytic leukemia, myelomonocytic leukemia, Naegeli leukemia, plasma cell leukemia, multiple myeloma, plasmacytic leukemia, promyelocytic leukemia, Rieder cell leukemia, Schilling's leukemia, stem cell leukemia, subleukemic leukemia or undifferentiated cell leukemia.

The inflammatory disease mentioned above includes, but is not limited to: postoperative cognitive dysfunction, arthritis (for example, rheumatoid arthritis, psoriatic arthritis, juvenile idiopathic arthritis), systemic lupus erythematosus (SLE), myasthenia gravis, Juvenile-onset diabetes, Type 1 diabetes, Guillain-Barré syndrome, Hashimoto's encephalitis, Hashimoto's thyroiditis, ankylosing spondylitis, psoriasis, Sjogren's syndrome, vasculitis, glomerulonephritis, autoimmune thyroiditis, Behcet's disease, Crohn's disease, ulcerative colitis, bullous pemphigoid, sarcoidosis, ichthyosis, Graves' ophthalmopathy, inflammatory bowel disease, Addison's disease, vitiligo, asthma (for example, allergic asthma), acne vulgaris, celiac disease, chronic prostatitis, pelvic inflammatory disease in inflammatory bowel disease, reperfusion injury, sarcoidosis, transplant rejection, interstitial cystitis, atherosclerosis and atopic dermatitis.

The musculoskeletal disease mentioned above includes, but is not limited to: muscular dystrophy(for example, Duchenne muscular dystrophy, Becker muscular dystrophy, distal muscular dystrophy, congenital muscular dystrophy, Emery-Dreifuss muscular dystrophy, facioscapulohumeral muscular dystrophy, myotonic dystrophy type 1 or myotonic dystrophy type 2), limb-girdle muscular dystrophy, multi-system protein denaturation, rhizomelic chondrodysplasia punctata, X-linked recessive chondrodysplasia punctata, Conradi-Hiinermann syndrome, autosomal chondrodysplasia punctata, stress-induced bone disease (for example, stress-induced osteoporosis), multiple sclerosis, amyotrophic lateral sclerosis (ALS), primary lateral sclerosis, progressive muscular atrophy, progressive bulbar palsy, pseudobulbar palsy, spinal muscular atrophy, progressive spinobulbar muscular atrophy, myelospasm, spinal muscular atrophy, myasthenia gravis, neuralgia, fibromyalgia, Machado-Joseph disease, Paget's disease of bone, spasmodic amyostasia syndrome, Freidrich's ataxia, muscle wasting disorder (for example, muscle atrophy, sarcopenia, cachexia), inclusion body myopathy, motor neuron disease or paralysis.

The metabolic disease mentioned above includes, but is not limited to: non-alcoholic steatohepatitis (NASH), non-alcoholic fatty liver disease(NAFLD), hepatic fibrosis, obesity, heart disease, atherosclerosis, arthritis, cystinosis, diabetes (for example, Type 1 diabetes, Type 2 diabetes or gestational diabetes), phenylketonuria, proliferative retinopathy or Kearns-Sayre disease.

The mitochondrial disease mentioned above includes, but is not limited to: Barth syndrome, chronic progressive external ophthalmoplegia (cPEO), Kearns-Sayre Syndrome (KSS), Leigh syndrome (for example, MILS or maternally inherited Leigh syndrome), mitochondrial DNA depletion syndrome (MDDS, such as Alpers syndrome), mitochondrial encephalomyopathy (for example, mitochondrial encephalomyopathy with lactic acidosis and stroke-like episodes (MELAS)), mitochondrial neurogastrointestinal encephalomyopathy (MNGIE), myoclonus epilepsy with ragged red fibers (MERRF), neuropathy, ataxia, neuropathy-ataxia-retinitis pigmentosa(NARP), Leber's hereditary optic neuropathy, LHON and Pearson syndrome.

The hearing impairment mentioned above includes, but is not limited to: mitochondrial nonsyndromic hearing loss and deafness, hair cell death, age-related hearing loss, noise-induced hearing loss, inherited or hereditary hearing loss, hearing loss caused by ototoxicity exposure, disease-related hearing loss and traumatic hearing loss. In some embodiments, the mitochondrial nonsyndromic hearing loss and deafness is MT-RNR1 related hearing loss.

The ophthalmic disease mentioned above includes, but is not limited to: cataract, glaucoma, endoplasmic reticulum (ER) stress, autophagic deficiency, age-related macular degeneration (AMD) or diabetic retinopathy.

The kidney disease mentioned above includes, but is not limited to: Abderhalden-Kaufmann-Lignac syndrome (Y disease type cystinosis), abdominal compartment syndrome, acetaminophen-induced nephrotoxicity, acute renal failure/acute kideny injury, acute lobar nephropathy, acute phosphate nephropathy, acute tubular necrosis, adenine phosphoribosyl transferase deficiency, adenovirus nephritis, Alagille Syndrome, Alport Syndrome, amyloidosis, ANCA vasculitis associated with endocarditis and other infections, angiomyolipoma, analgesic nephropathy, anorexia nervosa-related nephropathy, angiotensin antibody-related and focal segmental glomerulosclerosis, antiphospholipid syndrome, anti-TNF-$\alpha$ therapy-related glomerulonephritis, APOL1 mutation, apparent mineralocorticoid excess, aristolochic acid nephropathy, Chinese herb nephropathy, Balkan Endemic Nephropathy, urinary arteriovenous malformation and fistula, autosomal dominant hypocalcaemia, Bardet-Biedl Syndrome, Bartter Syndrome, bath salt-induced acute injury, Beer Potomania, beeturia, Beta thalassemia nephropathy, bile cast nephropathy, autologous BK polyomavirus nephropathy, bladder rupture, bladder sphincter dyssynergia, bladder tamponade, Border-Crossers' Nephropathy, Bourbon virus-related acute kidney injury, burn type cane harvesting-associated acute renal dysfunction, Byetta renal failure, Clq nephropathy, C3 glomerulopathy, C3 glomerulopathy with monoclonal gammopathy, C4 glomerulopathy, calcineurin inhibitor nephrotoxicity, Callilepsis Laureola poisoning, Cannabinoid reduced hyperemesis type acute renal failure, cardiorenal syndrome, Carfilzomib-induced kidney injury, CFHR5 nephropathy, Charcot-Marie-Tooth disease with glomerulopathy, herbal nephrotoxicity, cherry concentrate-induced acute kidney injury, cholesterol embolism, Churg-Strauss syndrome, chyluria, ciliopathy, cocaine nephropathy, cold diuresis, colistin nephrotoxicity, collagenous glomerulopathy, collapsing glomerulopathy, CMV-associated collapsing glomerulopathy, combination antiretroviral therapy (cART)-associated nephropathy, congenital anomalies of the kidney and urinary tract (CAKUT), congenital nephrotic syndrome, congestive renal failure, nephrotic syndrome with cone-shaped epiphyses (Mainzer-Saldino syndrome or Saldino-Mainzer disease), contrast-induced nephropathy, copper sulfate poisoning, cortical necrosis, Ccrizotinib-associated acute kidney injury, cryoglobulinemia with crystalline deposits, cryoglobulinemia, crystal globulin-induced nephropathy, crystal-induced acute kidney injury, crystal-storing histiocytosis, acquired renal cystic disease, cystinuria, Dasatinib-induced nephrotic proteinuria, dense deposit disease (MPGN Type 2), Dent Disease (X-linked recessive nephrolithiasis), DHA crystalline nephropathy, dialysis disequilibrium syndrome, diabetes and diabetic nephropathy, diabetic insipidus, dietary supplement-induced renal failure, diffuse mesangial sclerosis, diuresis, Djenkol Bean poisoning (Djenkolism), Down syndrome-related kidney disease, drug abuse nephropathy, duplication of ureter, EAST syndrome, Ebola-related nephropathy, ectopic kidney, ectopic ureter, edema, swelling, Erdheim-Chester disease, Fabry's disease, familial hypocalciuric hypercalcemia, Fanconi syndrome, Fraser syndrome, fibronectin glomerulopathy, fibrillary glomerulonephritis and immunotactoid glomerulopathy, Fraley syndrome, fluid volume excess, hypervolemia, focal segmental glomerulosclerosis, focal sclerosis, focal glomerulosclerosis, Galloway Mowat syndrome, giant cell arteritis with renal involvement, hypertensive disorders of pregnancy, Gitelman syndrome, glomerular diseases, glomerulotubular reflux, diabetes mellitus, Goodpasture Syndrome, Green Smoothie Cleanse Nephropathy, HANAC syndrome, Harvoni (Ledipasvir and Sofosbuvir)-induced kidney injury, acute kidney injury due to hair dye ingestion, Hantavirus infection podocytopathy, heat stress nephropathy, hematuria (urine containing blood), hemolytic uremic syndrome(HUS), atypical hemolytic uremic syndrome (aHUS), hemophagocytic syndrome, hemorrhagic cystitis, hemorrhagic fever with renal syndrome (HFRS, hantavirus nephropathy, Korean haemorrhagic fever, epidemic hemorrhagic fever, nephropathis epidemica), hemosiderinuria, hemosiderosis associated with paroxysmal nocturnal hemoglobinuria and hemolytic anemia, hepatorenal glomerulopathy, hepatic veno-occlusive disease, sinusoidal obstruction syndrome, HCV-associated renal disease, hepatocyte nuclear factor 1B-related renal disease, hepatorenal syndrome, herbal supplement-associated renal disease, high Altitude Renal Syndrome, hypertensive renal disease, HIV-associated immune complex kidney disease (HIVICK), HIV-associated nephropathy (HIVAN), HNF1B-associated autosomal dominant tubulointerstitial kidney disease, horseshoe kidney (renal fusion), Hunner's ulcer, hydroxychloroquine-induced phospholipidosis, hyperaldosteronism, hypercalcemia, hyperkalemia, hypermagnesemia, hypernatremia, hyperoxaluria, hyperphosphatemia, hypocalcemia, hypocomplementemic urticarial vasculitis syndrome, hypokalemia, hypokalemia-induced renal dysfunction, hypokalemic periodic paralysis, hypomagnesemia, hyponatremia, hypophosphatemia, hypophosphatemia in hashishin, hypertension, monogenic hypertension, ice tea nephropathy, ifosfamide nephrotoxicity, IgA nephropathy, IgG4 nephropathy, immersion diuresis, immune checkpoint therapy-associated interstitial nephritis, Infliximab-related nephropathy, interstitial cystitis, bladder pain syndrome (questionnaire), interstitial nephritis, megakaryocytic interstitial nephritis, Ivemark's syndrome, JC virus nephropathy, Joubert Syndrome, ketamine-associated bladder dysfunction, kidney stone, nephrolithiasis, Kombucha tea toxicity, lead nephropathy and lead nephrotoxicity, lecithin-cholesterol acyltransferase deficiency (LCAT deficiency), leptospiral nephropathy, light chain deposition disease, monoclonal immunoglobulin deposition disease, light chain proximal tubulopathy, Liddle syndrome, Lightwood-Albright syndrome, lipoprotein glomerulopathy, lithium nephrotoxicity, LMX1B mutation-induced hereditary FSGS, loin pain and haematuria syndrome, lupus, systemic lupus erythematosus, lupus kidney disease, lupus nephritis, Lupus nephritis with positive anti-neutrophil cytoplasmic antibody serum, lupus podocytopathy, glomerulonephritis associated with Lyme disease, lysinuric protein intolerance, lysozyme renal disease, malarial nephropathy, malignancy-associated nephropathy, malignant hypertension, malakoplakia, McKittrick-Wheelock syndrome, MDMA (Molly; Ecstacy; 3,4-methylenedioxymethyl amphetamine)-related renal failure, urethral stricture, medullary cystic kidney disease, uromodulin-associated kidney disease, juvenile hyperuricemic nephropathy type 1, medullary sponge kidney, megaureter, melamine toxicity induced nephropathy, MELAS syndrome, membranoproliferative glomerulonephritis, membranous nephropathy, membranous glomerulopathy with occult IgGk deposits, Meso American nephropathy, metabolic acidosis, metabolic alkalosis, methotrexate-related renal failure, microscopic polyangiitis, milk-alkali syndrome, minimal change disease, monoclonal gammopathy of renal significance, dysproteinemia, mouthwash toxicity, MUC1-associated kidney disease, multicystic dysplastic kidney, multiple myeloma, myeloproliferative neoplasm-associated glomerulopathy, nail-patella syndrome, NARP syndrome, nephrocalcinosis, nephrogenic systemic fibrosis, nephroptosis (floating kidney or renal ptosis), nephrotic syndrome, neurogenic bladder, 9/11 and nephrosis, nodular glomerulosclerosis, non-gonococcal urethritis, nutcracker syndrome, oligomeganephronia, orofaciodigital syndrome, orotic aciduria, orthostatic hypotension, orthostatic proteinuria, osmotic diuresis, osmotic nephropathy, ovarian hyperstimulation syndrome, oxalate nephropathy, Page kidney, papillary necrosis, papillorenal syndrome (renal coloboma syndrome, unilateral renal agenesis), PARN mutation nephropathy, parvovirus B19-associated kidney disease, peritoneo-renal syndrome, posterior urethral valve POEMS syndrome, podocytic infolding glomerulopathy, postinfectious glomerulonephritis, poststreptococcal glomerulonephritis, atypical postinfectious glomerulonephritis, postinfectious glomerulonephritis (IgA dominant), IgA-like nephropathy, polyarteritis nodosa, posterior urethral valve polycystic kidney disease, post-obstructive diuresis, pre-eclampsia, propofol infusion syndrome, proliferative glomerulonephritis with monoclonal IgG deposits (Nasr disease), propolis (bees resin)-related renal failure, proteinuria (urine containing protein), pseudo-hyperaldosteronism, pseudo-hypobicarbonatemi, pseudo-hypoparathyroidism, Goodpasture syndrome, pyelonephritis (kidney infection), pyonephrosis, pyridium-induced renal failure, radiation nephropathy, ranolazine-induced nephropathy, refeeding syndrome, reflux nephropathy, rapidly progressive glomerulonephritis, nephrapostasis, perirenal abscess, renal hypoplasia, acute kidney injury associated with renal arcuate vein microthrombi, aneurysm of renal artery, spontaneous renal artery dissection, renal artery stenosis, renal cell carcinoma, renal cyst, exercise-induced acute renal failure with renal hypouricemia, renal infarction, renal osteodystrophy, renal tubular acidosis, renin mutated and autosomal dominant tubular-interstitial nephropathy, renin-secreting tumor (juxtaglomerular cell tumor), reset osmostat, postcaval ureter, retroperitoneal fibrosis, rhabdomyolysis, bariatric surgery-associated rhabdomyolysis, rheumatoid arthritis-associated nephropathy, sarcoidosis-related kidney disease, renal and cerebral salt wasting, schistosomal glomerulopathy, Schimke immuno-osseous dysplasia, scleroderma renal crisis, serpentine fibula-polycystic kidney syndrome, Exner syndrome, sickle cell nephropathy, silica exposure-induced chronic kidney disease, Sri Lankan farmers' kidney disease, Sjögren's syndrome-related kidney disease, synthetic cannabinoid-induced acute kidney injury, hematopoietic cell transplantation-associated nephropathy, stem cell transplantation-related kidney disease, TAFRO syndrome, tea and toast hyponatremia, tenofovir-induced nephrotoxicity, thin basement membrane disease, benign familial hematuria, monoclonal gammopathy-associated ranolazine-induced nephropathy, refeeding syndrome, reflux nephropathy, rapidly progressive glomerulonephritis, nephrapostasis, perirenal abscess, renal hypoplasia, acute kidney injury associated with renal arcuate vein microthrombi, aneurysm of renal artery, spontaneous renal artery dissection, renal artery stenosis, renal cell carcinoma, renal cyst, exercise-induced acute renal failure with renal hypouricemia, renal infarction, renal osteodystrophy, renal tubular acidosis, renin mutated and autosomal dominant tubular-interstitial nephropathy, renin-secreting tumor (juxtaglomerular cell tumor), reset osmostat, postcaval ureter, retroperitoneal fibrosis, rhabdomyolysis, bariatric surgery-associated rhabdomyolysis, rheumatoid arthritis-associated nephropathy, sarcoidosis-related kidney disease, renal and cerebral salt wasting, schistosomal glomerulopathy, Schimke immuno-osseous dysplasia, scleroderma renal crisis, serpentine fibula-polycystic kidney syndrome, Exner syndrome, sickle cell nephropathy, silica exposure-induced chronic kidney disease, Sri Lankan farmers' kidney disease, Sjögren's syndrome-related kidney disease, synthetic cannabinoid-induced acute kidney injury, hematopoietic cell transplantation-associated nephropathy, stem cell transplantation-related kidney disease, TAFRO syndrome, tea and toast hyponatremia, tenofovir-induced nephrotoxicity, thin basement membrane disease, benign familial hematuria, monoclonal gammopathy-associated thrombotic microangiopathy, war nephritis, trigonitis, genitourinary tuberculosis, tuberous sclerosis, renal tubular dysgenesis, immune complex tubulointerstitial nephritis due to anti-proximal tubule brush border autoantibody, tumor lysis syndrome, uremia, uremic optic neuropathy, cystic ureteritis, ureteral hernia, urethral caruncle, urethral stricture, urinary incontinence, urinary tract infection, ureteral obstruction, urogenital fistula, uromodulin-associated nephropathy, vancomycin-induced cast nephropathy, vasomotor nephropathy, enterovesical fistula, vesicoureteral reflux, VGEF inhibitory renal thrombotic microangiopathy, volatile anesthetic-induced acute kidney injury, Von Hippel-Lindau disease, Waldenstrom's macroglobulinemic glomerulonephritis, Warfarin-related neuropathy, wasp sting-induced acute kidney injury, Wegener's granulomatosis, granulomatosis with polyangiitis, West Nile virus-induced chronic kidney disease, Wunderlich syndrome, Zellweger syndrome or ceribro-hepatorenal syndrome.

The skin disease mentioned above includes, but is not limited to: acne vulgaris, alopecia areata, basal cell carcinoma, Bowen's disease, congenital erythropoietic porphyria, contact dermatitis, Darier's disease, disseminated superficial actinic porokeratosis, dystrophic epidermolysis bullosa, eczema (atopic eczema), extramammary Paget's disease, epidermolysis bullosa simplex, erythropoietic Protoporphyria, fungal infection of nails, Hailey-Hailey disease, herpes simplex, hidradenitis suppurativa, hirsutism, polyhidrosis, ichthyosis, impetigo, keloid, keratosis pilaris, lichen planus, lichen sclerosus, melanoma, melanoderma, mucous membrane pemphigoid, pemphigoid, pemphigus vulgaris, pityriasis lichenoides, pityriasis rubra pilaris, plantar wart (wart), polymorphous light eruption, psoriasis, plaque psoriasis,

pyoderma gangrenosum, rosacea, scabies, scleroderma, herpes zoster, squamous cell carcinoma, sweet's syndrome, urticaria, angioedema and vitiligo.

The fibrotic disease mentioned above includes, but is not limited to: adhesive capsulitis, arterial stiffness, articular fibrosis, atrial fibrosis, cardiac fibrosis, sclerosis, congenital hepatic fibrosis, Crohn's disease, cystic fibrosis, Dupuytren's contracture, endomyocardial fibrosis, glial scar, hepatitis C, hypertrophic cardiomyopathy, hypersensitivity pneumonitis, idiopathic pulmonary fibrosis, idiopathic interstitial pneumonia, interstitial lung disease, keloid, mediastinal fibrosis, myelofibrosis, nephrogenic systemic fibrosis, non-alcoholic fatty liver disease, old myocardial infarction, Peyronie's disease, pneumoconiosis, pneumonia, progressive massive fibrosis, pulmonary fibrosis, radiation-induced lung injury, retroperitoneal fibrosis, scleroderma/systemic sclerosis, silicosis and ventricular remodeling.

The hemoglobinopathy mentioned above includes, but is not limited to: "dominant" β-thalassemia, acquired (toxic) methemoglobinemia, carboxyhemoglobinemia, congenital Heinz body hemolytic anemia, HbH disease, HbS/β-thalassemia, HbE/β-thalassemia, HbSC disease, homozygous α+-thalassemia (α0-thalassemia phenotype), hydrops fetalis with Hb Bart's, sickle cell anemia/disease, sickle cell trait, sickle β-thalassemia, α+-thalassemia, α0-thalassemia, α-thalassemia associated with myelodysplastic syndrome, α-thalassemia with mental retardation syndrome (ATR), β0 -thalassemia, β+-thalassemia, δ-thalassemia, γ-thalassemia, severe β-thalassemia, moderate β-thalassemia, δβ-thalassemia and εγδβ-thalassemia.

The autoimmune disease mentioned above includes, but is not limited to: achalasia, Addison's disease, adult Still's disease, agammaglobulinemia, alopecia areata, amyloidosis, ankylosing spondylitis, anti-GBM/anti-TBM nephritis, antiphospholipid syndrome, autoimmune angioedema, autoimmune autonomic dysfunction, autoimmune encephalomyelitis, autoimmune hepatitis, autoimmune inner ear disease (AIED), autoimmune myocarditis, autoimmune oophoritis, autoimmune orchitis, autoimmune pancreatitis, autoimmune retinopathy, autoimmune urticaria, acute motor axonal neuropathy (AMAN), Balódisease, Behcet's disease, benign mucous membrane pemphigoid, bullous pemphigoid, Castleman disease (CD), celiac disease, Chagas disease, chronic inflammatory demyelinating polyneuropathy (CIDP), chronic recurrent multifocal osteomyelitis (CRMO), Churg-Strauss syndrome (CSS) or Eosinophilic Granulomatosis with Polyangiitis (EGPA), cicatricial pemphigoid, Cogan's syndrome, cold agglutinin disease, congenital heart block, Coxsackie myocarditis, CREST syndrome, Crohn's disease, dermatitis herpetiformis, dermatomyositis, Devic's disease (neuromyelitis optica), discoid lupus, Dressler's syndrome, Endometriosis, eosinophilic esophagitis (EoE), eosinophilic fasciitis, erythema nodosum, essential mixed cryoglobulinemia, Evans syndrome, fibromyalgia, fibrosing alveolitis, giant cell arteritis (temporal Arteritis), giant cell myocarditis, glomerulonephritis, Goodpasture syndrome, granulomatosis with polyangiitis, Graves' disease, Guillain-Barré syndrome, Hashimoto's thyroiditis, hemolytic anemia, Henoch-Schonlein purpura (HSP), herpes gestationis or pemphigoid gestationis (PG), hidradenitis suppurativa (HS) (acne inversa), Hypogammaglobulinemia, IgA nephropathy, IgG4-related sclerosing diseases, immune thrombocytopenic purpura (ITP), inclusion body myositis (IBM), interstitial cystitis (IC), juvenile arthritis, juvenile diabetes (type 1 diabetes), juvenile myositis (JM), Kawasaki disease, Lambert-Eaton syndrome, leukocytoclastic vasculitis, lichen planus, lichen Sclerosus, ligneous conjunctivitis, linear IgA disease (LAD), lupus, chronic Lyme disease, Meniere's disease, microscopic polyangiitis (MPA), mixed connective tissue disease (MCTD), Mooren's ulcer, Mucha-Habermann disease, multifocal motor neuropathy (MMN) or MMNCB, multiple sclerosis, myasthenia gravis, myositis, narcolepsy, neonatal lupus, neuromyelitis optica, neutropenia, ocular cicatricial pemphigoid, optic neuritis, palindromic rheumatism (PR), PANDAS, paraneoplastic cerebellar degeneration (PCD), paroxysmal mocturnal hemoglobinuria (PNH), Parry Romberg syndrome, pars planitis (peripheral uveitis), Parsonnage-Turner syndrome, Pemphigus, peripheral neuropathy, perivenous encephalomyelitis, pernicious anemia (PA), POEMS syndrome, polyarteritis nodosa, polyglandular syndrome type I, polyglandular syndrome type II, polyglandular syndrome type III, polymyalgia rheumatica, polymyositis, post-myocardial infarction syndrome, postpericardiotomy syndrome, primary biliary cholangitis, primary sclerosing cholangitis, progesterone dermatitis, psoriasis, psoriatic arthritis, pure red-cell aplasia (PRCA), pyoderma gangrenosum, Raynaud's phenomenon, reactive arthritis, reflex sympathetic dystrophy, relapsing polychondritis, restless legs syndrome (RLS), retroperitoneal fibrosis, rheumatic fever, rheumatoid arthritis, sarcoidosis, Schmidt syndrome, scleritis, scleroderma, sjögren's syndrome, autoimmune diseases of semen and testes, stiff-person syndrome (SPS), subacute bacterial endocarditis (SBE), Susac's syndrome, sympathetic ophthalmia (SO), Takayasu's arteritis, temporal arteritis/giant cell arteritis, thrombotic thrombocytopenic purpura (TTP), Tolosa-Hunt syndrome (THS), transverse myelitis, type 1 diabetes, ulcerative colitis (UC), undifferentiated connective tissue disease (UCTD), uveitis, vasculitis, vitiligo, Vogt-Koyanagi-Harada disease and Wegener's granulomatosis (or granulomatosis with polyangiitis (GPA)).

The virus infection mentioned above includes, but is not limited to: influenza, human immunodeficiency virus (HIV) and herpes.

The malaria infection mentioned above includes, but is not limited to infections caused by Plasmodium vivax, Plasmodium ovale, Plasmodium malariae and Plasmodium falciparum.

The disease caused by mutations that induce unfolded protein response (UPR), malaria infection mentioned above includes, but is not limited to: Marinesco-Sjogren syndrome, neuropathic pain, diabetic neuropathic pain, noise-induced hearing loss, nonsyndromic sensorineural hearing loss, age-related hearing loss, Wolfram syndrome, Darier

White disease, Usher syndrome, collagen disease, thin basement membrane nephropathy, Alport syndrome, achondroplasia, metaphyseal chondrodysplasia type Schmid and pseudoachondroplasia.

The compounds and derivatives provided in the present invention may be named according to the nomenclature system from IUPAC (International Union of Pure and Applied Chemistry) or CAS (Chemical Abstracts Service, Columbus, OH).

Referring to the definitions of terms used in the present invention, unless otherwise indicated, the original definitions provided for the groups or terms herein would apply to those groups or terms throughout the specification. For terms not specifically defined herein, their meanings shall be determined based on publicly available content and context, as would be understood by a skilled person in the art.

"Substitution" means that a hydrogen atom present in a molecule is replaced by another different atom or group; or the lone pair of electrons on an atom in a molecule is replaced by another atom or group, for example, the lone pair on an S atom can be substituted with a O atom to form

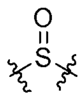 or 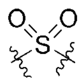 .

"Optional" or "optionally substituted" means that the "substitution" may occur or may not occur, and that the description includes the instances that the substitution occurs and the instances in which it does not.

The maximum and minimum numbers of carbon atoms in hydrocarbon groups are indicated by prefixes. For example, the prefixes in Ca-Cb alkyl means any alkyl containing "a" to "b" carbon atoms. Thus, C1-C6 alkyl refers to an alkyl containing 1-6 carbon atoms, for example.

"Alkyl" refers to a saturated hydrocarbon chain having a specific number of constituent atoms. An alkyl group may be a linear or branched chain. A representative branched alkyl group has one, two or three branches. As such, the C1-C6 alkyl includes methyl, ethyl, propyl (n-propyl or isopropyl), butyl (n-butyl, isobutyl and tert-butyl), pentyl (n-pentyl, isopentyl and neopentyl), and hexyl.

The term "C1-C6 alkyl" means any one of linear or branched groups containing 1-6 carbon atoms, such as methyl, ethyl, n-propyl, isopropyl, n-butyl, isobutyl, tert-butyl, sec-butyl, n-pentyl, tert-pentyl and n-hexyl.

Also, the "C1-C6 alkyl" includes any one of linear or branched groups with any range of carbon atom numbers from 1 to 6 as endpoints. For example, the "C1-C6 alkyl" includes C1-C6 alkyl, C1-C5 alkyl, C1-C4 alkyl, C2-C6 alkyl, C2-C5 alkyl, C2-C4 alkyl, C1-C3 alkyl and the like, as for illustration only rather than limiting the described range.

The term "alkoxy" and derivatives thereof means any of alkyls described above (such as C1-C6 alkyl and C1-C3 alkyl), which is attached to the rest of the molecule by an oxygen atom (-O-).

"Alkylene" refers to a divalent saturated aliphatic hydrocarbon group having a specific number of constituent atoms. Ca-Cb alkylene refers to an alkylene group having a to b carbon atoms. The alkylene group includes branched and linear hydrocarbonyl group. For example, the term "propylene" may be exemplified by the following structure:

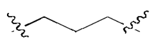 .

Likewise, the term "dimethylbutylene" may be exemplified, for example, by any of the following structures:

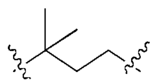 or 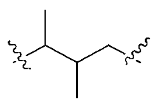 .

The C1-C6 alkylene in the present invention may be a C1 alkylene (for example, $-CH_2-$), C2 alkylene (for example, $-CH_2CH_2-$ or the like), C3 alkylene, C4 alkylene, C5 alkylene or C6 alkylene.

"Cycloalkylene" as described in the present invention refers to divalent saturated cyclic alkane having multiple carbon atoms and no heteroatoms, containing a single ring or multiple rings (fused, spiro, bridged rings). Examples of monocarbocyclic group include, such as divalent cyclopropyl, divalent cyclobutyl, divalent cyclohexyl, divalent cyclopentyl, divalent cyclooctyl, divalent cyclopentenyl and divalent cyclohexenyl. Examples of bridged alkylene system include bicyclo[3,1,0]hexylene, bicyclo[3,1,1]hexylene, bicyclo[2,2,1]hexylene, bicyclo[2,2,2]hexylene, bicyclo[2.2.1]heptylene, bicyclo[2.2.2]octylene, bicyclo[1.1.2]pentylene.

Examples of "cycloalkylene" as described in the present invention include, but are not limited

to 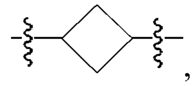, 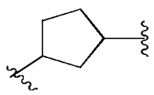, 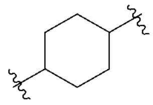, 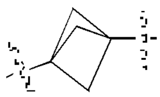 or 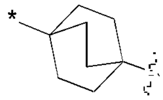

and the like.

The term "C3-C10 cycloalkyl" refers to 3-10-membered all-carbon monocyclic, fused and bridged rings, in which zero, one or more double bonds are present, but no completely conjugated $\pi$-electron system exists. Examples of C3-C10 cycloalkyl include, but are not limited to cyclopropyl, cyclobutyl, cyclopentyl, cyclopentenyl, cyclohexyl, cyclohexenyl, cyclohexadienyl and the like.

"Heterocycloalkyl" as described in the present invention refers to a monovalent or divalent saturated ring (the divalent saturated ring of a heterocycloalkyl means a heterocycloalkylene) containing at least one heteroatom and having a single ring or multiple rings (fused, spiro, bridged rings), where the heteroatom refers to an N, O or S atom or the like. Examples of 3-10-membered heterocyclyl may be oxetanyl, azetidinyl, oxolanyl, dioxanyl, piperazinyl, piperidyl, morpholinyl, sym-trioxanyl and the like. Examples of "heterocycloalkyl" as described in the present invention include, but are not limited to

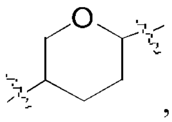, 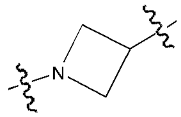, 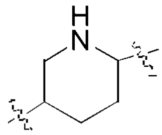

or piperidyl and the like.

The unsaturation as described in the present invention means that a group or molecule contains a carbon-carbon double bond, carbon-carbon triple bond, carbon-oxygen double bond, carbon-sulfur double bond, carbon-nitrogen triple bond and the like.

"Aryl" as described in the present invention refers to an aromatic hydrocarbon group having multiple carbon atoms, and the arylene refers to a divalent aryl. The aryl generally be a monocyclic, bicyclic or tricyclic aryl having multiple carbon atoms. Moreover, the term "aryl" as used herein refers to an aromatic substituent that may be a single aromatic ring or multiple aromatic rings fused together. Non-limiting examples include phenyl, naphthyl, tetrahydronaphthyl,

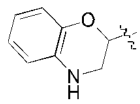,

and

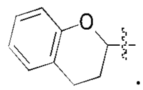.

"5-10-membered heteroaryl" as described in the present invention refers to an unsaturated aromatic ring containing at least one heteroatom, where the heteroatom refers to an N, O or S atom or the like. The heteroarylene refers to divalent heteroaryl. Generally included is an aromatic monocyclic or bicyclic hydrocarbon containing multiple ring atoms, with one or more ring atoms selected from O, N, S heteroatoms. Preferably, one to three heteroatoms are present. For example, 5-6-membered heteroaryl represents, such as, pyridyl, pyrrolyl, furyl, thienyl, pyrazolyl, imidazolyl, thiazolyl, pyranyl, thiopyranyl, piperazinyl, vic-triazolyl, 1,2,4-triazolyl, 1,2,5-triazolyl, 1,3,4-triazolyl, 1,2,3,4-tetrazolyl, 1,2,3,5-tetrazolyl, isoxazolyl, oxazolyl, 1,2,3-oxadiazolyl, 1,2,4-oxadiazolyl, 1,3,4-oxadiazolyl,, furazanyl, 1,2,3,5-oxatriazolyl, 1,2,3,4-oxatriazolyl, 1,3,2-dioxazolyl, 1,2,3-dioxazolyl, 1,2,3,4-dioxadiazolyl,, 1,2,3,5-dioxadiazolyl,, 1,3,3,4-dioxadiazolyl,, 1,3,4,5-dioxadiazolyl,, 1,2,3,4-dioxadiazolyl,, isothiazolyl, pyridazinyl, vic-triazinyl, 1,2,4-triazinyl, 1,3,5-triazinyl, 1,2,4,5-tetrazinyl 1,2,4-oxazinyl, 1,2,6-oxazinyl, 1,3,2-oxazinyl, 1,3,6-oxazinyl, 1,4,2-oxazinyl, 1,2-isoxazinyl, and 1,4-isoxazinyl.

"Halogen" as described in the present invention refers to fluorine, chlorine, bromine or iodine.

The "alkyl substituted with a halogen" as described in the present invention refers to an alkyl in which one or more hydrogen atoms have been replaced by a halogen; for example, the C1-C6 alkyl substituted with a halogen refers to an alkyl containing 1-6 carbon atoms in which a hydrogen atom has been replaced by one or more halogen atoms, such as fluoromethyl, difluoromethyl and trifluoromethyl.

"=O" as described in the present invention means that two hydrogen atoms in a molecule have been replaced by oxygen atoms via a double bond.

In the present invention, it is apparent to a person skilled in the art that any group which is named by a combined name, for example, "6-10-membered aryl-D-C1-C6 alkyl", should mean that it is conventionally constructed from the left to the right starting with a derivative moiety such as C1-C6 alkyl thereof. It should be understand that the alkyl here is a divalent alkyl.

"Stereoisomer" in the present invention refers to a compound having the same chemical structure, but different spatial arrangements of atoms or groups. The stereoisomer includes enantiomers, diastereomers, conformational isomers (rotamers), geometric isomers (cis/trans isomers), atropisomer and the like. Unless indicated otherwise, all of the stereoisomers or mixture thereof having the formulas as described in the present invention fall within the scope of the invention. The term "enantiomer" as used in the present invention refers to stereoisomers that are physical entities and mirror images of each other but cannot be superimposed. "Diastereomer" refers to a stereoisomers in which molecules has two or more chiral centers and are not mirror images, where by way of example,

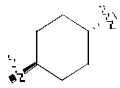

represents the chiral center of the compound.

The term "geometric isomer" as used in the present invention includes: cis/trans isomers, isotactic polymers and syndiotactic polymers, rotamers generated by steric hindrance, where by way of example,

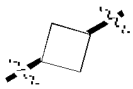

represents a cis-configuration. In other words, two substituents on the cyclobutyl ring are at the same side.

The definitions and rules of stereochemistry as used in the present invention generally follow S. P. Parker, Ed., McGraw-Hill Dictionary of Chemical Terms (1984) McGraw-Hill Book Company, New York; and Eliel, E. and Wilen, S., "Stereochemistry of Organic Compounds," John Wiley & Sons, Inc., New York, 1994.

"Tautomer" in the present invention typically refers to structural isomers with different energies which can interconvert through a low energy barrier. For example, a proton tautomer (also referred to as prototropic tautomer) involves interconversion that occurs through proton migration, such as keto-enol tautomerism and imine-enamine tautomerism. A valence tautomer involves interconversion that occurs through reorganization of some bonding electrons.

"Polymorph" in the present invention refers to a crystalline form of a compound (or salts, hydrates or solvates thereof) that exhibits specific crystal packing arrangements. All polymorphs share the same elemental composition. Different crystal forms usually have different X-ray diffraction patterns, infrared spectrums, melting points, densities, hardnesses, crystal shapes, optical and electrical properties, stabilities and solubilities. The dominance of one particular crystal polymorph may be caused by factors such as recrystallization solvent, crystallization rate, storage temperature, and others.

"Solvate" in the present invention is the mixture produced when a compound is dissolved in a solvent.

"N-oxide" in the present invention, also referred to as amine oxide, is a class of organic compound s with the general formula $R_3N$+-O- (also written as $R_3N$=O or $R_3N \rightarrow O$).

"Isotopically labeled compound" in the present invention means that one or more atoms in a molecule or group have been replaced by their isotopic atoms, for example, a hydrogen has been replaced by a deuterium atom, with the proportion of deuterium atoms exceeding its abundance in nature; as another example, 12C has been replaced by 13C.

"Metabolites" in the present invention refers to the material generated when a drug molecule is taken up by an organism and undergoes structural changes under the action of the organism.

"Prodrug" in the present invention refers to a compound that is chemically modified from a drug, exhibiting little or no activity in vitro but releasing the active drug through enzymatic or non-enzymatic transformation in vivo to exert its therapeutic effect.

The term "pharmaceutically acceptable" means that a carrier, vehicle, diluent, adjuvant, and/or salt formed is generally compatible with other ingredients of a drug formulation in terms of chemistry or physics, and physiologically compatible with the recipient.

The term "salt" and "pharmaceutically acceptable salt" refers to acid salts and/or base salts formed by the aforementioned compound or stereoisomers thereof with inorganic and/or organic acids and bases, including zwitterionic salts (inner salts), as well as quaternary ammonium salts, such as alkylammonium salts. These salts can be directly obtained in the final separation and purification of the compound. Alternatively, these salts can be obtained by mixing the

aforementioned compound or stereoisomers thereof with an appropriate amount of acid or base (for example, in stoichiometric quantities). These salts may be collected by filtration after forming precipitates in solution, recovered upon solvent evaporation, or obtained via lyophilization following reaction in aqueous media.

The term "prevention", "preventing" or "prevent" involves delaying the onset of and inhibiting a disease, and includes not only the prevention of the progression, but also the recurrence of the disease.

The term "treatment", "treating" or "treat" means reversing, alleviating or eradicating the disorder or condition to which the term is applied, or the progression of one or more symptoms of such condition or disease.

In certain embodiments, one or more compounds of the present invention can be used in combination with each other. Alternatively, the compounds of the present invention may be combined with any other active agents for use in preparing medicaments or pharmaceutical compositions that regulate cellular function or treat diseases. If a group of compounds is used, these compounds may be administered to the subject simultaneously, separately, or sequentially.

Apparently, based on the foregoing description of the present invention, modifications, substitutions or alterations in various forms can be made in accordance with common technical knowledge and conventional means in the art, without departing from the basic technical idea as defined in the invention.

The following provides a more detailed description of the foregoing aspects of the present invention through specific embodiments. However, this should not be understood as limiting the scope of the subject matters of the present invention to the following examples. Any technology implemented based on the foregoing content of the present invention fall within the scope of the present invention.

## DETAILED DESCRIPTION

The following is a detailed description of the implementation of the present application in conjunction with the examples. The detailed description of the following examples is provided for illustrative purposes only to demonstrate the principles of the present application and is not to be construed as limiting the scope thereof. In other words, the present application is not limited to examples described herein.

The known starting materials of the invention may be synthesized by methods known in the art or purchased from companies such as Bidepharm, Energy Chemical, Tansoole, Pharmablock Sciences (Nanjing), Jiangsu Aikon, and Beijing InnoChem Science & Technology Co., Ltd.

Unless otherwise specified, the reaction is carried out under nitrogen atmosphere. Unless otherwise specified, the reaction is carried out at room temperature. The room temperature refers to the conventional indoor temperature in this field, typically 20°C to 30°C. Unless otherwise specified, M is moles per liter.

The structures of compounds were determined by nuclear magnetic resonance (NMR) and mass spectrometry (MS). NMR shift (δ) is given in units of $10^{-6}$(ppm). The NMR measurements were performed on a Bruker AvanceIII 400 using deuterated dimethyl sulfoxide (DMSO-$d_6$), deuterated chloroform ($CDCl_3$) and deuterated methanol (Methanol-$d_4$) as solvents and tetramethyl silane (TMS) as the internal standard. The LC-MS analysis was performed using a Shimadzu LC-MS system (Shimadzu LC-MS 2020(ESI)). The HPLC determination was performed using a Shimadzu high-performance liquid chromatograph (Shimadzu LC-20A). The MPLC determination (medium-pressure liquid chromatography) was performed using a Gilson GX-281 reversed-phase preparative chromatograph. Thin-layer chromatography was performed using Yantai Huanghai HSGF254 or Qingdao GF254 silica gel plates, with a separation and purification product thickness of 0.4 mm to 0.5 mm. Column chromatography usually employs Yantai Huanghai silica gel (200-300 meshes) as the support.

Abbreviations: DMF: N,N'-dimethylformamide (also called dimethylformamide); DIEA/DIPEA: N,N'-diisopropylethylamine; HATU: 2-(7-azabenzotriazol)-N,N,N',N'-tetramethyluronium hexafluorophosphate; TFA: trifluoroacetic acid.

The following further illustrates the beneficial effects of the present application by combining examples and comparative examples, but the scope of the invention is not limited to these examples.

### Example 1: Synthesis of Compound 1

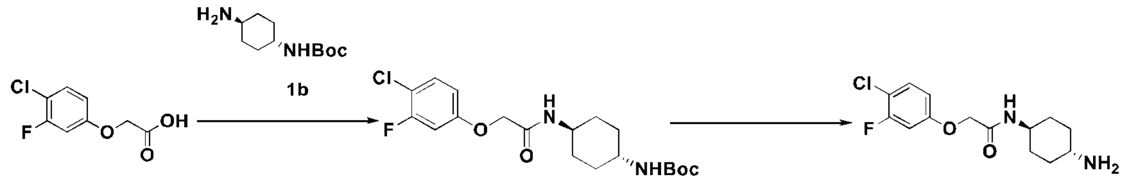

1b

1a 1c 1d

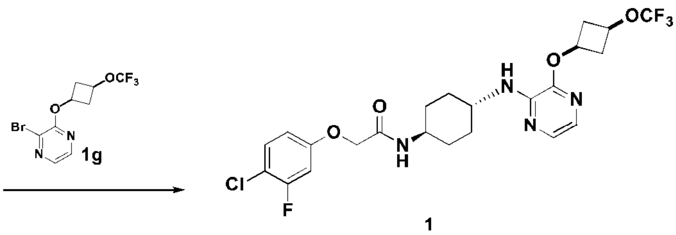

1g

1

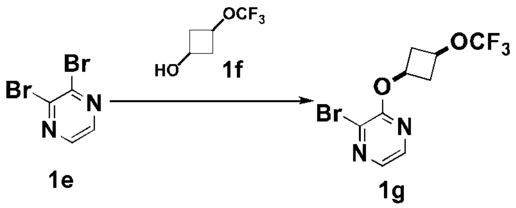

1f

1e 1g

Step 1: Preparation of Intermediate 1c

A single-neck flask was charged with 1a (500 mg, 2.44 mmol) and dichloromethane (10 mL) at room temperature, added 1b (576 mg, 2.68 mmol), N,N'-diisopropylethylamine (632 mg, 4.89 mmol) and 1-propyl phosphoric anhydride (3.11 g, 4.88 mmol) under stirring, stirred overnight at room temperature. The reaction solution was concentrated under reduced pressure, and the crude product was purified on silica gel chromatographic column (petroleum ether: ethyl acetate=100:1~3:1) to afford Intermediate 1c (795 mg). LC-MS: m/z :401.1(M+H)$^+$.

Step 2: Preparation of Intermediate 1d

A single-neck flask was charged with 1c (795 mg, 1.98 mmol) and dichloromethane (10 mL) at room temperature, added trifluoroacetic acid (3 mL) under stirring, stirred for 1 h at room temperature. The reaction solution was concentrated under reduced pressure to give crude Intermediate 1d (732 mg), which was carried to the next step without any purification. LC-MS: m/z :300.1(M+H)$^+$.

Step 3: Preparation of Intermediate 1g

A single-neck flask was charged with 1f (50 mg, 0.32 mmol) and tetrahydrofuran (3 mL) at room temperature, added sodium hydride (19 mg, 0.48 mmol) (60% in oil) in an ice bath. After stirring for 0.5 h in an ice bath, the reaction solution was added 1e (76 mg, 0.32 mmol), stirred 1 h at room temperature. The reaction was quenched by adding 10 mL ammonium chloride into the reaction solution, then extracted with 15 mL dichloromethane for 3 times. The combined organic layer was washed with 30 mL brine, dried over anhydrous sodium sulfate, filtered and concentrated. The crude product was purified on silica gel chromatographic column (petroleum ether: ethyl acetate=100:1~10:1) to afford Intermediate 1g (33 mg). LC-MS: m/z :312.9(M+H)$^+$.

Step 4: Preparation of Compound 1

A single-neck flask was charged with 1g (33 mg, 0.11 mmol), 1d (59 mg, 0.13 mmol) and 1,4-dioxane (2 mL) at room temperature, sequentially added sodium tert-butoxide (30.5 mg, 0.33 mmol), tris(dibenzylideneacetone)dipalladium(19.4 mg, 0.02 mmol) and 4,5-bis(diphenylphosphino)-9,9-dimethylxanthene (24.5 mg, 0.04 mmol) under stirring, and the reaction was carried out at 100°C for 18 h after three cycles of nitrogen purging. After filtration, the reaction solution was directly concentrated under pressure to provide a crude product. Finally, the crude product was purified by high-performance liquid phase (ammonium bicarbonate/acetonitrile/water system) to afford Compound 1 (3 mg). LC-MS:

m/z :533.2(M+H)$^+$. $^1$H NMR (DMSO-d6) δ: 8.00 (d, J=8.0 Hz, 1H), 7.44-7.58 (m, 2H), 7.16 (d, J=3.1 Hz, 1H), 7.07 (dd, J=11.4, 2.8 Hz, 1H), 6.85 (dd, J=9.0, 1.9 Hz, 1H), 6.29 (d, J=8.0 Hz, 1H), 4.85 (t, J=6.9 Hz, 1H), 4.66 (t, J=7.0 Hz, 1H), 4.51 (s, 2H), 3.72-3.87 (m, 1H), 3.56-3.70 (m, 1H), 2.98 (m, 2H), 2.34-2.48 (m, 2H), 1.93 (d, J=9.9 Hz, 2H), 1.81 (d, J=10.4 Hz, 2H), 1.29-1.51 (m, 4H).

**Example 2: Synthesis of Compound 2**

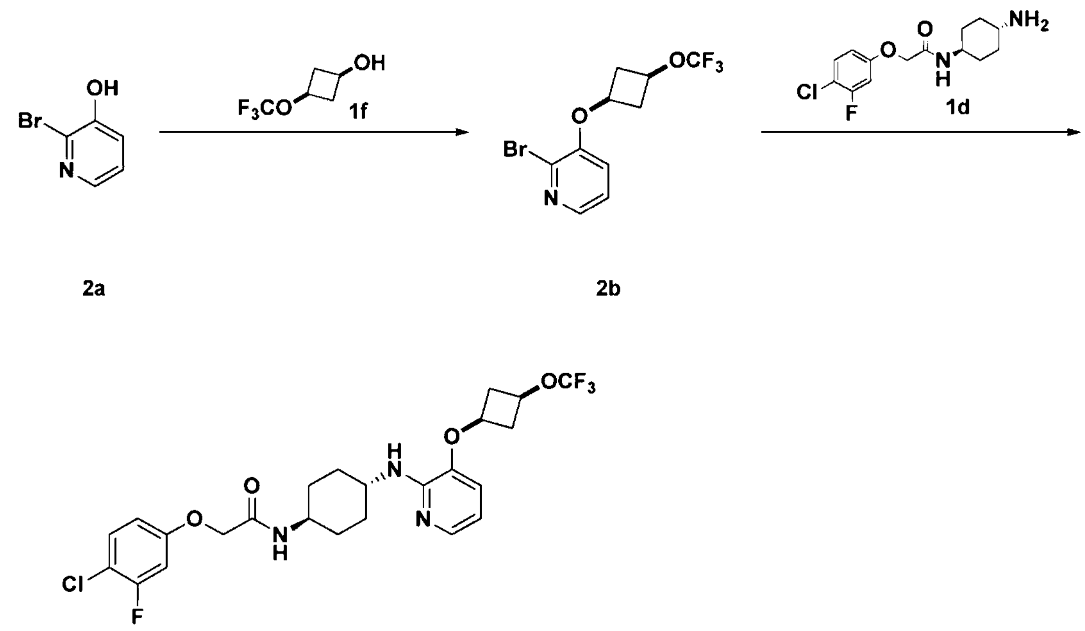


2a

2b

Compound 2

Step 1: Preparation of Intermediate 2b

A single-neck flask was charged with 2a (33 mg, 0.19 mmol), 1f (30 mg, 0.19 mmol) and tetrahydrofuran (5 mL) at 0°C, added triphenylphosphine (60 mg, 0.23 mmol) and diisopropyl azodiformate (0.04 mL, 0.21 mmol) under stirring, stirred for 5 h at room temperature. The reaction solution was diluted with 10 mL water, then extracted 3 times each with 15 mL ethyl acetate, the combined organic layer was washed with 20 mL sat. brine, dried over sodium sulfate, filtered and concentrated. The crude product was purified on silica gel chromatographic column (petroleum ether: ethyl acetate=100:1~2:1) to afford Intermediate 2b (33 mg). LC-MS: m/z :311.9(M+H)$^+$.

Step 2: Preparation of Compound **2**

A single-neck flask was charged with 2b (33 mg, 0.11 mmol), 1d (60 mg, 0.13 mmol) and 1,4-dioxane (2 mL) at room temperature, sequentially added sodium tert-butoxide (31 mg, 0.33 mmol), tris(dibenzylideneacetone)dipalladium(20 mg, 0.02 mmol) and 4,5-bis(diphenylphosphino)-9,9-dimethylxanthene (26 mg, 0.04 mmol) under stirring, and the reaction was carried out at 100°C for 18 h after three cycles of nitrogen purging. After filtration, the reaction solution was directly concentrated under pressure to provide a crude product. The crude product was purified by high-performance liquid phase (trifluoroacetic acid/acetonitrile/water system) to finally afford Compound **2** (9 mg). LC-MS: m/z :532.2(M+H)$^+$. $^1$H NMR (DMSO-d6) δ: 8.00 (d, J=7.9 Hz, 1H), 7.57 (d, J=4.5 Hz, 1H), 7.50 (t, J=8.9 Hz, 1H), 7.07 (dd, J=11.4, 2.7 Hz, 1H), 6.85 (dd, J=8.8, 1.9 Hz, 1H), 6.76 (d, J=7.8 Hz, 1H), 6.41 (dd, J=7.6, 5.2 Hz, 1H), 5.58 (d, J=8.0 Hz, 1H), 5.09 (s, 1H), 4.85-4.93 (m, 1H), 4.51 (s, 2H), 3.81 (s, 1H), 3.57-3.72 (m, 1H), 2.66-2.75 (m, 2H), 2.55-2.66 (m, 2H), 1.96 (s, 2H), 1.79 (s, 2H), 1.37 (t, J=9.6 Hz, 4H).

**Example 3: Synthesis of Compound 3**

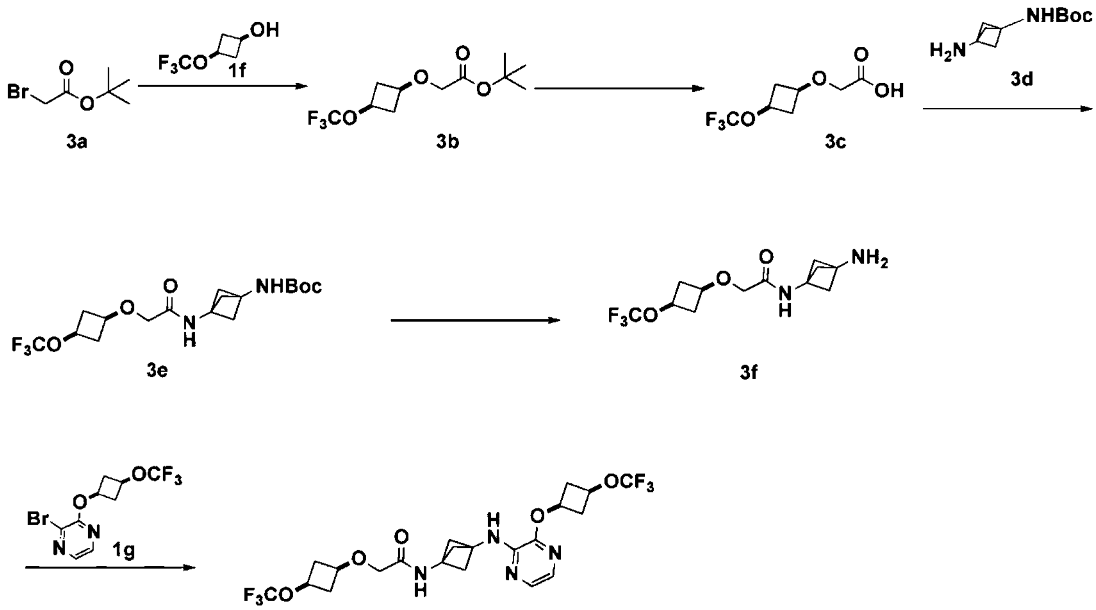

3a 3b 3c 3e 3f

Compound 3

Step 1: Preparation of Intermediate 3b

A single-neck flask was charged with 1f (135 mg, 0.86 mmol), toluene (2 mL) and water (0.6 mL) at room temperature, sequentially added 3a (219 mg, 1.30 mmol), tetrabutylammonium bisulfate (15 mg, 0.04 mmol) and sodium hydroxide (520mg, 12.98 mmol) under stirring, stirred for 4 h at room temperature. The reaction was diluted with water (10 mL), extracted 3 times each with 5 mL ethyl acetate, the combined organic phase was washed with 5 mL sat. brine, dried over sodium sulfate, filtered and concentrated. The crude product was purified on silica gel chromatographic column (petroleum ether: ethyl acetate=100:1~4:1) to afford 3b (219 mg). $^1$H NMR (DMSO-d6) δ: 4.47 (t, J=7.1 Hz, 1H), 3.92 (s, 2H), 3.73 (t, J=6.9 Hz, 1H), 2.69-2.80 (m, 2H), 2.09 (m, 2H), 1.42 (s, 9H).

Step 2: Preparation of Intermediate 3c

A single-neck flask was charged with 3b (219 mg, 0.81 mmol) and dichloromethane (2 mL) at 0°C, added trifluoroacetic acid (2 mL) under stirring, stirred for 1 h at 0°C. The reaction solution was concentrated under reduced pressure to give crude 3c (145 mg), which was carried to the next step without any purification.

Step 3: Preparation of Intermediate 3e

A single-neck flask was charged with 3c (145 mg, 0.67 mmol) and dichloromethane (3 mL) at room temperature, added 3d (175.92 mg, 0.89 mmol), N,N'-diisopropylethylamine (0.59 mL, 3.55 mmol) and 1-propyl phosphoric anhydride (1.13 g, 1.77 mmol) under stirring, stirred for 16 h at room temperature. The reaction solution was concentrated under pressure to provide a crude product. The crude product was purified on silica gel chromatographic column (petroleum ether: ethyl acetate=100:1~1:1) to afford 3e (290 mg). LC-MS: m/z :339.1(M+H-56)$^+$.

Step 4: Preparation of Intermediate 3f

A single-neck flask was charged with 3e (290 mg, 0.74 mmol) and dichloromethane (3 mL) at 0°C, added 4 M HCl in dioxane (3 mL) under stirring, stirred for 2 h at room temperature. The reaction solution was concentrated under pressure to provide a crude product 3f (217 mg). The crude product was carried to the next step without any purification, LC-MS: m/z :295.1(M+H)$^+$.

Step 5: Preparation of Compound **3**

A single-neck flask was charged with 3f (217 mg, 0.74 mmol), 1g (133 mg, 0.42 mmol) and 1,4-dioxane (3 mL) at room temperature, sequentially added sodium tert-butoxide (123 mg, 1.47 mmol), tris(dibenzylideneacetone)dipalladium(78 mg, 0.08 mmol) and 1,1-binaphthyl-2,2'-bis(diphenylphosphine) (106 mg, 0.17 mmol) under stirring, and the

reaction was carried out at 100°C for 18 h after three cycles of nitrogen purging. After filtration, the reaction solution was directly concentrated under pressure to provide a crude product. The crude product was purified by high-performance liquid phase (trifluoroacetic acid/acetonitrile/water system) to finally afford Compound 3 (19.02 mg). LC-MS: m/z :527.2$(M+H)^+$. $^1H$ NMR (DMSO-d6 ) δ: 8.43 (s, 1H), 7.64 (d, J=3.1 Hz, 1H), 7.30 (d, J=3.1 Hz, 1H), 4.92 (t, J=6.9 Hz, 1H), 4.73 (s, 1H), 4.55 (t, J=7.1 Hz, 1H), 3.80 (s, 2H), 3.73-3.79 (m, 1H), 3.04 (m, 2H), 2.77-2.85 (m, 2H), 2.41-2.48 (m, 2H), 2.37-2.41 (m, 6H), 2.22 (dd, J=9.6, 2.5 Hz, 2H).

**Example 4: Synthesis of Compound 4**

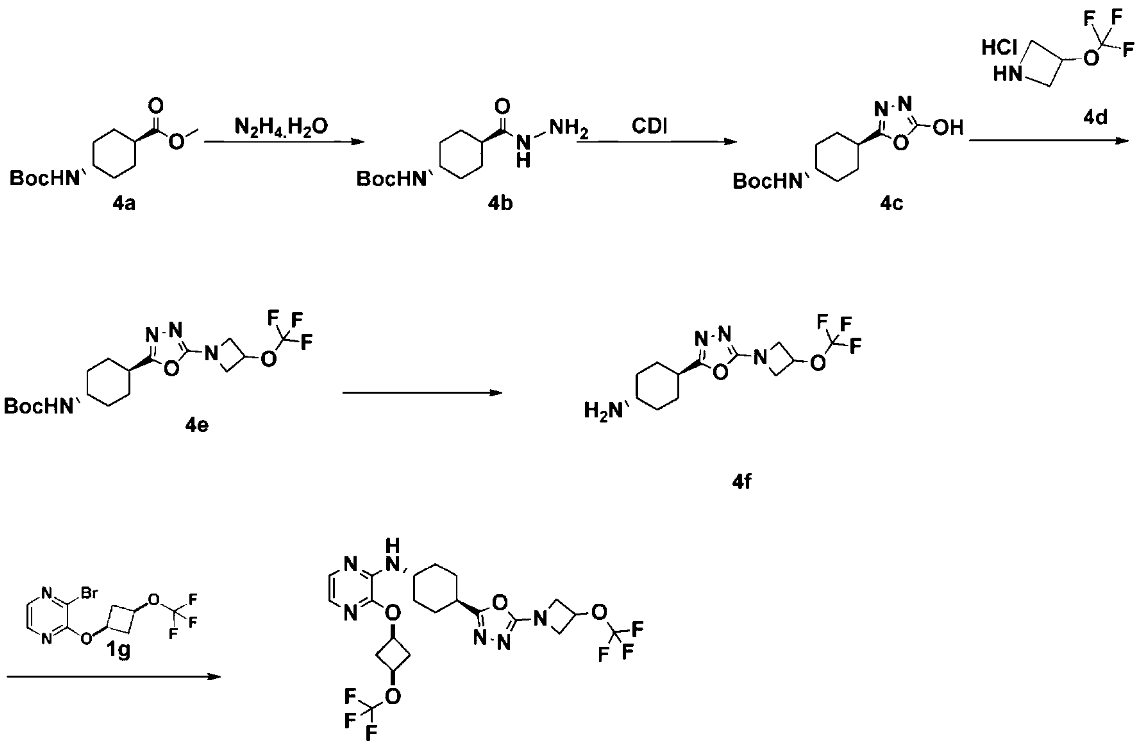


4a 4b 4c 4e 4f 1g

Compound 4

Step 1: Preparation of Intermediate 4b

A single-neck flask was charged with 4a (4.0 g, 15.54 mmol) and dry ethanol (50.0 mL) at room temperature, sequentially added a compound hydrazine hydrate (9.1 g, 155.54 mmol) under stirring. The temperature of the reaction was raised to 80°C, stirred for 18 h. The reaction solution was concentrated under pressure to give a crude product. The crude product was purified by triturating with acetonitrile to give Intermediate 4b (3.8 g). LC-MS: m/z :202.1$(M+H-56)^+$.

Step 2: Preparation of Intermediate 4c

A single-neck flask was charged with 4b (3.8 g, 13.29 mmol) and 1,2-dichloroethane (30.0 mL) at room temperature, added N,N'-carbonyldiimidazole (5.0 g, 26.58 mmol) under stirring, reacted for 18 h at room temperature. The reaction solution was concentrated under pressure to provide a crude product. The crude product was purified on silica gel chromatographic column (petroleum ether: ethyl acetate=100:1~1:1) to afford Intermediate 4c (2.9 g). LC-MS: m/z :228.0$(M+H-56)^+$.

Step 3: Preparation of Intermediate 4e

A single-neck flask was charged with 4c (2.9 g, 9.26 mmol) and dimethylformamide (30 mL) at room temperature, sequentially added Compound 4d (1.73 g, 12.28 mmol), N,N'-diisopropylethylamine (6.65 g, 51.18 mmol) and Carter condensation agent (6.45 g, 15.53 mmol) under stirring, reacted for 16 h at room temperature. To the reaction solution added 300 mL water, extracted once with 300 mL ethyl acetate, the organic phase was washed with 300 mL sat. brine,

dried over sodium sulfate, filtered, and the filtrate was then concentrated under pressure to afford a crude product. The crude product was purified by high-performance liquid phase (ammonium bicarbonate/acetonitrile/water system) to finally afford Intermediate 4e (1.82 g) as a white solid. LC-MS: m/z :407.2$(M+H)^+$.

Step 4: Preparation of Intermediate 4f

A single-neck flask was charged with 4e (140 mg, 0.34 mmol) and dichloromethane (6.0 mL) at room temperature, added trifluoroacetic acid (2.0 mL, 4.5 mmol) under stirring, reacted for 2 h at room temperature. The reaction solution was concentrated under pressure to provide Intermediate 4f (98 mg). The crude product was carried to the next step without any purification, LC-MS: m/z :307.2$(M+H)^+$.

Step 5: Preparation of Compound **4**

A single-neck flask was charged with compounds 1g (30.66 mg, 0.10 mmol), 4f (25 mg, 0.08 mmol), tris(dibenzylideneacetone)dipalladium (7.47 mg, 0.01 mmol), 1,1-binaphthyl-2,2'-bis(diphenylphosphine) (10.17 mg, 0.02 mmol), sodium tert-butoxide (23.53 mg, 0.24 mmol) and dry toluene (2 mL) at room temperature. The reaction solution was sparged with nitrogen gas for 5 min under stirring, then reacted for 4 h at 100°C under the protection of a nitrogen balloon. The reaction solution was cooled to room temperature, then filtered, the filtrate was concentrated under pressure, and the crude product was purified by high-performance liquid phase (ammonium bicarbonate/acetonitrile/water system) to afford Compound 4 (2.11 mg). $^1$H NMR (400 MHz, MeOD) δ 7.46 (d, J=3.2 Hz, 1H), 7.18 (d, J=3.2 Hz, 1H), 5.28 (s, 1H), 4.93-4.88 (m, 1H), 4.64-4.55 (m, 1H), 4.55-4.47 (m, 2H), 4.27 (dd, J=9.4, 4.0 Hz, 2H), 3.87 (s, 1H), 3.10-2.97 (m, 2H), 2.83 (s, 1H), 2.44 (d, J=6.7 Hz, 2H), 2.16 (d, J=11.1 Hz, 4H), 1.70 (d, J=11.1 Hz, 2H), 1.49 (d, J=11.0 Hz, 2H), LC-MS: m/z : 539.2$(M+H)^+$.

**Example 5: Synthesis of Compound 5**

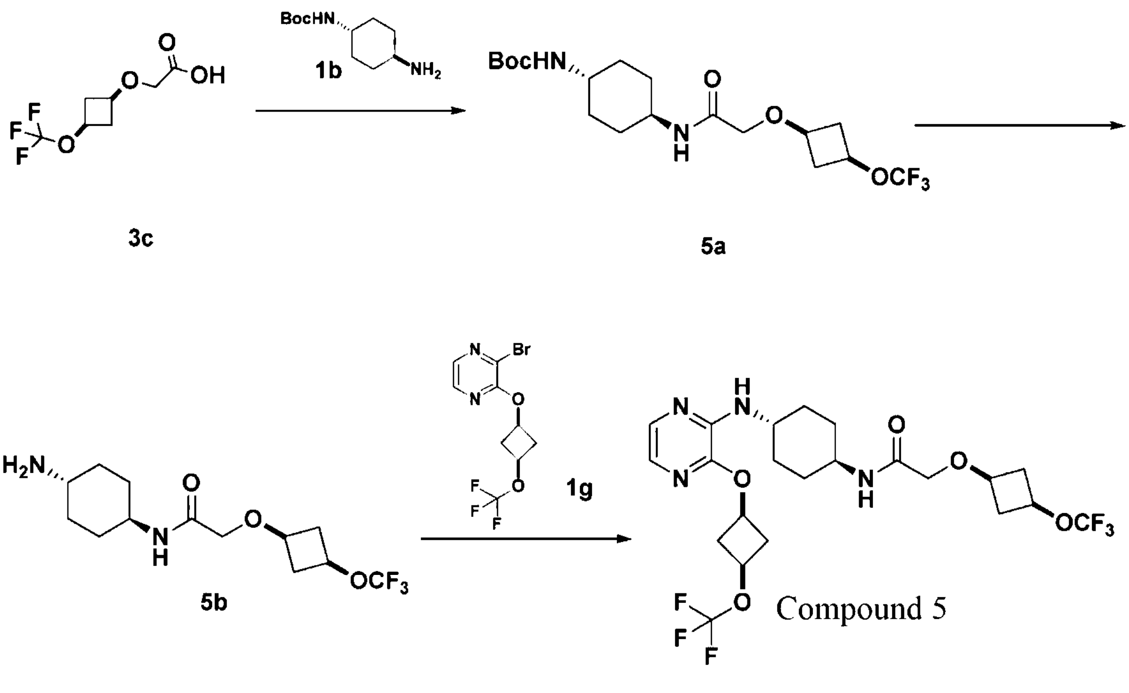

3c 1b 5a

5b 1g Compound 5

Step 1: Preparation of Intermediate 5a

A single-neck flask was charged with 3c (100 mg, 0.42 mmol), 1b (100.08 mg, 0.47 mmol) and dimethylformamide (2 mL) at room temperature, added 2-(7-azabenzotriazol)-N,N,N',N'-tetramethyluronium hexafluorophosphate (266.35 mg, 0.70 mmol) and N,N'-diisopropylethylamine (0.23 mL, 1.40 mmol) under stirring, reacted for 16 h at room temperature. The reaction solution was poured into 30 mL water, then extracted two times each with 30 mL ethyl acetate. The organic phase was washed with 50 mL sat. brine, dried over sodium sulfate, filtered, and the filtrate was then

concentrated under pressure to give a crude product. The crude product was purified on silica gel chromatographic column (dichloromethane: methanol=30:1~10:1) to afford Intermediate 5a (100 mg). LC-MS: m/z :355.0(M+H-56)$^+$.

Step 2: Preparation of Intermediate 5b

A single-neck flask was charged with compounds 5a (50 mg, 0.12 mmol) and dichloromethane (10 mL) at room temperature, added trifluoroacetic acid (0.05 mL, 0.73 mmol) in an ice bath under stirring. After reaction at room temperature for 1 h, the reaction solution was concentrated under pressure to provide Intermediate 5b (17 mg). The crude product was carried to the next step without any purification, LC-MS: m/z :311.0(M+H)$^+$.

Step 3: Preparation of Compound 5

A single-neck flask was charged with 5b (17 mg, 0.05 mmol), 1g (17.15 mg, 0.05 mmol) and 1,4-dioxane (5 mL) at room temperature, sequentially added tris(dibenzylideneacetone) dipalladium (5.02 mg, 0.01 mmol), 1,1-binaphthyl-2,2'-bis(diphenylphosphine) (6.82 mg, 0.01 mmol) and sodium tert-butoxide (7.90 mg, 0.08 mmol) under stirring. The reaction was carried out at 100°C for 18 h under nitrogen protection. The reaction solution was poured into 20 mL water, then extracted two times each with 30 mL ethyl acetate. The organic phase was washed with 10 mL sat. brine, dried over sodium sulfate, and the filtrate was concentrated under pressure after filtration to give a crude product. The crude product was separated by high-performance liquid phase (trifluoroacetic acid/acetonitrile/water system) to afford Compound 5 (2.34 mg). $^1$H NMR (400 MHz, $CDCl_3$) $\delta$ 7.56 (d, J=3.6 Hz, 1H), 7.32 (s, 1H), 6.36 (s, 1H), 4.98-4.88 (m, 1H), 4.47 (t, J=7.0 Hz, 1H), 4.35-4.23 (m, 1H), 3.83 (s, 3H), 3.71 (t, J=6.9 Hz, 1H), 3.08 (d, J=7.1 Hz, 2H), 2.82 (m, 2H), 2.50 (s, 2H), 2.26 (m, 4H), 2.08 (d, J=12.6 Hz, 2H), 1.51-1.17 (m, 6H). LC-MS: m/z :543.2(M+H)$^+$. **Example 6: Synthesis of Compound 6**

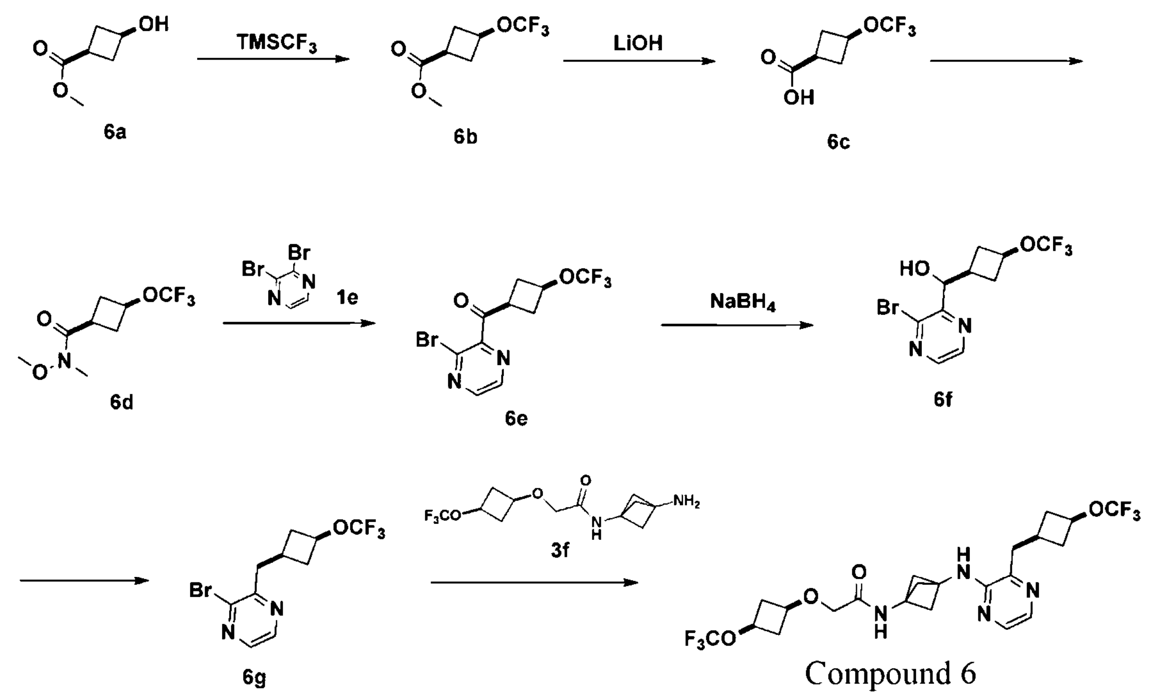

6a 6b 6c 6d 6e 6f 6g Compound 6

Step 1: Preparation of Intermediate 6b

A single-neck flask was charged with 6a (4.0 g, 30.74 mmol) and ethyl acetate (120.0 mL) at room temperature, added silver trifluoromethane sulfonate (23.69 g, 92.21 mmol), potassium fluoride (7.14 g, 122.94 mmol), N-fluoro-N'-(fluoromethyl)triethylenediamine bis(tetrafluoroborate) (16.33 g, 46.10 mmol), 2-fluoropyridine (7.99 mL, 92.21 mmol) and (trifluoromethyl)trimethyl saline(13.80 mL, 92.21 mmol) under stirring. The reaction was carried out at room temperature for 48 h under nitrogen protection in the dark. The reaction solution was filtered, the filter cake was washed with 100 mL ethyl acetate, the filtrate was concentrated under pressure, and the crude product was purified on silica gel chromatographic column (petroleum ether: ethyl acetate=100:1~10:1) to afford Intermediate 6b (760 mg).

Step 2: Preparation of Intermediate 6c

A single-neck flask was charged with 6b (760 mg, 3.84 mmol), tetrahydrofuran (10.0 mL), methanol (5 mL) and water (5.0 mL) at room temperature, added lithium hydroxide (276 mg, 11.51 mmol) under stirring, reacted for 3 h at room temperature. The reaction solution was adjusted to pH=3 with 1 N hydrochloric acid, extracted with 20 mL ethyl acetate, the organic phase was dried with anhydrous sodium sulfate, filtered, the filtrate was then concentrated under pressure to afford Intermediate 6c (700 mg). LC-MS: m/z :183.0$(M-H)^-$.

Step 3: Preparation of Intermediate 6d

A single-neck flask was charged with 6c (650 mg, 3.53 mmol) and N,N-dimethylformamide (10.0 mL) at room temperature, added N,N'-diisopropylethylamine (3.51 mL, 21.18 mmol), N,O-dimethylhydroxylamine hydrochloride (431 mg, 7.06 mmol) and 2-(7-azabenzotriazol)-N,N,N',N'-tetramethyluronium hexafluorophosphate (2.01 g, 5.30 mmol) under stirring, reacted for 2 h at room temperature. The reaction solution was poured into 50 mL water, extracted once with 50 mL ethyl acetate, the organic phase was dried with anhydrous sodium sulfate, filtered, the filtrate was then concentrated under pressure to afford a crude product, which was purified on silica gel chromatographic column (petroleum ether: ethyl acetate=100:1~5:1) to give Intermediate 6d (640 mg). LC-MS: m/z :228.2$(M+H)^+$, $^1H$ NMR (400 MHz, DMSO-d6) δ 4.85-4.75 (m, 1H), 3.64 (s, 3H), 3.20-3.00 (m, 4H), 2.59-2.51 (m, 2H), 2.35-2.23 (m, 2H).

Step 4: Preparation of Intermediate 6e

A 50 mL single-neck flask was charged with 1e (848 mg, 3.56 mmol) and tetrahydrofuran (6.0 mL) at room temperature, added butyl lithium (1.43 mL, 3.57 mmol) dropwise at -60°C, stirred for 0.5 h at this temperature, dissolved 6d (540 mg, 2.38 mmol) into tetrahydrofuran (5 mL), added into the reaction solution dropwise, reacted for 1.5 h at -60°C. The reaction solution was poured into ice water (50 mL), extracted once with 50 mL ethyl acetate, the organic phase was washed with 50 mL sat. brine, dried with sodium sulfate, then filtered, the filtrate was concentrated under pressure, and the crude product was purified on silica gel chromatographic column (petroleum ether: ethyl acetate=100:1~5:1) to afford Intermediate 6e (480 mg). LC-MS: m/z :325.1/327.1$(M+H)^+$, $^1H$ NMR (400 MHz, DMSO-d6) δ 8.79 (d, J=2.4 Hz, 1H), 8.69 (d, J=2.4 Hz, 1H), 5.00-4.87 (m, 1H), 3.90-3.78 (m, 1H), 2.72-2.61 (m, 2H), 2.46-2.34 (m, 2H).

Step 5: Preparation of Intermediate 6f

A single-neck flask was charged with 6e (300 mg, 0.92 mmol) and methanol (3.0 mL) in an ice bath, added sodium borohydride (52 mg, 1.38 mmol) under stirring, reacted for 2 h in the ice bath. The reaction solution was poured into 30 mL water, extracted once with 30 mL ethyl acetate, the organic phase was dried with anhydrous sodium sulfate, filtered, the filtrate was then concentrated under pressure to afford a crude product, which was purified on silica gel chromatographic column (petroleum ether: ethyl acetate=100:1~3:1) to give Intermediate 6f (240 mg). LC-MS: m/z : 327.1/329.1$(M+H)^+$.

Step 6: Preparation of Intermediate 6g

A single-neck flask was charged with 6f (130 mg, 0.40 mmol) and tetrahydrofuran (2.0 mL) at room temperature, sequentially added imidazole (81 mg, 1.19 mmol), triphenylphosphine (156 mg, 0.60 mmol) and iodine (151 mg, 0.60 mmol) under stirring. The reaction was carried out at 65°C for 18 h. The reaction solution was concentrated under pressure, and the crude product was purified on silica gel chromatographic column (petroleum ether: ethyl acetate=100:1~5:1) to afford Intermediate 6g (60 mg). LC-MS: m/z : 311.0/313.0$(M+H)^+$.

Step 7: Preparation of Compound **6**

A single-neck flask was charged with 6g (50 mg, 0.16 mmol) and dioxane (2.0 mL) at room temperature, sequentially added cesium carbonate (157 mg, 0.48 mmol), 3f (47 mg, 0.16 mmol), tris(dibenzylideneacetone)dipalladium (15 mg, 0.02 mmol) and 1,1-binaphthyl-2,2'-bis(diphenylphosphine) (20 mg, 0.03 mmol) under stirring. The reaction was carried out at 100°C for 18 h. The reaction solution was filtered, the filter cake was washed with 10 mL ethyl acetate, the filtrate was concentrated under pressure, and the crude product was prepared by high-performance liquid phase chromatography (formic acid/acetonitrile/water system) to afford Compound 6 (12 mg). LC-MS: m/z :525.2$(M+H)^+$, $^1H$ NMR (400 MHz, DMSO-d6) δ 8.35 (s, 1H), 7.87 (d, J=2.7 Hz, 1H), 7.66 (d, J=2.8 Hz, 1H), 7.06 (s, 1H), 4.74-4.64 (m, 1H), 4.54-4.44 (m, 1H), 3.74 (s, 2H), 3.73-3.66 (m, 1H), 2.80-2.69 (m, 4H), 2.47-2.43 (m, 1H), 2.40-2.23 (m, 8H), 2.20-2.10 (m, 2H), 1.95-1.83 (m, 2H).

**Example 7: Synthesis of Compound 7**

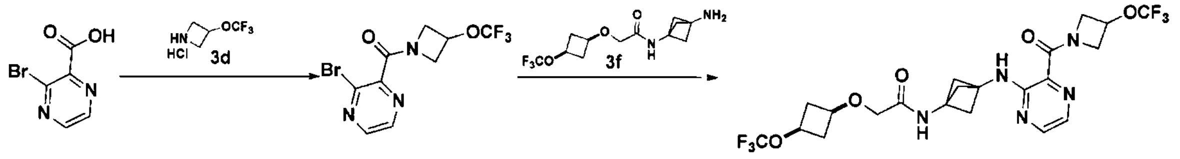


7a 7b Compound 7

Step 1: Preparation of Intermediate 7b

A single-neck flask was charged with 7a (300.0 mg, 1.48 mmol) and N,N-dimethylformamide (3.0 mL) at room temperature, sequentially added N,N'-diisopropylethylamine (380.02 mg, 2.96 mmol), N,N,N',N'-tetramethyl-O- (7-azabenzotriazole-1-yl)uronium hexafluorophosphate (842.9 mg, 2.22 mmol) and 3d (313.0 mg, 2.22 mmol) under stirring, reacted for 18 h at room temperature. To the reaction solution added 50 mL water, extracted once with 50 mL ethyl acetate, the organic phase was washed with 50 mL sat. brine, dried over sodium sulfate, filtered, and the filtrate was then concentrated under pressure to afford a crude product. The crude product was purified by flash column chromatography (petroleum ether: ethyl acetate=100:1~10:1) to afford Intermediate 7b (130 mg). LC-MS: m/z :326.0(M+H)$^+$.

Step 2: Preparation of Compound 7

A single-neck flask was charged with Intermediate 7b (100 mg, 0.31 mmol) and 1,4-dioxane (0.50 mL) at room temperature, sequentially added 3f (90.25 mg, 0.31 mmol), cesium carbonate (299.77 mg, 0.92 mmol), tris(dibenzylideneacetone)dipalladium(34 mg, 0.04 mmol) and 1,1-binaphthyl-2,2'-bis(diphenylphosphine) (46 mg, 0.07 mmol) under stirring, and the reaction was carried out at 100°C for 18 h after three cycles of nitrogen purging. The reaction solution was filtered, the filtrate was concentrated under pressure, the crude product was purified by high-performance liquid phase chromatography (ammonium bicarbonate/acetonitrile/water system) to finally afford Compound 7 (26 mg), $^1$H NMR (400 MHz, DMSO-d6) δ 8.91 (s, 1H), 8.45 (s, 1H), 8.27 (d, J=2.3 Hz, 1H), 7.88 (d, J=2.3 Hz, 1H), 5.21 (dd, J=6.5, 3.1 Hz, 1H), 4.92 (dd, J=11.3, 6.6 Hz, 1H), 4.65-4.57 (m, 1H), 4.54-4.40 (m, 2H), 4.11 (d, J=9.4 Hz, 1H), 3.75 (s, 2H), 3.73-3.66 (m, 1H), 2.81-2.68 (m, 2H), 2.35 (s, 6H), 2.19-2.12 (m, 2H), LC-MS: m/z :540.2(M+H)$^+$.

**Example 8: Synthesis of Compound 8**

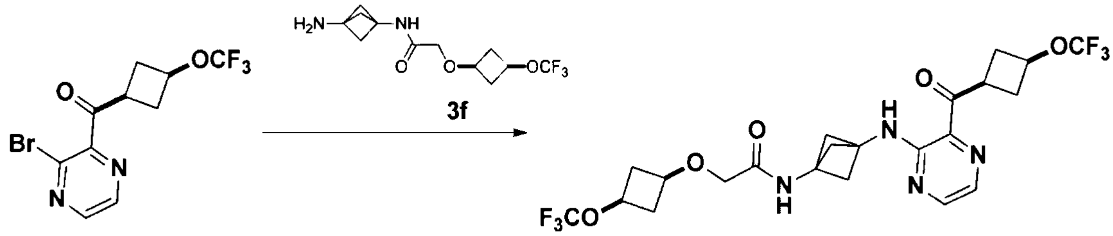


6e Compound 8

A single-neck flask was charged with 6e (120 mg, 0.37 mmol) and dioxane (2.0 mL) at room temperature, sequentially added cesium carbonate (361 mg, 1.11 mmol), 3f (109 mg, 0.37 mmol), tris(dibenzylideneacetone)dipalladium (34 mg, 0.04 mmol) and 1,1-binaphthyl-2,2'-bis(diphenylphosphine) (46 mg, 0.07 mmol) under stirring. The reaction was carried out at 100°C for 18 h after three cycles of nitrogen purging. The reaction solution was filtered, the filtrate was concentrated under pressure, and the crude product was prepared by high-performance liquid phase chromatography (formic acid/acetonitrile/water system) to afford Compound **6** (12 mg). LC-MS: m/z :539.3(M+H)$^+$. $^1$H NMR (400 MHz, DMSO-d6) δ 8.94 (s, 1H), 8.44 (s, 1H), 8.42 (d, J=2.1 Hz, 1H), 7.99 (d, J=2.2 Hz, 1H), 4.98-4.86 (m, 1H), 4.55-4.43 (m, 1H), 4.11-3.99 (m, 1H), 3.75 (s, 2H), 3.73-3.67 (m, 1H), 2.79-2.70 (m, 2H), 2.64-2.56 (m, 2H), 2.39 (s, 6H), 2.37-2.32 (m, 2H), 2.21-2.10 (m, 2H).

## Biological Assay

### 1. Cellular Activity Assay

The ATF4 luciferase reporter plasmid consists of two components: the 5' untranslated region sequence of the ATF4 gene and the luciferase encoding sequence. Specifically, the ATF4 5' untranslated region sequence (NCBI Database accession number BC022088.2) containing two upstream open reading frames (uORFs) and the firefly luciferase encoding gene were cloned into the pLVX-Puro vector (Unibio, VT1465). The packaging plasmids for lentivirus are psPAX2 (Unibio, VT1444) and pMD2.G (Unibio, VT1443). The three plasmids above were co-transfected into HEK293T/17 cells using X-tremeGENE 9 DNA transfection reagent, and the lentivirus-containing culture medium was collected 48 hours post-transfection. The HEK293T/17 cells transduced with the lentivirus were selected using 1 μg/mL puromycin, followed by obtaining a monoclonal cell line via limited dilution.

Using this cell line, the translational regulation of ATF4 can be detected via cold fluorescence readings, and the activity of the eIF2B activator can be tested. The specific experimental procedure was as follows: Seed 6,000 HEK293T/17-ATF4 uORF-Luc-Puro monoclonal cells in a 384-well plate and allow the cells to adhere overnight. The test compounds were dissolved in DMSO and added along with 50 nM thapsigargin into the cell culture medium, followed by incubation for 6 h. Thapsigargin was used to induce cellular stress, thereby upregulating translation of the ATF4 protein. After 6 hours of treatment, cells were lysed using the One-Glo Luciferase Assay Kit (Promega #E6120), then the luminescence values were measured using the LUM program of the EnVision 2104 plate reader.

The relative expression level of the ATF4 reporter gene (ATF4 reporter expression %) was calculated as follows:

- ATF4 reporter expression %=(ave_sample-ave_vc)/(ave_pc-ave_vc)*100%.
- ave_vc: mean signal value of negative control
- ave_pc: mean signal value of positive control
- ave_sample: mean signal value of sample

The $EC_{50}$ value was calculated by fitting a dose-response curve:

- The correspondence between the relative expression level of the ATF4 reporter gene and the compound concentration was fitted using the nonlinear regression log(inhibitor) vs. response - variable slope (four parameters) approach in GraphPad 9 software.
- X axis: log of compound concentration; Y: relative expression level of ATF4 reporter gene.
- 

$$\text{Equation: } Y=\text{Bottom}+(\text{Top}-\text{Bottom})/(1+10^{\wedge}((\text{LogEC}_{50}-X)*\text{HillSlope}))$$

In Table 1, "+" represents $EC_{50}$ that was>100 nM, "++" represents $EC_{50}$ between 10 nM and 100 nM, "+++" represent $EC_{50}$ between 1 nM to 10 nM, and "++++" represents $EC_{50}$ that was<1 nM.

Table 1 Structures of Compounds and Activity of HEK293T/17-ATF4 uORF-Luc-Puro Monoclonal Cells.

| Compound No. | Structures | $EC_{50}$ (nM) |
|---|---|---|
| 1 | 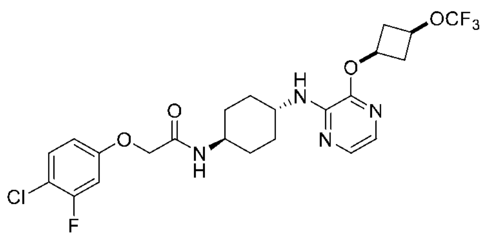 | ++ |

(continued)

| Compound No. | Structures | $EC_{50}$ (nM) |
|---|---|---|
| 2 | 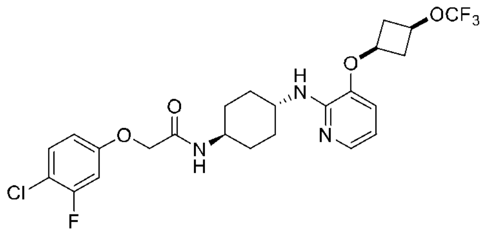 | + |
| 3 | 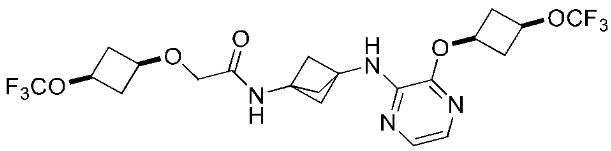 | +++ |
| 4 | 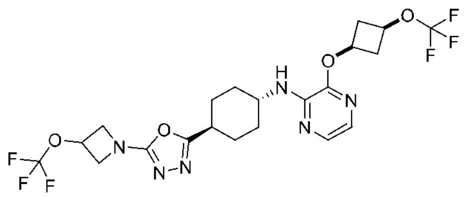 | + |
| 5 | 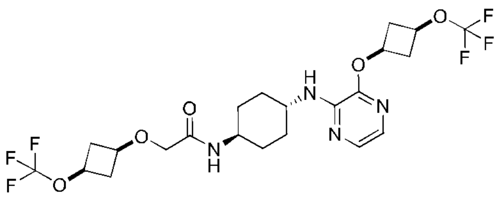 | + |
| 6 | 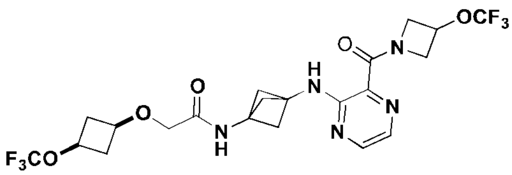 | + |
| 7 | 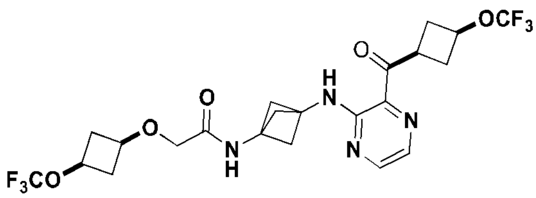 | + |

2. Pharmacokinetic Assessment in Mice

The test compound was dissolved in a solvent to prepare a clear solution or homogeneous suspension. Three mice per group were administered 1 mg/kg intravenously in the tails and 30 mg/kg orally (PO). Blood samples were collected at 0.083 h, 0.25 h, 0.5 h, 1 h, 2 h, 4 h, 8 h, and 24 h post intravenous administration, and at 0.25 h, 0.5 h, 1 h, 2 h, 4 h, 6 h, 8 h, and 24 h post oral administration. Plasma samples were centrifuged to obtain the supernatant, which was prepared as samples for quantitative analysis by LC/MS/MS.

Table 2 PK Properties of Exemplary Compound 3 in Mice

| | | |
|---|---|---|
| mouse IV (1 mg/kg) | $C_0$ (ng/mL) | 2619.53 |
| | $T_{1/2}$ (h) | 7.37 |
| | Cl (mL/h/kg) | 149.38 |
| | $V_{ss}$ (L/kg) | 1408.82 |
| | $AUC_{(0-t)}$ (ng*h/ml) | 6323.91 |
| mouse PO (30 mg/kg) | $C_{max}$ (ng/mL) | 20061.53 |
| | $T_{1/2}$ (h) | 8.52 |
| | $T_{max}$ (h) | 2.33 |
| | $AUC_{(0-t)}$ (ng*h/ml) | 217169.94 |
| | F% | 114.47 |

The experimental results demonstrated that some of the compounds in this application exhibited excellent pharmacokinetic properties in mice, including but not limited to CL (clearance), AUC, t1/2 (half-life), $C_{max}$ (peak concentration), AUC (area under the curve), F (bioavailability) and the like.

It should be noted that the present application is not limited to the above embodiments. The above embodiments are merely illustrative. All embodiments within the scope of the present application that have substantially the same composition as the technical idea and achieve the same effect are included in the technical scope of the present application. Furthermore, within the scope of the present application without departing from its subject matter, various modifications that a person skilled in the art would conceive of, as well as other embodiments constructed by combining a portion of the constituent elements of the embodiments, are also included in the scope of the present application.

## Claims

1. A compound of Formula 0, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof:

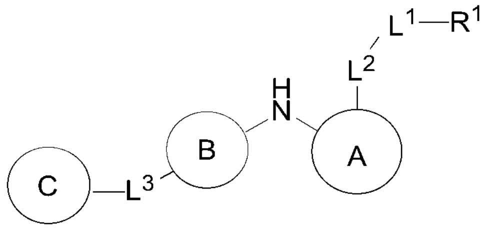

Formula 0

wherein in Formula 0, $R^1$ is selected from halogen, C1-C3 alkyl, C1-C3 haloalkyl, C1-C3 deuteroalkyl, C1-C3 alkylhydroxy, C1-C6 alkoxy and C1-C6 haloalkoxy;

$L^1$ is selected from C3-C10 cycloalkylene, 3-10-membered heterocycloalkylene and C1-C6 alkylene;

$L^2$ is selected from -O-, -NH-, C1-C6 alkylene and carbonyl;

ring A is selected from 5-10-membered heteroarylene containing at least one heteroatom N, and is optionally substituted with X $R^A$, wherein $R^A$ is selected from halogen, hydroxy, nitro, cyano, C1-C6 alkyl, C1-C6 alkoxy, C1-C6 haloalkoxy, C1-C6 haloalkyl, C3-C10 cycloalkyl and 3-10-membered heterocycloalkyl, and X is any integer from 0 to 4;

$L^2$ and -NH- are linked to ortho carbon atoms of ring A, respectively;

ring B is selected from C3-C10 cycloalkylene and 5-10-membered heterocycloalkylene, and is optionally substituted with Y $R^B$, wherein $R^B$ is selected from halogen, hydroxy, nitro, cyano, C1-C6 alkyl, C1-C6 alkoxy, C1-C6 haloalkyl, C1-C6 haloalkoxy, C3-C10 cycloalkyl and 3-10-membered heterocycloalkyl, and Y is any integer from 0 to 4;

$L^3$ is selected from a single bond, amide bond, thioamide bond, -O-$(CH_2)$n-amide bond, amide bond-$(CH_2)$n-O-, -O-$(CH_2)$m-thioamide bond, -thioamide bond-$(CH_2)$m-O- and 5-10-membered heteroarylene, wherein m and n are each independently 0, 1, 2 or 3; and

ring C is selected from C3-C10 cycloalkyl, 3-10-membered heterocycloalkyl, C6-C10 aryl, 5-10-membered heteroaryl and 3-10-membered benzoheterocycloalkyl, and is optionally substituted with Z $R^C$, wherein $R^C$ is selected from halogen, hydroxy, amino, nitro, cyano, C1-C6 alkyl, C1-C6 alkoxy, C1-C6 haloalkyl and C1-C6 haloalkoxy, and Z is any integer from 0 to 4.

2. The compound according to claim 1, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that**:
$R^1$ is selected from C1-C3 alkoxy and C1-C3 haloalkoxy, wherein preferably, the halogens of the C1-C3 haloalkoxy are each independently selected from F, Cl or Br, the number of C atoms of the C1-C3 haloalkoxy is preferably p, and the number of the halogens is any integer from 1 to 2p+1; the halogens of the C1-C3 haloalkoxy are preferably F or Cl; and $R^1$ is preferably C1-C3 perfluoroalkyl, more preferably -O-$CF_3$.

3. The compound according to claim 1 or 2, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that**:

$L^1$ is selected from C3-C6 cycloalkylene, 3-6-membered heterocycloalkylene and C1-C3 alkylene, preferably from cyclobutylene, cyclopentylene, cyclohexylene, azacyclobutylene, oxacyclobutylene, azacyclopentylene, oxacyclopentylene, azacyclohexylene, oxacyclohexylene, methylene, ethylene and propylene, more preferably from

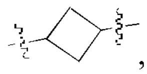, 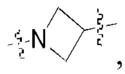,

methylene and ethylene, and even more preferably from

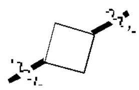, 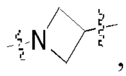,

methylene and ethylene,
preferably, the compound of Formula 0 is any one selected from the structures shown in the following Formulas I-1 and I-2:

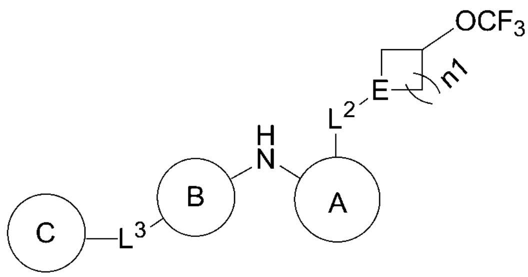

Formula I-1

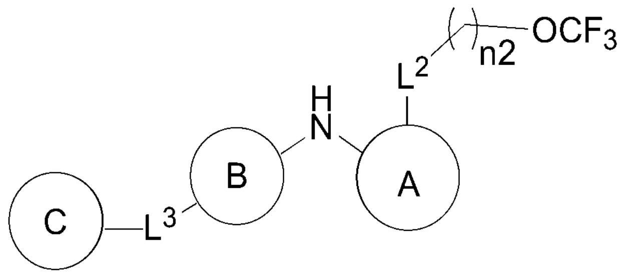

Formula I-2

wherein E is CH or N; n1 is 1, 2 or 3, preferably 1; n2 is 1, 2, 3 or 4, preferably 2; and $L^2$, ring A, $R^A$, X, ring B, $R^B$, Y, $L^3$, ring C, $R^C$ and Z are as defined in claim 1 or 2; and

preferably, the compound of Formula I-1 is any one selected from the structures shown in the following Formulas I-1-1 and I-1-2:

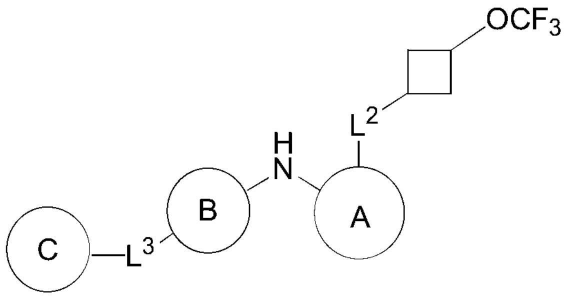

Formula I-1-1

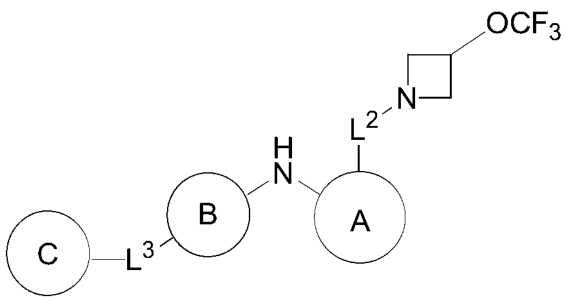

Formula I-1-2

wherein $L^2$, ring A, $R^A$, X, ring B, $R^B$, Y, $L^3$, ring C, $R^C$ and Z are as defined in claim 1 or 2.

4. The compound according to any one of claims 1 to 3, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that**:

$L^2$ is selected from -O-, -NH-, C1-C3 alkylene and carbonyl, preferably from -O-, -NH-, methylene, ethylene and carbonyl; and

preferably, the compound of Formula 0 is any one selected from the structures shown in the following Formulas II-1 to II-4:

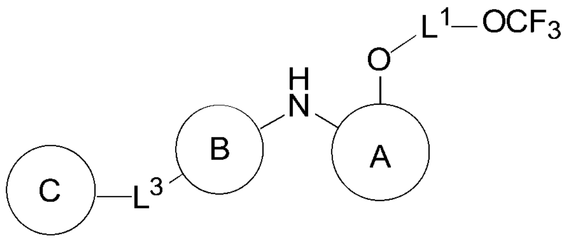

Formula II-1

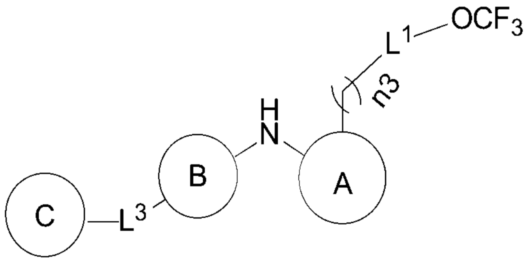

Formula II-2

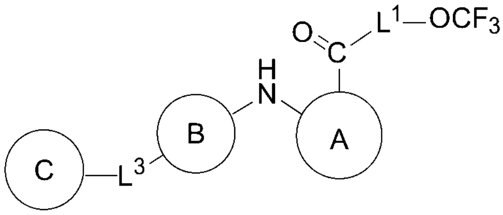

Formula II-3

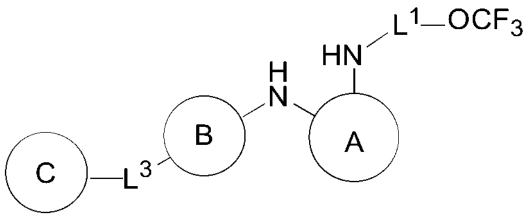

Formula II-4

wherein n3 is 1, 2 or 3, preferably 1; $L^1$, ring A, $R^A$, X, ring B, $R^B$, Y, $L^3$, ring C, $R^C$ and Z are as defined in claim 1 or 2.

**5.** The compound according to any one of claims 1 to 4, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that**:

ring A is selected from 5-6-membered heteroarylene containing at least one heteroatom N, preferably from pyridylene, pyrimidylene, pyrazinylene, pyridazinylene, imidazolylene, pyrazolylene, triazolylene, oxazolylene, oxadiazolylene, furazanylene, furylene, thiazolylene, isoxazolylene, isothiazolylene and

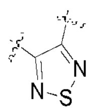 ;

ring A is preferably selected from pyridylene, pyrazinylene, triazolylene, furazanylene, isoxazolylene, and

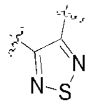 ;

ring A is more preferably selected from

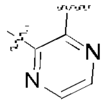, 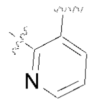, 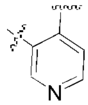, 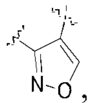, 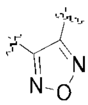, 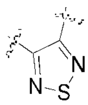,

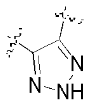, and 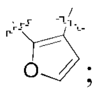 ;

ring A is even more preferably selected from

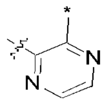, 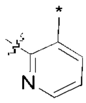, 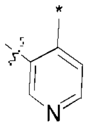, 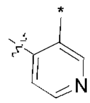, 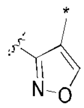,

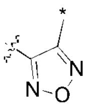, 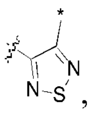, 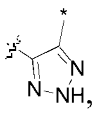, 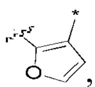, and 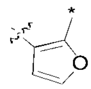,

wherein

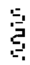

end is linked to -NH- and * end is linked to $L^2$;

$R^A$ is selected from halogen, hydroxy, nitro, cyano, C1-C3 alkyl, C1-C3 alkoxy, C1-C3 haloalkoxy, C1-C3 haloalkyl, C3-C6 cycloalkyl and 3-6-membered heterocycloalkyl, wherein X is any integer from 0 to 4, preferably 0 or 1;

preferably, the compound of Formula 0 is any one selected from the structures shown in the following Formulas III-1 and III-2:

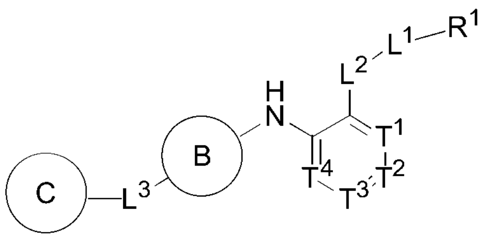

Formula III-1

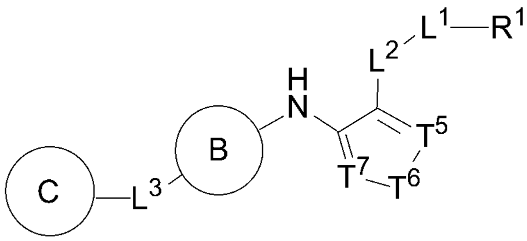

Formula III-2

wherein $T^1$, $T^2$, $T^3$ and $T^4$ are each independently C or N, and at least one of $T^1$, $T^2$, $T^3$ and $T^4$ is N; preferably, $T^1$ is N, $T^2$ and $T^3$ are C, and $T^4$ is C or N; or preferably, one of $T^1$, $T^2$ and $T^3$ is N, and $T^4$ is C;

$T^5$, $T^6$ and $T^7$ are each independently C, N, O or S; and

$R^1$, $L^1$, $L^2$, ring B, $R^B$, Y, $L^3$, ring C, $R^C$ and Z are as defined in any one of claims 1 to 4;

preferably, the compound of Formula III-1 is any one selected from the structures shown in the following Formula III-1-1:

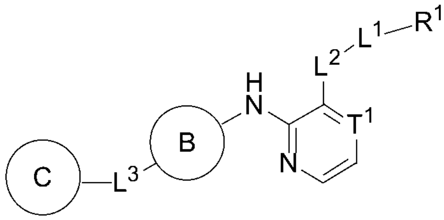

Formula III-1-1

wherein $T^1$ is C or N, and $R^1$, $L^1$, $L^2$, ring B, $R^B$, Y, $L^3$, ring C, $R^C$ and Z are as defined in any one of claims 1 to 4; and preferably, the compound of Formula III-2 is any one selected from the structures shown in the following Formulas III-2-1 or III-2-2:

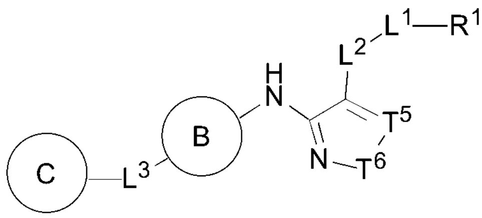

Formula III-2-1

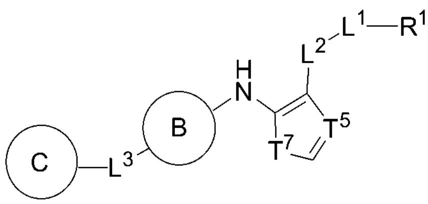

Formula III-2-2

wherein in Formula III-21, $T^5$ and $T^6$ are each independently C, N, O or S, and preferably, $T^5$ is C or N, and $T^6$ is N, O or S;

in Formula III-22, $T^5$ and $T^7$ are each independently C, O or S, and preferably, $T^5$ is C or O, $T^7$ is C or 0, and $T^5$ is different from $T^7$; and

$R^1$, $L^1$, $L^2$, ring B, $R^B$, Y, $L^3$, ring C, $R^C$ and Z are as defined in any one of claims 1 to 4.

**6.** The compound according to any one of claims 1 to 5, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that**:

ring B is selected from C4-C8 cycloalkylene and 5-6-membered heterocycloalkylene; the C4-C8 cycloalkylene is preferably C4-C8 monocycloalkyl or C4-C8 bridged cycloalkyl; ring B is preferably selected from cyclobutylene, cyclopentylene, cyclohexylene, bicyclo[2.2.1]heptanylene, bicyclo[2.2.2]octanylene, bicyclo[1.1.2]pentanylene, piperidylene and oxanylene, more preferably from

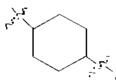, 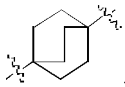, 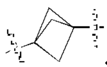, 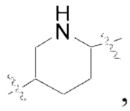, and 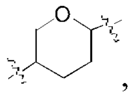,

even more preferably from

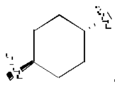, 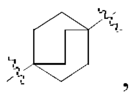, 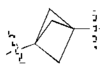, 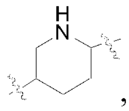, and 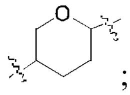;

$R^B$ is selected from halogen, hydroxy, nitro and cyano; and Y is any integer from 0 to 4, preferably 0; preferably, the compound of Formula 0 is any one selected from the structures shown in the following Formulas IV-1 to IV-3:

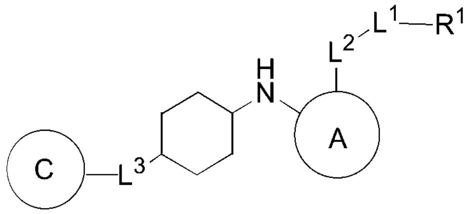

Formula IV-1

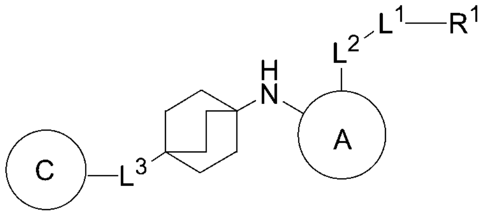

Formula IV-2

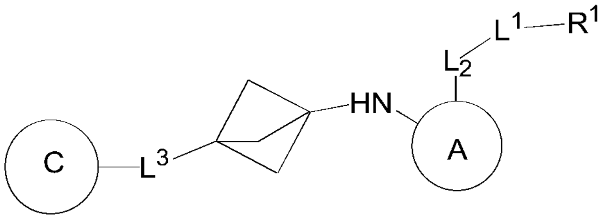

Formula IV-3

wherein $R^1$, $L^1$, $L^2$, ring A, $R^A$, X, $L^3$, ring C, $R^C$ and Z are as defined in any one of claims 1 to 5; preferably, the compound of Formula IV-1 is any one selected from the structures shown in the following Formulas

IV-1-1 and IV-1-2:

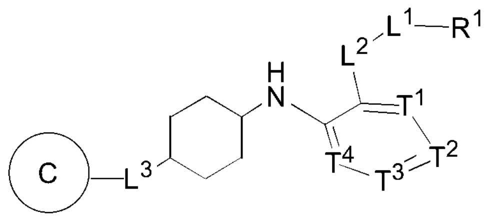

Formula IV-1-1

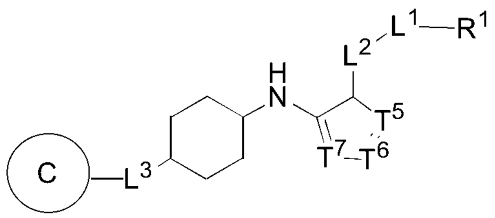

Formula IV-1-2

preferably, the compound of Formula IV-2 is any one selected from the structures shown in the following Formulas IV-2-1 and IV-2-2:

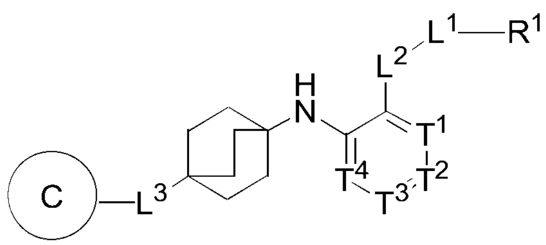

Formula IV-2-2

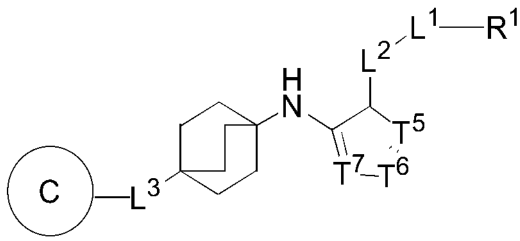

Formula IV-2-2

and preferably, the compound of Formula IV-3 is any one selected from the structures shown in the following Formulas IV-3-1 and IV-3-2:

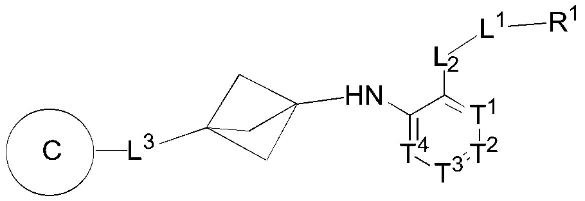

Formula IV-3-1

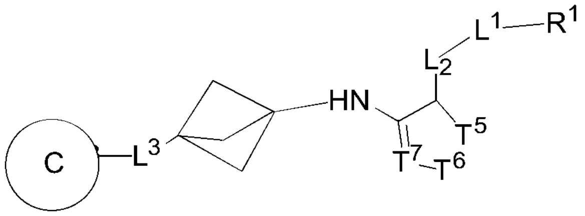

Formula IV-3-2

wherein $R^1$, $L^1$, $L^2$, $R^A$, X, $L^3$, ring C, $R^C$ and Z are as defined in any one of claims 1 to 5.

**7.** The compound according to any one of claims 1 to 6, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that**:

$L^3$ is selected from

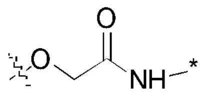, 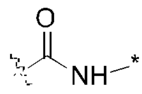, 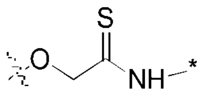

and 5-6-membered heteroarylene, wherein preferably,

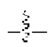

end is linked to ring C, and -* end is linked to ring B; the 5-6-membered heteroarylene is preferably selected from pyrrolylene, pyrazolylene, imidazolylene, triazolylene, oxazolylene, isoxazolylene, oxadiazolylene, and

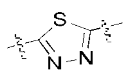 ;

$L^3$ is
preferably selected from

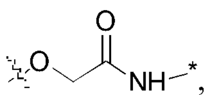, 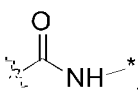, 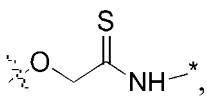, 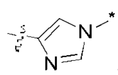, 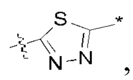,

and

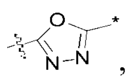,

wherein

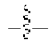

end is linked to ring C, and -* end is linked to ring B; and $L^3$ is more preferably selected from

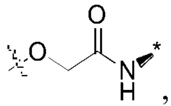, 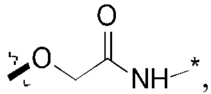, 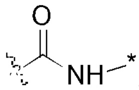, 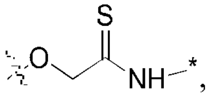, 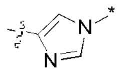,

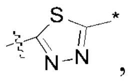, and 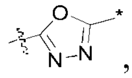,

wherein

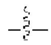

end is linked to ring C, and -* end is linked to ring B;
preferably, the compound of Formula 0 is any one selected from the structures shown in the following Formulas V-1 to V-3:

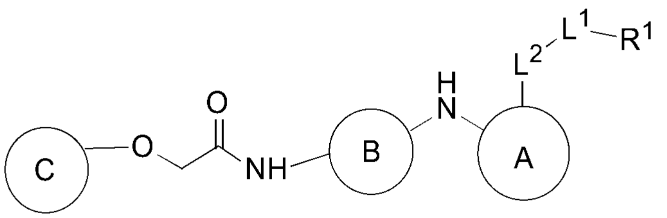

Formula V-1

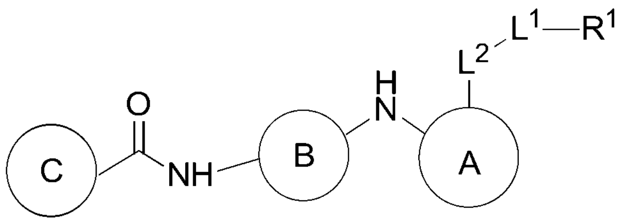

Formula V-2

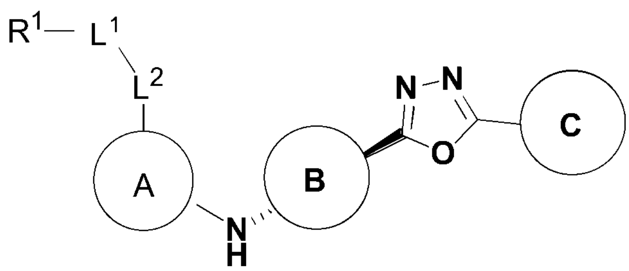

Formula V-3

wherein $R^1$, $L^1$, $L^2$, ring A, $R^A$, X, ring B, $R^B$, Y, ring C, $R^C$ and Z are as defined in any one of claims 1 to 6;
preferably, the compound of Formula V-1 is any one selected from the structures shown in the following Formulas V-1-1 to V-1-11:

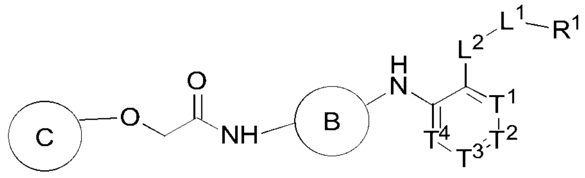

Formula V-1-1

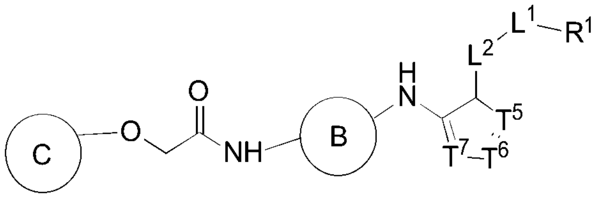

Formula V-1-2

wherein $R^1$, $L^1$, $L^2$, $R^A$, X, ring B, $R^B$, Y, ring C, $R^C$ and Z are as defined in any one of claims 1 to 6;

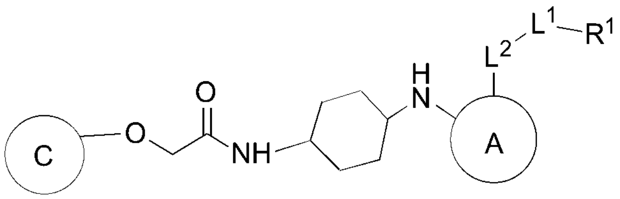

Formula V-1-3

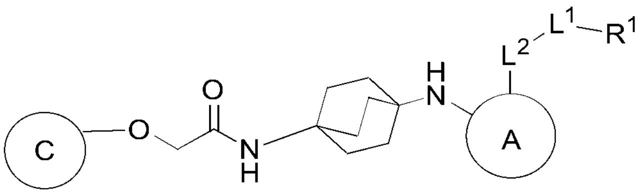

Formula V-1-4

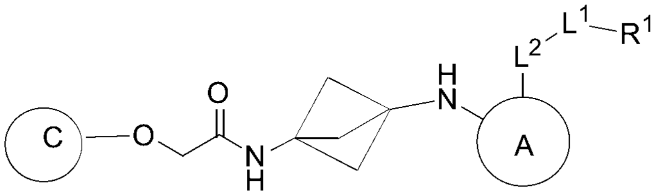

Formula V-1-5

$R^1$, $L^1$, $L^2$, ring A, $R^A$, X, $R^B$, Y, ring C, $R^C$ and Z are as defined in any one of claims 1 to 6; and

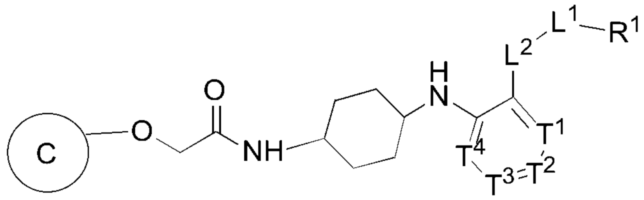

Formula V-1-6

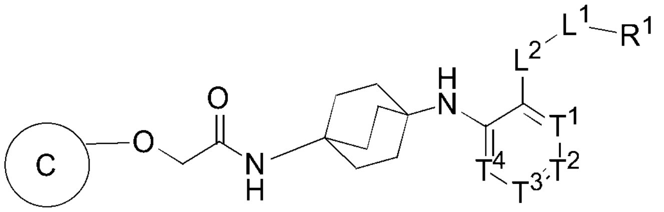

Formula V-1-7

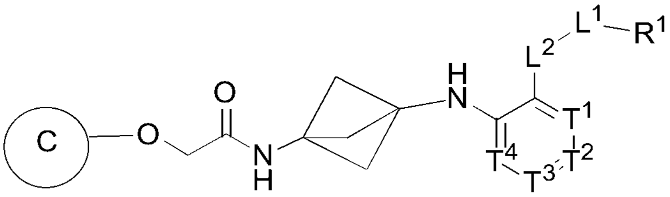

Formula V-1-8

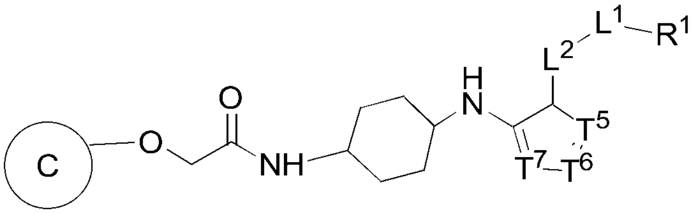

Formula V-1-9

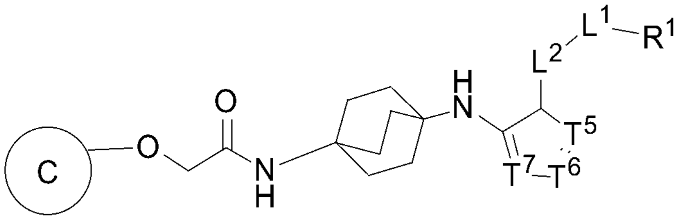

Formula V-1-10

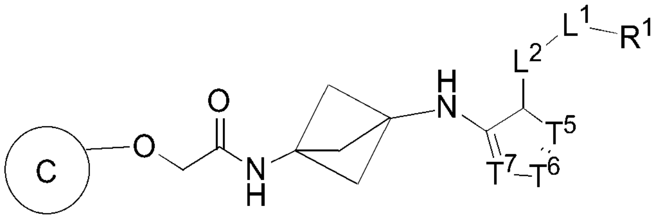

Formula V-1-11

$R^1$, $L^1$, $L^2$, $R^A$, X, $R^B$, Y, ring C, $R^C$ and Z are as defined in any one of claims 1 to 6; and preferably, the compound of Formula V-2 is any one selected from the structures shown in the following Formulas V-2-1 to V-2-11:

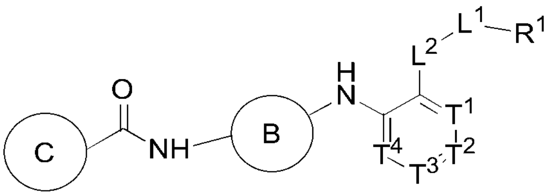

Formula V-2-1

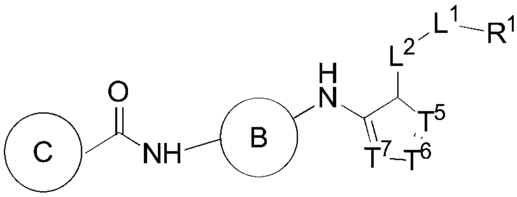

Formula V-2-2

wherein $R^1$, $L^1$, $L^2$, $R^A$, X, ring B, $R^B$, Y, ring C, $R^C$ and Z are as defined in any one of claims 1 to 6;

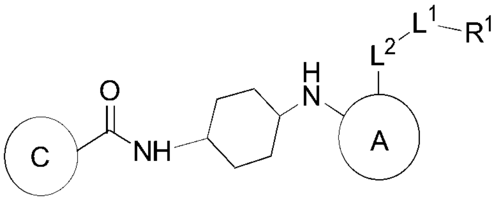

Formula V-2-3

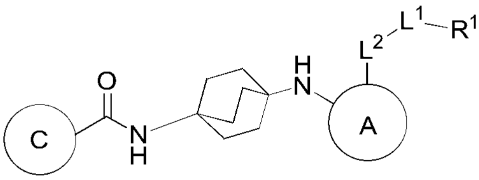

Formula V-2-4

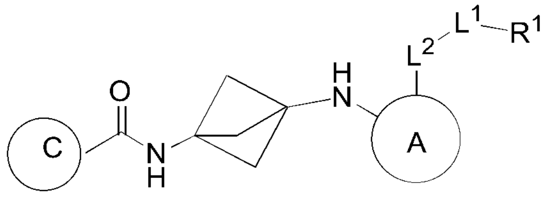

Formula V-2-5

$R^1$, $L^1$, $L^2$, ring A, $R^A$, X, $R^B$, Y, ring C, $R^C$ and Z are as defined in any one of claims 1 to 6; and

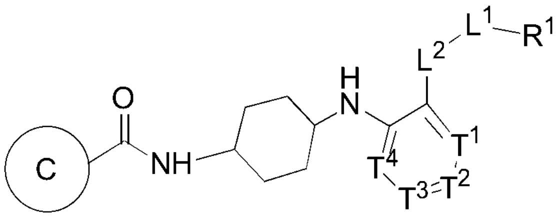

Formula V-2-6

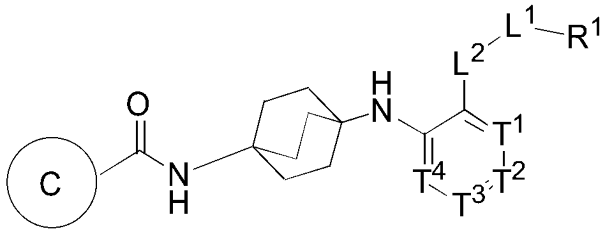

Formula V-2-7

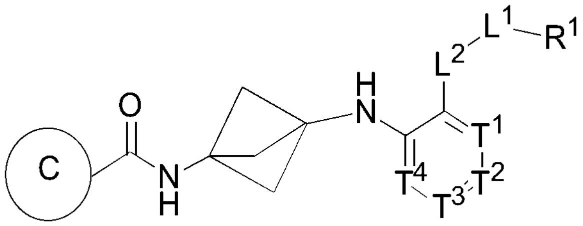

Formula V-2-8

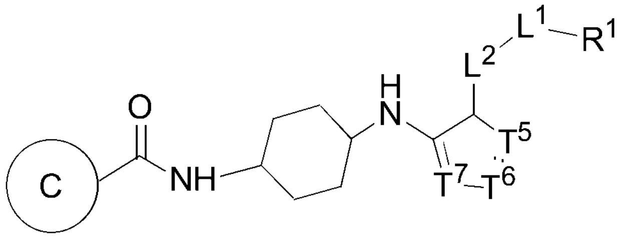

Formula V-2-9

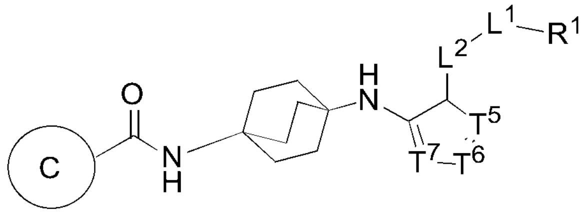

Formula V-2-10

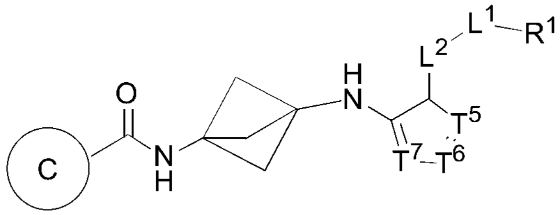

Formula V-2-11

$R^1$, $L^1$, $L^2$, $R^A$, X, $R^B$, Y, ring C, $R^C$ and Z are as defined in any one of claims 1 to 6.

**8.** The compound according to any one of claims 1 to 7, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that**:

ring C is selected from C3-C6 cycloalkyl, 3-6-membered heterocycloalkyl, C6-C10 aryl, 5-6-membered heteroaryl and 3-6-membered benzoheterocycloalkyl, preferably from cyclobutyl, cyclohexyl, azacyclobutyl, piperidyl, phenyl, thienyl, furyl, pyrrolyl, pyrazolyl, imidazolyl, triazolyl, pyridyl, pyrimidyl, pyrazinyl, pyridazinyl, triazinyl, quinolyl, isoquinolyl, quinazolinyl, quinoxalinyl, benzomorpholinyl and benzoxocyclohexyl, more preferably from cyclobutyl, phenyl, pyrazinyl, quinolyl, benzomorpholinyl and benzoxocyclohexyl, even more preferably from

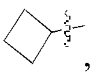, 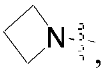, 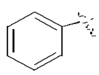, 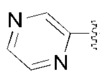, 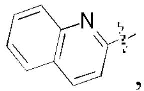, 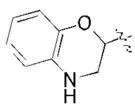, and 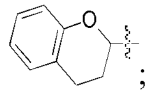;

ring C is more preferably selected from

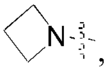, 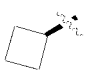, 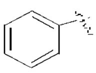, 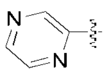, 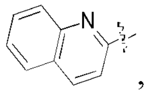,

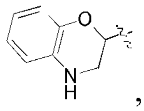, and 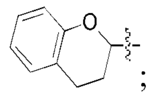;

$R^C$ is selected from halogen, hydroxy, C1-C3 alkyl, C1-C3 alkoxy, C1-C3 haloalkyl and - C1-C3 haloalkoxy; Z is any integer from 0 to 4; preferably, halogen, the halogen in the C1-C3 haloalkyl and the halogen in the C1-C3 haloalkoxy are each independently F, Cl or Br, more preferably F or Cl; $R^C$ is preferably selected from F, Cl, hydroxy, $-CH_2F$, $-CHF_2$, $-CF_3$ and $-O-CF_3$; and Z is preferably 1 or 2; and

preferably, the compound of Formula 0 is any one selected from the structures shown in the following Formulas VI-1 and VI-2:

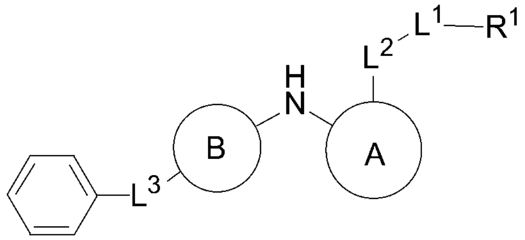

Formula VI-1

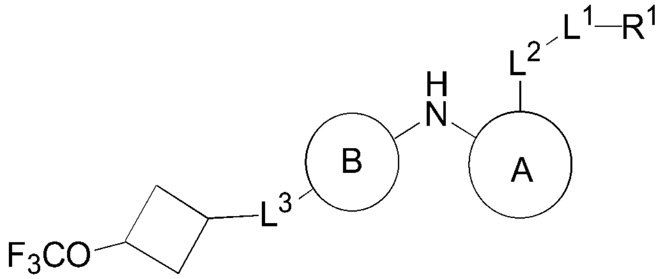

Formula VI-2

wherein $R^C$ in Formula VI-1 is selected from F, Cl, hydroxy, -CN, $-CH_3$, $-CH_2F$, $-CHF_2$, $-CF_3$, $-OCH_3$ and $-OCF_3$; Z is preferably 1 or 2; and
in Formulas VI-1 to VI-2, ring A, $R^A$, X, ring B, $R^B$, Y and $L^3$ are as defined in any one of claims 1 to 7.

**9.** The compound according to claim 1, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that**:

the compound of Formula 0 is any one selected from the structures shown in the following Formula VII:

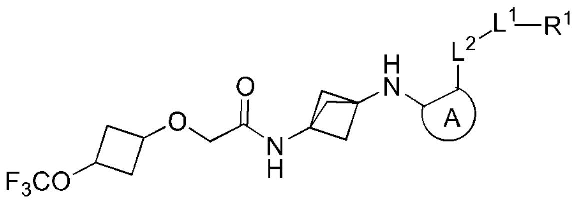

Formula VII

ring A is selected from 5-membered nitrogen-containing heteroarylene or 6-membered nitrogen-containing heteroarylene;
$L^1$ is selected from C3-C6-membered cycloalkylene or 4-6-membered heterocyclylene;
$L^2$ is selected from -O-; and
$R^1$ is selected from C1-C3 alkoxy or C1-C3 haloalkoxy.

**10.** The compound according to claim 9, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that** ring A is selected from 6-membered nitrogen-containing heteroarylene.

**11.** The compound according to claim 9 or 10, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that** $L^1$ is selected from C3-C5-membered cycloalkylene or 4-5-membered heterocyclylene.

**12.** The compound according to claim 11, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that** $L^1$ is selected from cyclobutylene or azacyclobutylene, preferably cyclobutylene.

**13.** The compound according to any one of claims 8 to 12, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that** $R^1$ is selected from C1-C3 haloalkoxy, preferably - $OCF_3$.

**14.** The compound according to claim 1, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that**:

the compound of Formula 0 is any one selected from the structures shown in the following Formula VIII:

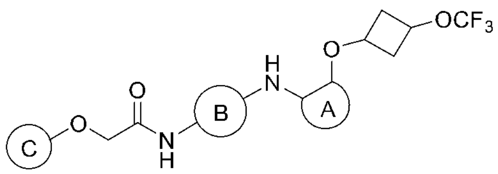

Formula VIII

ring A is selected from 5-membered nitrogen-containing heteroarylene or 6-membered nitrogen-containing heteroarylene;
ring B is selected from 4-8-membered cycloalkylene; and
ring C is selected from phenyl or cyclobutyl, each optionally substituted with 1 or 2 $R^C$, which is selected from halogen, C1-C3 alkyl, C1-C3 haloalkyl C1-C3 alkoxy or C1-C3 haloalkoxy.

**15.** The compound according to claim 14, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that** ring A is selected from 6-membered nitrogen-containing heteroarylene.

**16.** The compound according to claim 13, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that**:
the compound of Formula VIII is any one selected from the structures shown in the following Formula VIII-A:

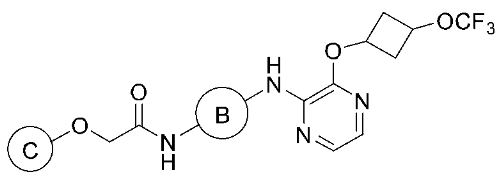

Formula VIII-A.

**17.** The compound according to any one of claims 13 to 16, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that**:
the compound of Formula VIII-A is any one selected from the structures shown in the following Formula VIII-A-1 or VIII-A-2:

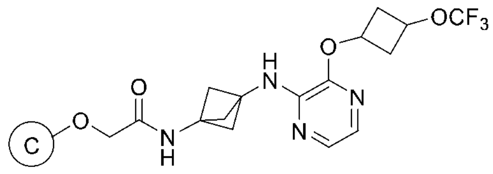

Formula VIII-A-1

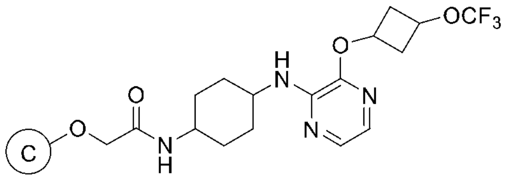

Formula VIII-A-2.

**18.** The compound according to any one of claims 13 to 16, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that** ring C is selected from cyclobutyl optionally substituted with C1-C3 haloalkyl.

**19.** The compound according to claim 1, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, **characterized in that** the compound is selected from any of the following compounds:

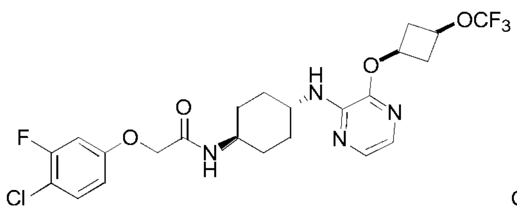

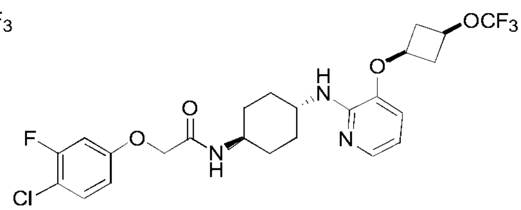

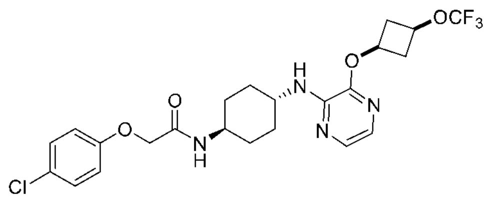

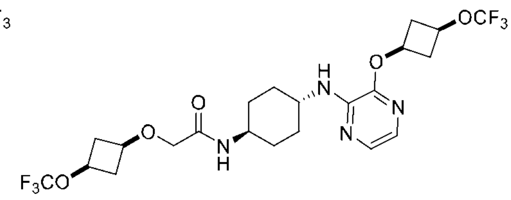

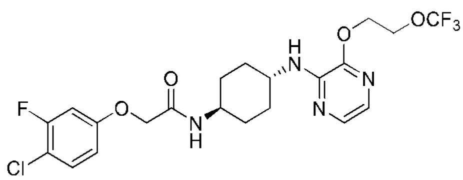

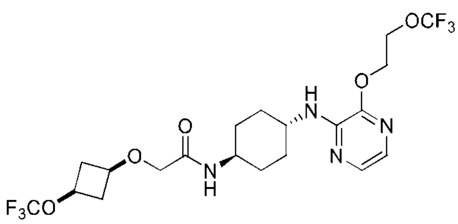

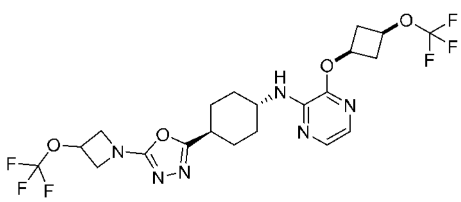

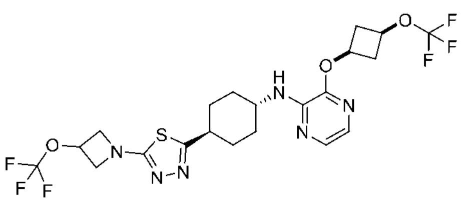

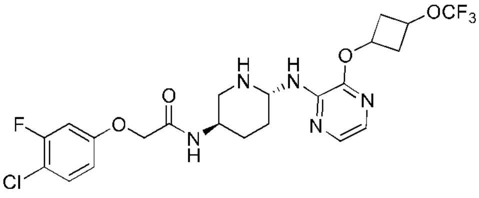
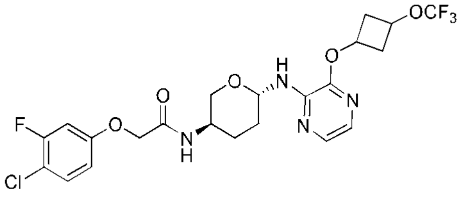

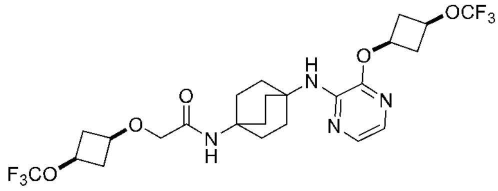
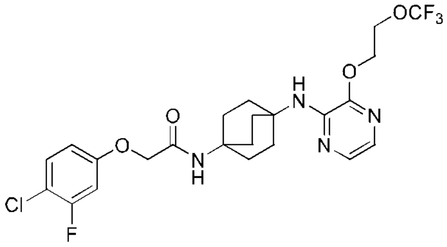

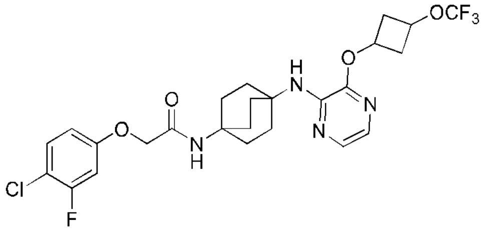
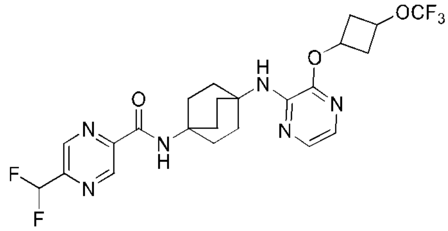

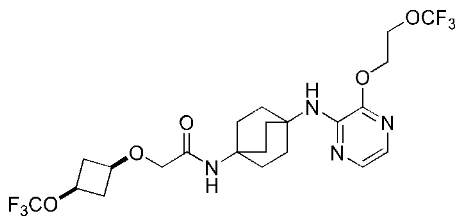
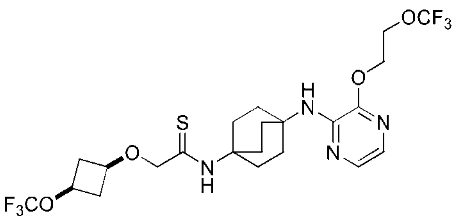

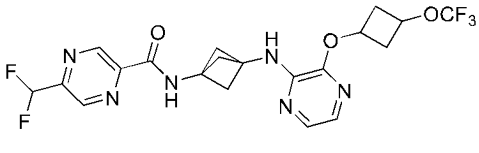
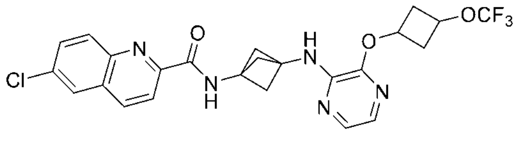

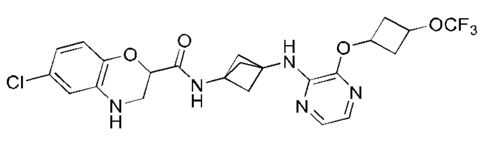
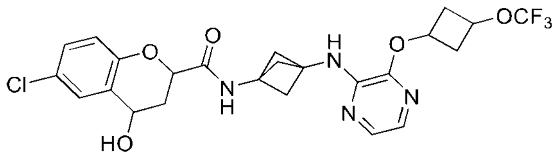

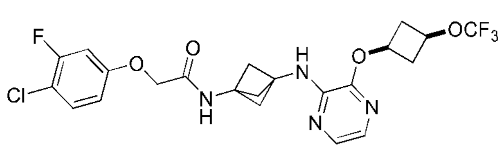
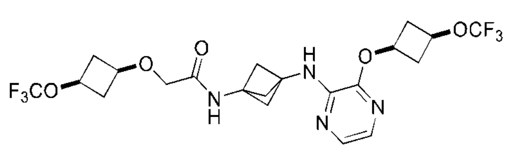

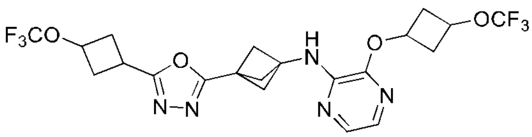
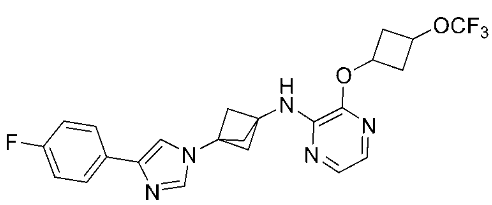

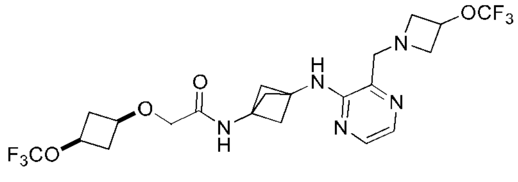
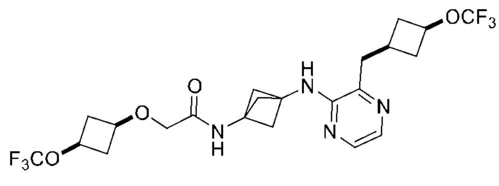

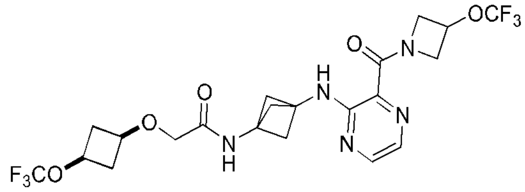
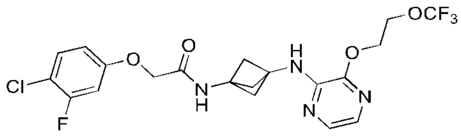

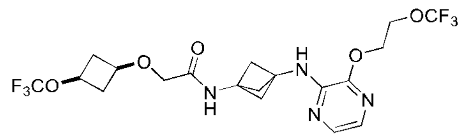
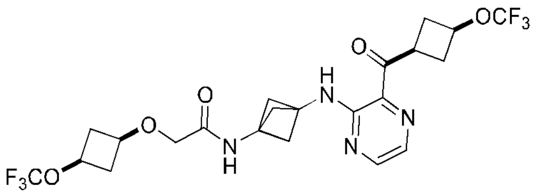

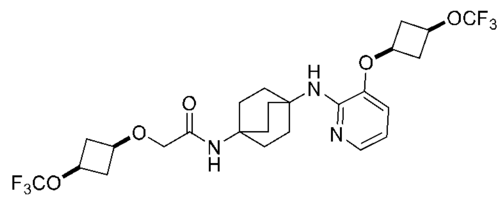
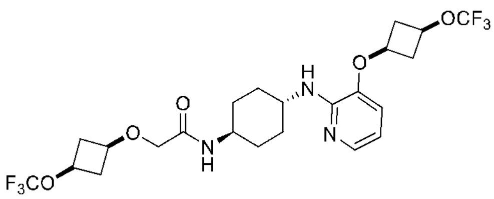

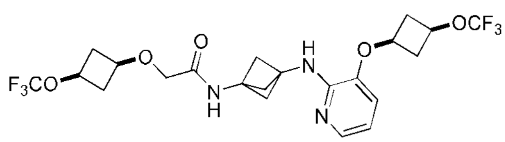
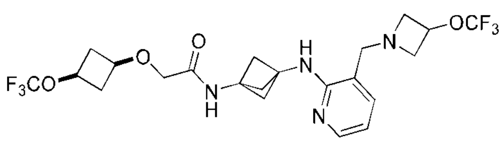

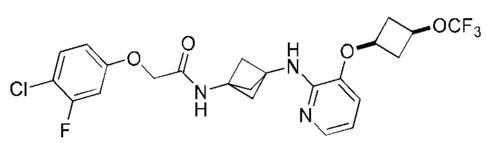
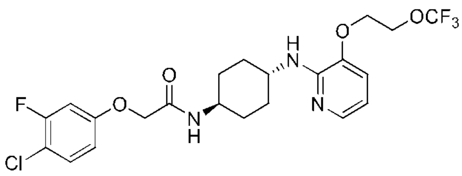

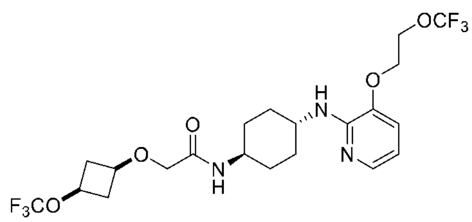
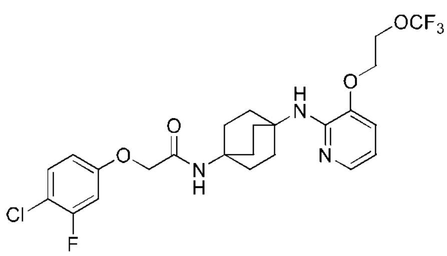

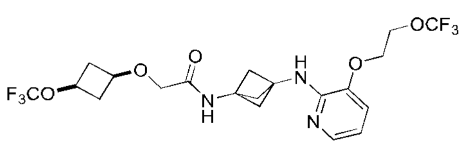
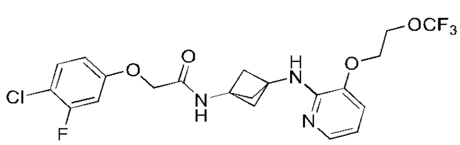

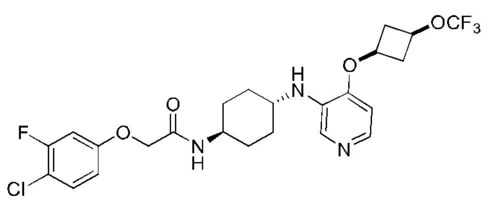 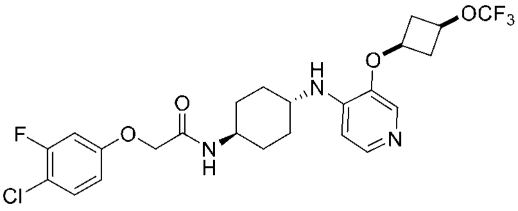

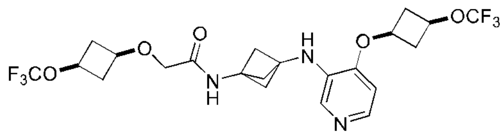 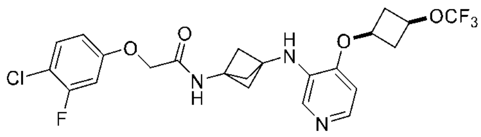

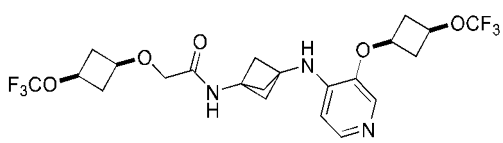 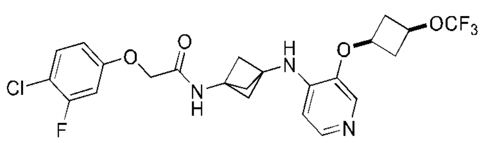

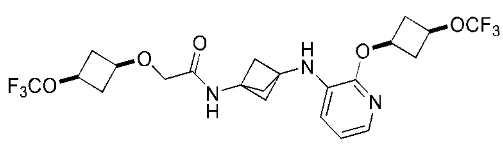 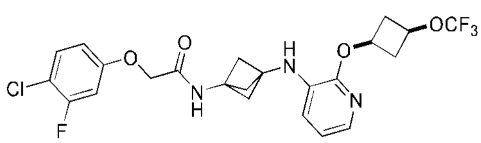

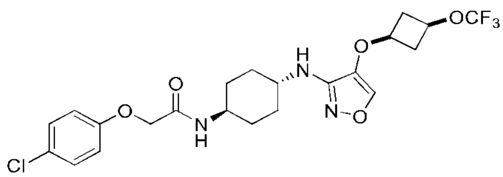 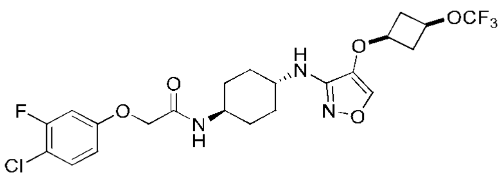

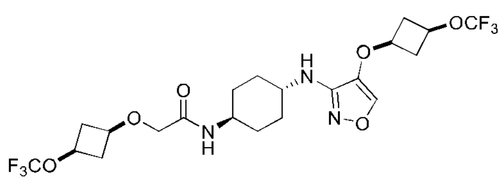 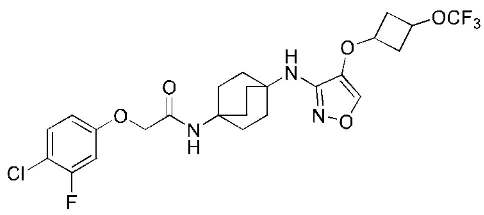

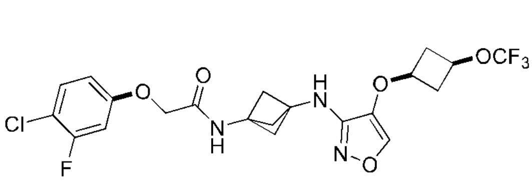 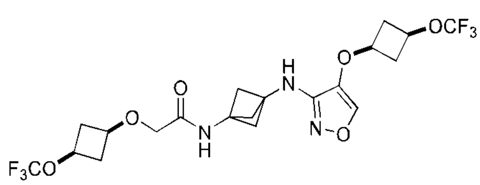

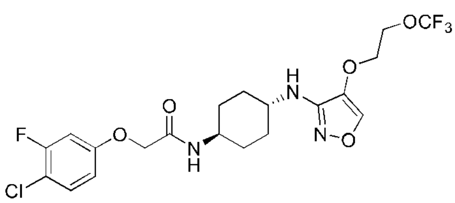 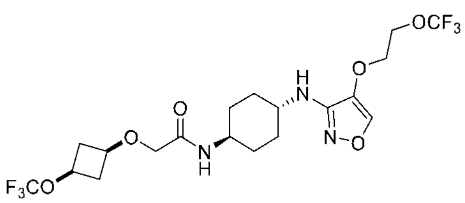

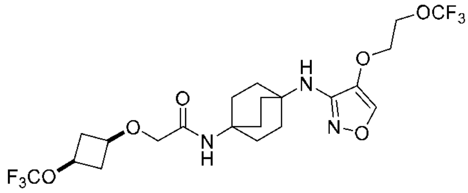
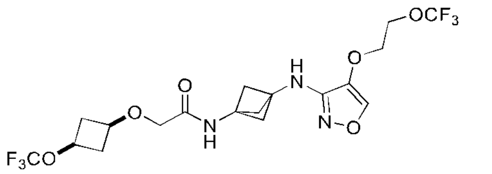

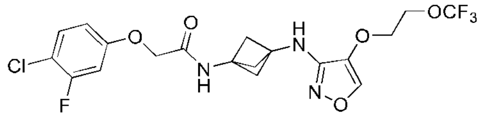
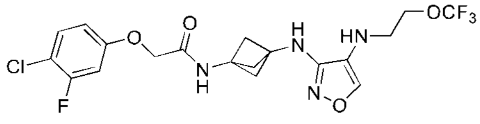

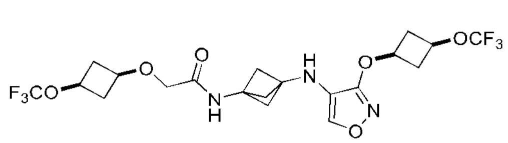
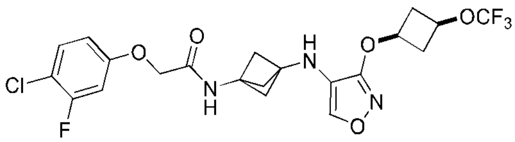

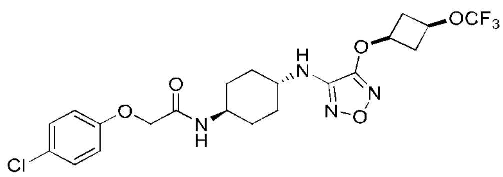
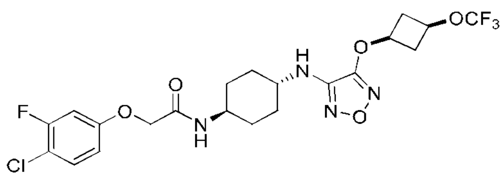

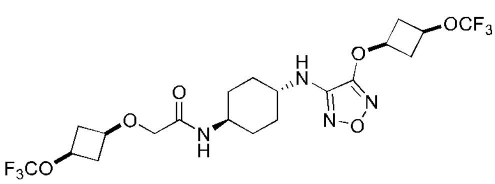
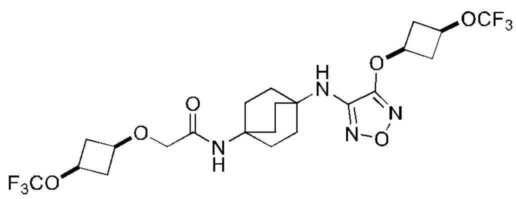

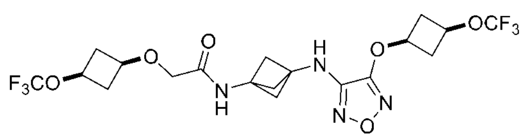
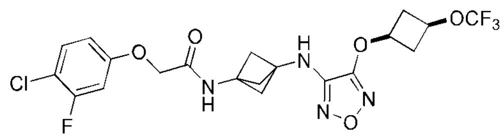

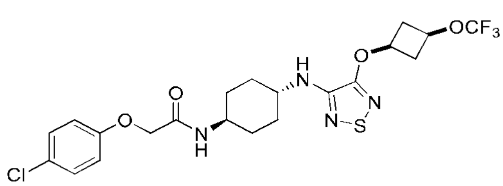
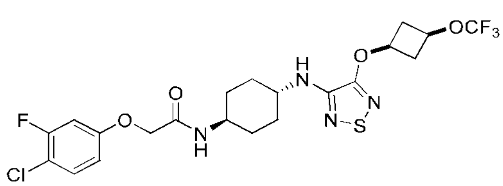

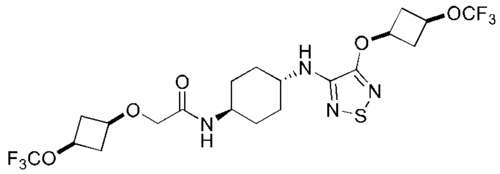
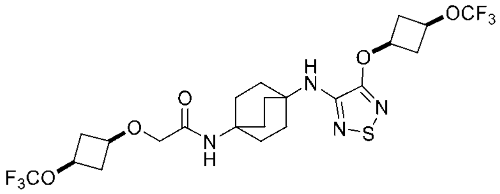

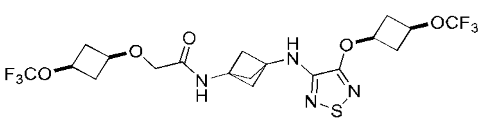
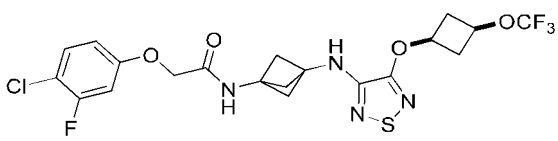

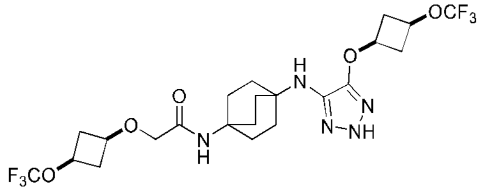

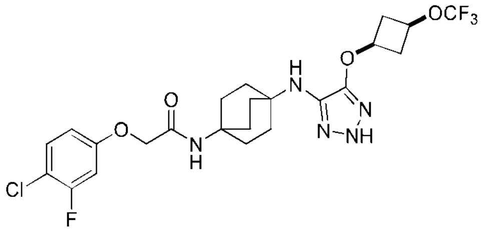

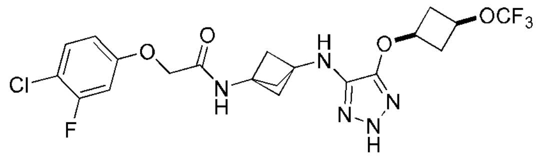

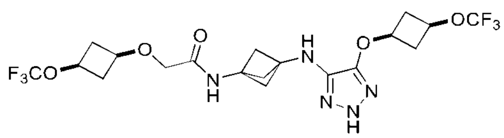

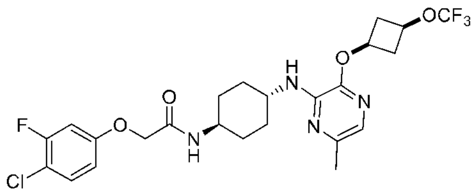

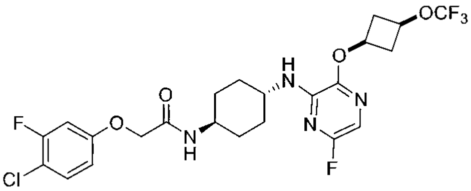

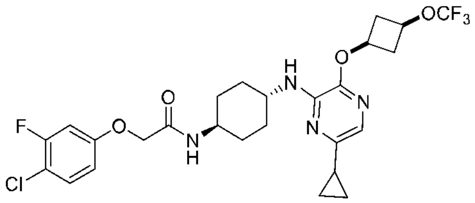

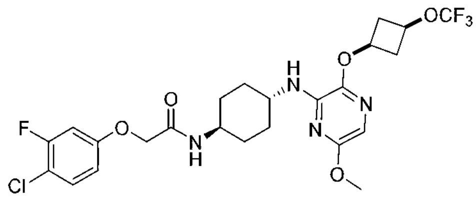

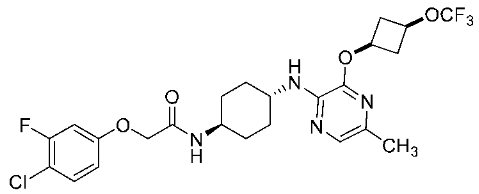

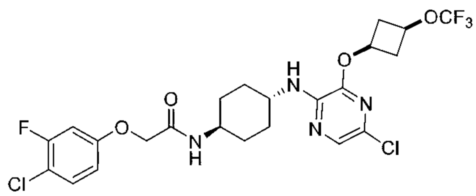

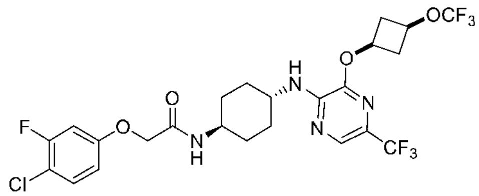

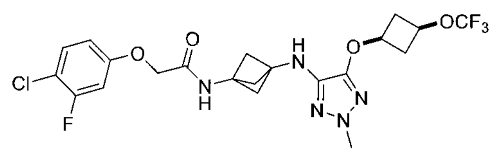

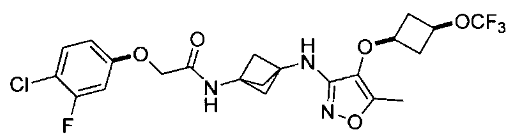

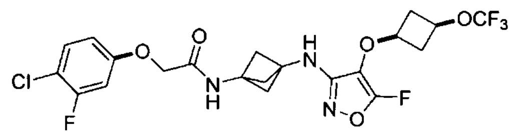

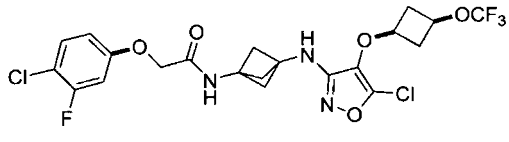

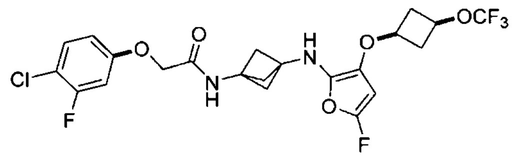

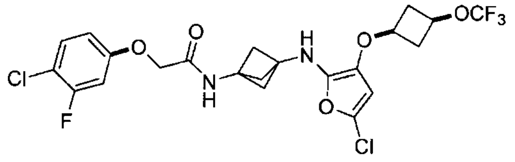

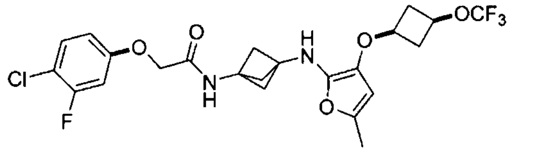

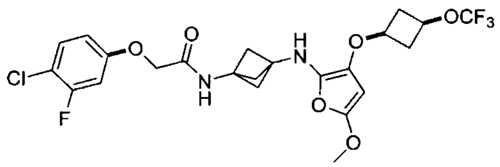

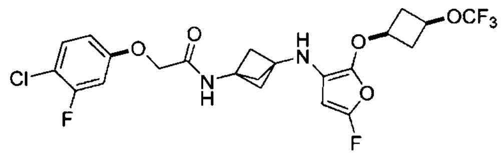

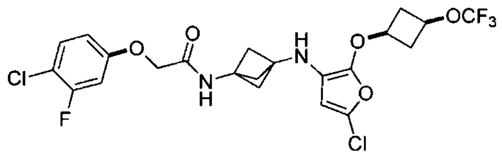

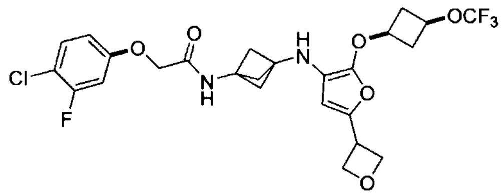

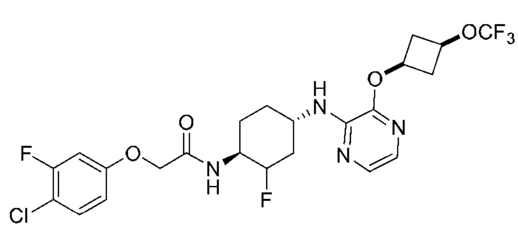

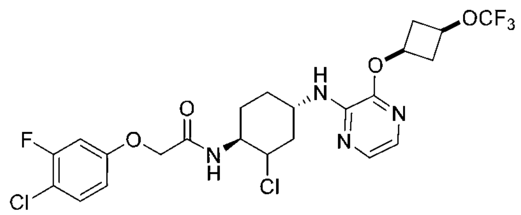

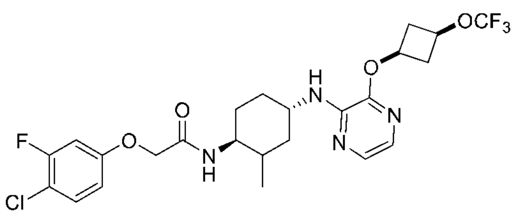

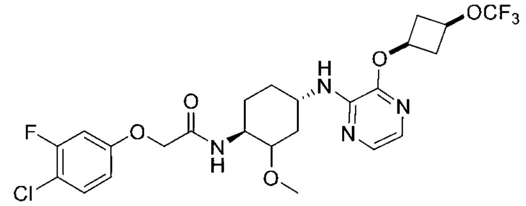

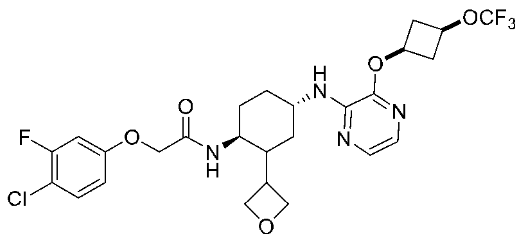

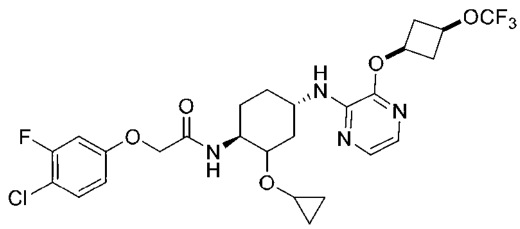

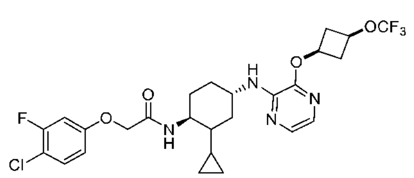

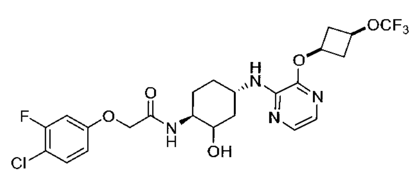

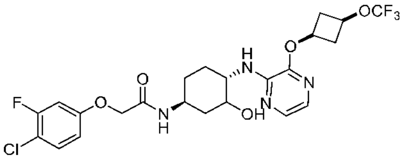 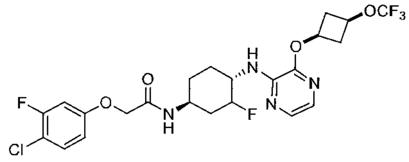

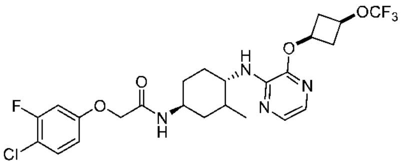 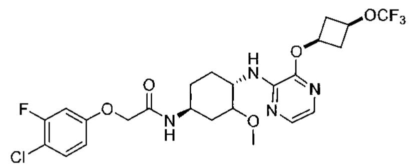

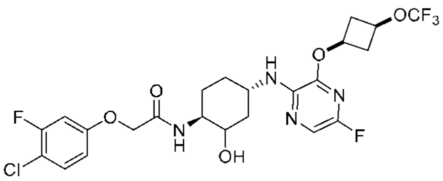 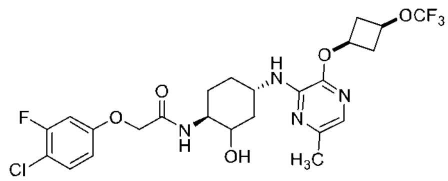

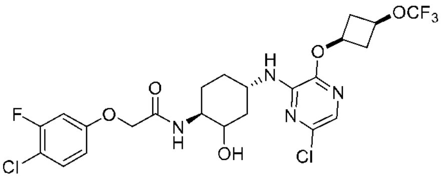

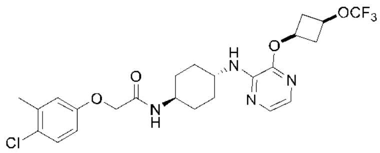 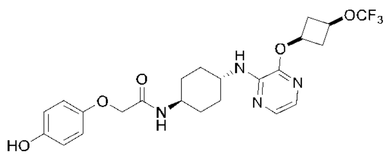

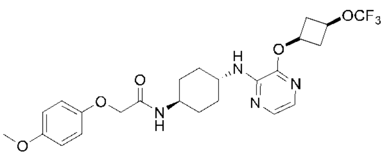 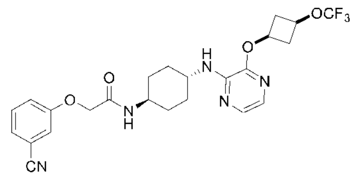

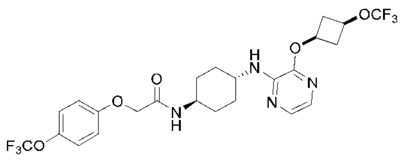 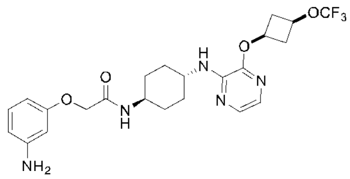

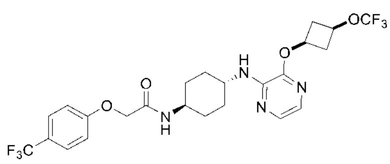

.

**20.** A synthesis method for compounds with the structures shown in Formulas V-1 to V-3 according to claim 7, comprising:

bringing raw material 1

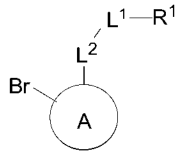

to undergo a substitution reaction with raw material 2

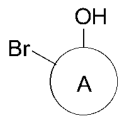

to afford intermediate 3, or bringing raw material 4 Br

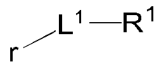

to undergo a substitution reaction with raw material 5

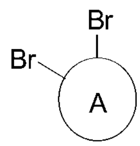

to afford intermediate 3, so as to synthesize the intermediate 3

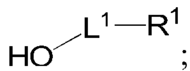
;

synthesizing the compound with the structure shown in Formula V-1 according to Scheme 1,

Scheme 1:

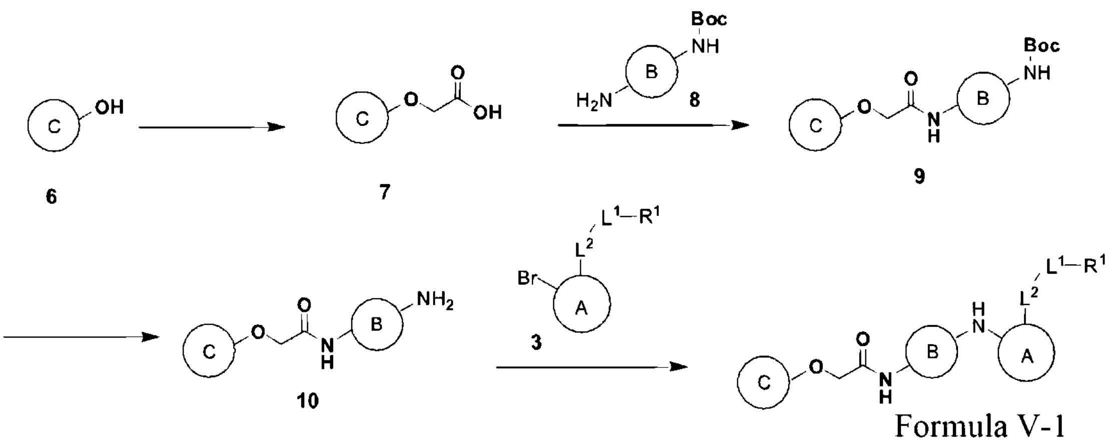

6 7 8 9 10 3

Formula V-1

wherein raw material 6, after undergoing a substitution reaction with methyl bromoacetate, was hydrolyzed to afford intermediate 7, which was then subjected to an amidation reaction with raw material 8 to give intermediate 9; then, the intermediate 9 was deprotected to provide intermediate 10; and the compound with the structure shown in Formula V-1 was obtained via a coupling reaction between the intermediate 10 and the intermediate 3; synthesizing the compound with the structure shown in Formula V-2 according to Scheme 2,

Scheme 2:

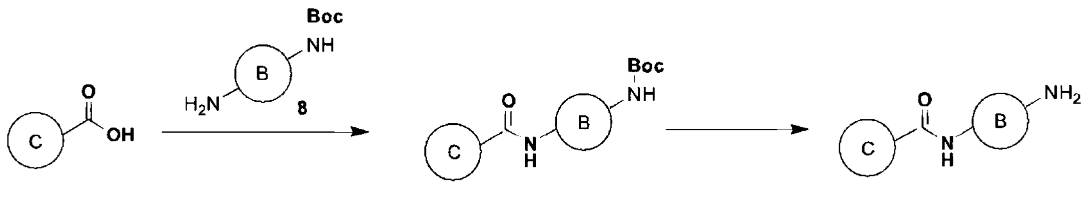

11 12 13

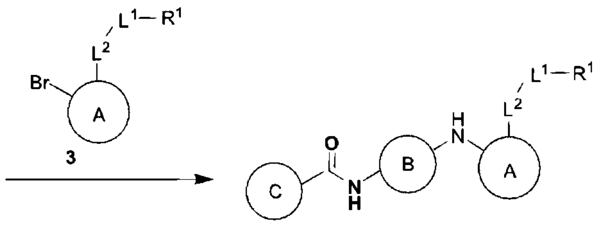

Formula V-2

wherein raw material 11 was subjected to an amidation reaction with the raw material 8 to give intermediate 12; then, the intermediate 12 was deprotected to provide intermediate 13; and the compound with the structure shown in Formula V-2 was obtained via a coupling reaction between the intermediate 13 and the intermediate 3; and

synthesizing the compound with the structure shown in Formula V-3 according to Scheme 3,

Scheme 3:

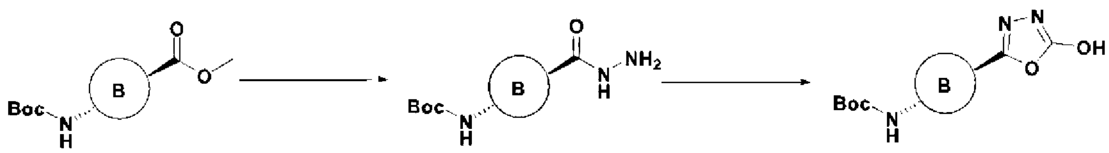

14 15 16

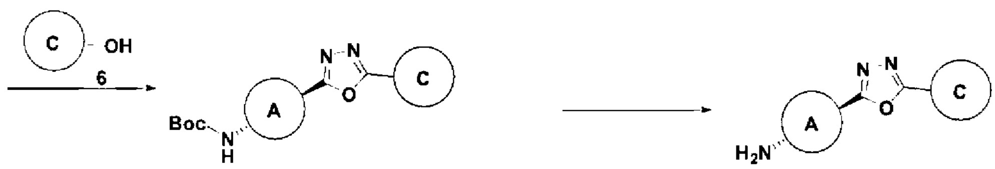

17 18

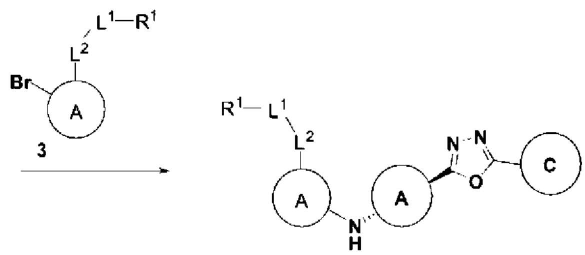

Formula V-3

wherein raw material 14 was reacted with hydrazine hydrate, and was subjected to functional group transformation to give intermediate 15, which was subjected to cyclization reaction with N,N-carbonyl diimidazole to give intermediate 16; then, condensation reaction was conducted between the intermediate 16 and the raw material 6

to afford intermediate 17, which was deprotected to provide intermediate 18; and the compound with the structure shown in Formula V-2 was obtained by coupling the intermediate 18 with the intermediate 3.

**21.** A pharmaceutical composition, comprising a preparation prepared from the compound according to any one of claims 1 to 19, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof.

**22.** The pharmaceutical composition according to claim 21, **characterized in** further comprising a pharmaceutically acceptable carrier, adjuvant and vehicle.

**23.** Uses of the compound according to any one of claims 1 to 19, or stereoisomers, tautomers, geometric isomers, enantiomers, diastereomers, racemates, polymorphs, solvates, hydrates, N-oxides, isotopically labeled compounds, metabolites, esters, prodrugs or pharmaceutically acceptable salts thereof, and the pharmaceutical composition according to claim 20 or 21 in the manufacture of a medicament for preventing and/or treating a neurodegenerative disease, cancer, inflammatory disease, autoimmune disease, virus infection, skin disease, fibrotic disease, hemoglobinopathy, kidney disease, hearing impairment, ophthalmic disease, disease caused by mutations that induce unfolded protein response (UPR), malaria infection, musculoskeletal disease, metabolic disease or mitochondrial disease.

**24.** Uses of the compound according to any one of claims 1 to 19, or stereoisomers, tautomers, geometric isomers, enantiomers, diastereomers, racemates, polymorphs, solvates, hydrates, N-oxides, isotopically labeled compounds, metabolites, esters, prodrugs, or pharmaceutically acceptable salts thereof, and the pharmaceutical composition according to claim 20 or 21 in the manufacture of a medicament for preventing and/or treating a disease or disorder mediated by an integrated stress response (ISR) pathway.

**INTERNATIONAL SEARCH REPORT**

International application No. **PCT/CN2024/105066**

**A. CLASSIFICATION OF SUBJECT MATTER**

C07D241/12(2006.01)i; C07D213/74(2006.01)i; A61K31/165(2006.01)i; C07D231/14(2006.01)i; C07D237/10(2006.01)i; C07D261/08(2006.01)i; C07D277/32(2006.01)i; C07D307/56(2006.01)i

According to International Patent Classification (IPC) or to both national classification and IPC

**B. FIELDS SEARCHED**

Minimum documentation searched (classification system followed by classification symbols)

IPC: C07D, A61K

Documentation searched other than minimum documentation to the extent that such documents are included in the fields searched

Electronic data base consulted during the international search (name of data base and, where practicable, search terms used)

CNTXT, ENTXTC, ENTXT, CNKI, 万方, WANFANG, 百度学术,BAIDU SCHOLAR, 必应, BING, ISI web of Science, STN: 众格生物, 曹斌, 赵立雨, 窦国生, 卢思竹, 谢德伟, 张启花, 王绍晖, 陈斌, 张佩宇, 整合应激, eIF2B, INTEGRATED STRESS RESPONSE, ISR, 结构式检索, structural formula search.

**C. DOCUMENTS CONSIDERED TO BE RELEVANT**

| Category* | Citation of document, with indication, where appropriate, of the relevant passages | Relevant to claim No. |
|---|---|---|
| PX | ACS. "RN 2988457-39-0" *STNEXT Registry,* 13 October 2023 (2023-10-13), page 1 | 1-8, 13, 18 |
| X | CN 112204009 A (CALICO LIFE SCIENCES LLC et al.) 08 January 2021 (2021-01-08) abstract, and description, paragraphs [0007]-[0051], [0145], [0405], [0473]-[0522], [0547]- [0558], and [2844] | 1-8, 13-24 |
| X | CN 109641854 A (CALICO LIFE SCIENCES LLC et al.) 16 April 2019 (2019-04-16) description, paragraphs [0006] and [0417], and claims 1, 31-32, and 40-52 | 1-8, 13, 18-24 |
| A | CN 110730777 A (GLAXOSMITHKLINE INTELLECTUAL PROPERTY DEVELOPMENT LIMITED) 24 January 2020 (2020-01-24) entire document | 1-24 |
| A | CN 112189008 A (CALICO LIFE SCIENCES LLC et al.) 05 January 2021 (2021-01-05) entire document | 1-24 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **11 October 2024** | **17 October 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/105066**

**C. DOCUMENTS CONSIDERED TO BE RELEVANT**

| Category* | Citation of document, with indication, where appropriate, of the relevant passages | Relevant to claim No. |
|---|---|---|
| A | WO 2019193541 A1 (GLAXOSMITHKLINE INTELLECTUAL PROPERTY DEVELOPMENT LIMITED) 10 October 2019 (2019-10-10)<br>entire document | 1-24 |

Form PCT/ISA/210 (second sheet) (July 2022)

**INTERNATIONAL SEARCH REPORT**
**Information on patent family members**

International application No.
**PCT/CN2024/105066**

| Patent document cited in search report | Publication date (day/month/year) | Patent family member(s) | Publication date (day/month/year) |
|---|---|---|---|
| CN 112204009 A | 08 January 2021 | TW 201927756 A | 16 July 2019 |
| | | CA 3080806 A1 | 09 May 2019 |
| | | MX 2020004555 A | 05 October 2020 |
| | | EP 3710428 A1 | 23 September 2020 |
| | | CO 2020006248 A2 | 10 August 2020 |
| | | CL 2020001167 A1 | 28 December 2020 |
| | | JP 2021501786 A | 21 January 2021 |
| | | WO 2019090076 A1 | 09 May 2019 |
| | | AU 2023202592 A1 | 18 May 2023 |
| | | BR 112020008839 A2 | 20 October 2020 |
| | | RU 2020117900 A | 02 December 2021 |
| | | KR 20200096522 A | 12 August 2020 |
| | | IL 274369 A | 30 June 2020 |
| | | IL 274369 B1 | 01 December 2023 |
| | | IL 274369 B2 | 01 April 2024 |
| | | AU 2018360857 A1 | 04 June 2020 |
| | | US 2020345727 A1 | 05 November 2020 |
| | | CN 112204009 B | 07 May 2024 |
| CN 109641854 A | 16 April 2019 | JP 2019530649 A | 24 October 2019 |
| | | JP 6899063 B2 | 07 July 2021 |
| | | CA 3023261 A1 | 09 November 2017 |
| | | US 2019135772 A1 | 09 May 2019 |
| | | US 10913727 B2 | 09 February 2021 |
| | | US 2022106281 A1 | 07 April 2022 |
| | | AU 2017261336 A1 | 22 November 2018 |
| | | AU 2017261336 B2 | 04 November 2021 |
| | | TW 201808888 A | 16 March 2018 |
| | | WO 2017193063 A1 | 09 November 2017 |
| | | EP 3452456 A1 | 13 March 2019 |
| | | EP 3452456 B1 | 05 July 2023 |
| | | JP 2021138718 A | 16 September 2021 |
| | | CN 109641854 B | 03 February 2023 |
| CN 110730777 A | 24 January 2020 | BR 112019025883 A2 | 30 June 2020 |
| | | CA 3066328 A1 | 13 December 2018 |
| | | EP 3634952 A1 | 15 April 2020 |
| | | JP 2020522553 A | 30 July 2020 |
| CN 112189008 A | 05 January 2021 | US 2020361941 A1 | 19 November 2020 |
| | | JP 2021501789 A | 21 January 2021 |
| | | JP 7337790 B2 | 04 September 2023 |
| | | MX 2020004551 A | 05 October 2020 |
| | | AU 2018358346 A1 | 04 June 2020 |
| | | IL 274368 A | 30 June 2020 |
| | | IL 274368 B1 | 01 December 2023 |
| | | IL 274368 B2 | 01 April 2024 |
| | | AU 2023201821 A1 | 27 April 2023 |
| | | BR 112020008841 A2 | 20 October 2020 |
| | | TW 201932447 A | 16 August 2019 |
| | | CA 3080801 A1 | 09 May 2019 |
| | | KR 20200096918 A | 14 August 2020 |
| | | WO 2019090069 A1 | 09 May 2019 |

Form PCT/ISA/210 (patent family annex) (July 2022)

**INTERNATIONAL SEARCH REPORT**
**Information on patent family members**

International application No.
**PCT/CN2024/105066**

| Patent document cited in search report | Publication date (day/month/year) | Patent family member(s) | Publication date (day/month/year) |
|---|---|---|---|
| | | EP 3704094 A1 | 09 September 2020 |
| | | RU 2020117927 A | 03 December 2021 |
| | | US 2024166652 A1 | 23 May 2024 |
| WO 2019193541 A1 | 10 October 2019 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- McGraw-Hill Dictionary of Chemical Terms. McGraw-Hill Book Company, 1984
- **ELIEL, E.** ; **WILEN, S.** Stereochemistry of Organic Compounds. John Wiley & Sons, Inc., 1994